(19) Europäisches Patentamt
European Patent Office
Office européen des brevets

(11) **EP 4 696 786 A2**

(12) **EUROPEAN PATENT APPLICATION**

(43) Date of publication:
**18.02.2026 Bulletin 2026/08**

(51) International Patent Classification (IPC):
***C12Q 1/6804*** (2018.01)

(21) Application number: **25194035.9**

(22) Date of filing: **28.06.2022**

(52) Cooperative Patent Classification (CPC):
(C-Sets available)
**C12Q 1/6841; C12Q 1/6804** (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.06.2021 US 202163216315 P**

(62) Document number(s) of the earlier application(s) in
accordance with Art. 76 EPC:
**22748129.8 / 4 363 614**

(71) Applicants:
• **The Broad Institute Inc.**
**Cambridge, MA 02142 (US)**
• **Massachusetts Institute of Technology**
**Cambridge, MA 02139 (US)**

(72) Inventors:
• **WANG, Xiao**
**Cambridge, MA 02142 (US)**
• **ZENG, Hu**
**Cambridge, MA 02142 (US)**
• **REN, Jingyi**
**Cambridge, MA 02139 (US)**

(74) Representative: **Impact Intellectual Property LLP**
**1 Sans Walk**
**London EC1R 0LT (GB)**

Remarks:
•This application was filed on 05-08-2025 as a
divisional application to the application mentioned
under INID code 62.
•Claims filed after the date of receipt of the divisional
application (Rule 68(4) EPC).

(54) **SINGLE-CELL PROFILING OF RNA TRANSLATION STATUS**

(57) The present disclosure provides methods and systems for profiling RNAs being translated in a cell. Also provided by the present disclosure are methods for diagnosing a disease or disorder in a subject based on a profile of the RNAs being translated in a cell, including cells within an intact tissue. Methods of screening for or testing a candidate agent capable of modulating translation of one or more RNAs are also provided by the present disclosure. The present disclosure also provides methods for treating a disease or disorder in a subject in need thereof. Pairs of probes and sets of probes comprising oligonucleotide portions, which may be useful for performing the methods described herein, are also described by the present disclosure. Additionally, the present disclosure provides kits comprising any of the probes described herein.

**FIG. 1**

(52) Cooperative Patent Classification (CPC): (Cont.)

C-Sets
**C12Q 1/6804, C12Q 2531/125, C12Q 2533/107,
C12Q 2535/122, C12Q 2537/159, C12Q 2537/162,
C12Q 2543/101, C12Q 2563/179;
C12Q 1/6841, C12Q 2531/125, C12Q 2533/107,
C12Q 2535/122, C12Q 2537/159, C12Q 2537/162,
C12Q 2543/101, C12Q 2563/179**

**Description**

**RELATED APPLICATIONS**

This application claims priority under 35 U.S.C § 119(e) to U.S. Provisional Application, U.S.S.N. 63/216,315, filed June 29, 2021, which is incorporated herein by reference.

**BACKGROUND OF THE INVENTION**

Transcriptional profiles in single cells do not consistently correlate with proteomic profiles from the same cells. This suggests that various mechanisms exist at the post-transcriptional level to regulate protein production and degradation, including some that have yet to be characterized. Therefore, mRNA levels are an imperfect proxy for estimating protein production and are likely biased in defining cellular states. Additional methods for directly quantifying single-cell proteomes could provide a more functionally relevant base to interpret cell types and cell-type-specific responses to environments. An accurate determination of protein production in a cell could be useful in studying an array of physiological states or even in treating various diseases, as well as in drug development. Accordingly, additional and better systems for accurately quantifying protein production in a single cell are needed.

**SUMMARY OF THE INVENTION**

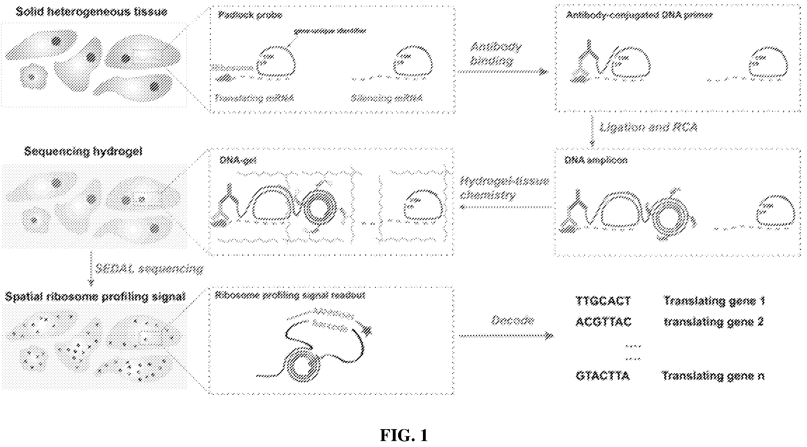

Methods, compositions, kits, and systems for *in situ* ribosome profiling *(i.e.,* profiling of RNAs of interest that are being actively translated) are described herein. Such systems represent a crucial technology to bridge the gap between the transcriptome and the proteome of a cell by quantifying/profiling actively translated mRNAs. A strategy based on proximity ligation is described herein to achieve specific and high-throughput characterization of RNA translation status *in situ* as an estimation of single-cell proteomes. The present system utilizes agents that recognize a ribosome conjugated to an oligonucleotide probe that recognizes the sequences of corresponding probes annealed to mRNA molecules that are bound by ribosomes (FIG. 1). Briefly, the present system utilizes a probe or set of probes comprising unique molecular barcodes to identify a target sequence within an mRNA of interest. Subsequently, ribosomes are bound by an oligonucleotide sequence portion of a primer that is complementary to a portion of the ribosome, or by a ribosome-specific primary antibody that is bound by a secondary antibody conjugated to a DNA primer . The DNA primer conjugated to the oligonucleotide sequence complementary to a portion of the ribosome, or to the secondary antibody (and thus bound to the ribosome) is ligated to the probe. Using the probe as a primer, the mRNA of interest is amplified by rolling circle amplification to yield a measurable sequence signal. Additionally, the antibodies are detectable *via* antibody staining to provide spatial information. By labeling ribosomes and localizing mRNAs of interest, precise spatial information of actively translated mRNAs can be extracted by means of highly selective RNA-ribosome colocalization, an important readout for understanding the spatiotemporal patterns of translation in both health and disease. Interactions between the ribosome and mRNA can be probed to determine the translation of mRNAs of interest.

Thus, in one aspect, the present disclosure provides methods for profiling RNAs being translated in a cell (see, for example, FIGs. 1, 3, and 6). In the methods disclosed herein, a cell may be contacted with one or more pairs of probes or sets of probes, which are described further herein and may be used to amplify (*e.g.,* by rolling circle amplification) RNAs that are actively being translated by a ribosome to produce one or more concatenated amplicons. The one or more concatenated amplicons may then be embedded in a polymeric matrix and sequenced to determine the identity of the transcripts and their location within the polymeric matrix (e.g., through SEDAL sequencing (Sequencing with Error-reduction by Dynamic Annealing and Ligation) as described further herein). Using the locations of the transcripts of interest, RNAs being translated in a cell may be profiled and spatiotemporal information obtained to improve the field's understanding of how translation location and timing affect cellular function in health and disease. The method may be useful for comparing RNA translation in, for example, a cell (or multiple cells) from diseased and healthy tissue samples; or for comparing RNA translation in, for example, a cell treated with an agent and an untreated cell.

Importantly, the method described herein may include two probes or three probes for profiling mRNA molecules during translation. Each method is useful for profiling mRNA molecules during translation, but the three-probe method has been demonstrated to produce a better signal-to-noise ratio.

In some embodiments, the present disclosure provides a two-probe method for profiling RNAs being translated in a cell comprising the steps of:

a) contacting the cell with one or more pairs of probes, wherein each pair of probes comprises a first probe and a second probe, wherein

i) the first probe comprises an oligonucleotide portion that is complementary to the second probe, an oligonucleotide portion that is complementary to an RNA of interest, and an oligonucleotide barcode sequence; and ii) the second probe comprises a portion that recognizes the ribosome and an oligonucleotide portion that is complementary to a portion of the first probe;

b) ligating the 5' end and the 3' end of the first probe together to produce a circular oligonucleotide;
c) performing rolling circle amplification to amplify the circular oligonucleotide using the second probe as a primer to produce one or more concatenated amplicons;
d) embedding the one or more concatenated amplicons in a polymeric matrix; and
e) sequencing the concatenated amplicons embedded in the polymeric matrix to determine the identity and location of each RNA of interest in the cell.

In some embodiments, the present disclosure provides a three-probe method for profiling RNAs being translated in a cell comprising the steps of:

a) contacting the cell with one or more sets of probes, wherein each set of probes comprises a first probe, a second probe, and a third probe, wherein

i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;
ii) the second probe comprises a portion that recognizes the ribosome and an oligonucleotide portion that is complementary to a portion of the first probe; and
iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe;

b) ligating the 5' end and the 3' end of the first probe together to produce a circular oligonucleotide;
c) performing rolling circle amplification to amplify the circular oligonucleotide using the third probe as a primer to produce one or more concatenated amplicons;
d) embedding the one or more concatenated amplicons in a polymeric matrix; and
e) sequencing the concatenated amplicons embedded in the polymeric matrix to determine the identity and location of each RNA of interest in the cell.

The methods, compositions, and systems described herein may be useful for studying RNA translation in tissues (e.g., developing tissues, normal tissues, diseased tissues), for diagnosing and treating various diseases, for research purposes, and for drug discovery. Thus, in one aspect, the present disclosure provides methods for diagnosing a disease or disorder in a subject (e.g., cancer). For example, the methods for profiling RNAs being translated described herein may be performed on a cell, or on multiple cells, taken from a subject (e.g., a subject who is thought to have or is at risk of having a disease or disorder, or a subject who is healthy or thought to be healthy). The expression of various RNAs of interest in the cell can then be compared to the expression of the same RNAs of interest in a non-diseased cell or a cell from a non-diseased tissue sample (e.g., a cell from a healthy individual, or multiple cells from a population of healthy individuals). Any difference in the RNA translation profile of the cell (including of a single RNA or of multiple RNAs of interest, e.g., a specific disease signature) relative to one or more non-diseased cells may indicate that the subject has the disease or disorder. RNA translation in one or more non-diseased cells (e.g., normal cells) may be profiled alongside expression in a diseased cell as a control experiment. RNA translation in one or more non-diseased cells (e.g., normal cells) may have also been profiled previously, and expression in a diseased cell may be compared to this reference data for a non-diseased cell.
In another aspect, the present disclosure provides methods for screening for an agent capable of modulating translation of one or more RNAs of interest. For example, the methods for profiling RNAs being translated described herein may be performed in a cell in the presence of one or more candidate agents. The expression of various RNAs of interest being translated in the cell (e.g., a normal cell, or a diseased cell) can then be compared to the expression of the same RNAs of interest in a cell that was not exposed to the one or more candidate agents. Any difference in the RNA translation profile relative to translation in the cell that was not exposed to the candidate agent(s) may indicate that translation (or potentially transcription) of the one or more RNAs of interest is modulated by the candidate agent(s). In some embodiments, a particular signature (e.g., of multiple RNAs of interest being translated) that is known to be associated with the treatment of a disease may be used to identify other agents capable of modulating translation in a desired manner and thus treating a disease. The methods described herein may also be used to identify drugs that have certain side effects, for example, by looking for specific RNA translation signatures when one or more cells is treated with a candidate agent or known drug. The methods described herein may also be used to identify research reagents or chemical probes that may be useful for studying the basic biology of cellular translation and protein production.

In another aspect, the present disclosure provides methods for treating a disease or disorder in a subject (*e.g.*, cancer). For example, the methods for profiling RNAs being translated described herein may be performed in a cell from a sample taken from a subject (*e.g.,* a subject who is thought to have or is at risk of having a disease or disorder). The profile of one or more RNAs being translated in the cell can then be compared to the translation status of the same RNAs of interest in a cell from a non-diseased tissue sample. A treatment for the disease or disorder may then be administered to the subject if any difference in the RNA translation profile relative to a non-diseased cell is observed. RNA translation in one or more non-diseased cells may be profiled alongside RNA translation in a diseased cell as a control experiment. RNA translation in one or more non-diseased cells (*e.g.,* normal cells) may have also been profiled previously, and translation in a diseased cell may be compared to this reference data for a non-diseased cell.

In one aspect, the present disclosure provides pairs of probes comprising a first probe (also referred to herein as the "padlock" probe) and a second probe (also referred to herein as the "primer" probe), wherein:

i) the first probe comprises an oligonucleotide portion that is complementary to the second probe, an oligonucleotide portion that is complementary to an RNA of interest, and an oligonucleotide barcode sequence; and
ii) the second probe comprises a portion that recognizes the ribosome and an oligonucleotide portion that is complementary to a portion of the first probe.

In another aspect, the present disclosure provides sets of oligonucleotide probes comprising a first probe (also referred to herein as the "padlock" probe), a second probe (also referred to herein as the "splint probe" or as the "blocked probe"), and a third probe (also referred to herein as the "primer probe"), wherein:

i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;
ii) the second probe comprises a portion that recognizes the ribosome and an oligonucleotide portion that is complementary to a portion of the first probe; and
iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe.

In another aspect, the present disclosure provides kits (*e.g.,* a kit comprising any of the pairs or sets of probes disclosed herein). In some embodiments, the kit comprises multiple pairs or sets of probes as described herein, each of which can be used to identify a specific RNA of interest that is being translated. The kits described herein may also include any other reagents or components useful in performing the methods described herein, including, but not limited to, cells, enzymes such as a ligase and/or a polymerase, amine-modified nucleotides, primary antibodies, secondary antibodies, buffers, reagents (including dyes, stains, and more), and monomers for making a polymeric matrix (*e.g.,* a polyacrylamide matrix).

In another aspect, the present disclosure provides systems for profiling RNA translation in a cell. In some embodiments, such a system comprises:

a) a cell;
b) one or more pairs of probes comprising a first probe and a second probe, wherein

i) the first probe comprises an oligonucleotide portion that is complementary to the second probe, an oligonucleotide portion that is complementary to an RNA of interest, and an oligonucleotide barcode sequence; and
ii) the second probe comprises a portion that recognizes the ribosome and an oligonucleotide portion that is complementary to a portion of the first probe;

c) a microscope; and
d) a computer.

In some embodiments, such a system comprises:

a) a cell;

b) one or more sets of probes comprising a first probe, a second probe, and a third probe, wherein

i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;

ii) the second probe comprises a portion that recognizes the ribosome and an oligonucleotide portion that is complementary to a portion of the first probe; and

iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe;

c) a microscope; and

d) a computer.

Any of the probes (*i.e.,* the pairs of probes or sets of probes) described herein may be used in the systems contemplated by the present disclosure.

It should be appreciated that the foregoing concepts, and additional concepts discussed below, may be arranged in any suitable combination, as the present disclosure is not limited in this respect. Further, other advantages and novel features of the present disclosure will become apparent from the following detailed description of various nonlimiting embodiments when considered in conjunction with the accompanying figures.

## BRIEF DESCRIPTION OF THE DRAWINGS

The following drawings form part of the present specification and are included to further demonstrate certain aspects of the present disclosure, which can be better understood by reference to one or more of these drawings in combination with the detailed description of specific embodiments presented herein.

**FIG. 1** is a schematic for a method of characterizing/profiling RNA translation *in situ.* A DNA-conjugated secondary antibody is used to recognize padlock probes residing on the same RNA in close proximity, enabling their amplification. Each padlock probe contains a unique barcode encoding the identity of the RNA site. The barcode information is amplified by rolling circle amplification and decoded by SEDAL *in situ* sequencing.

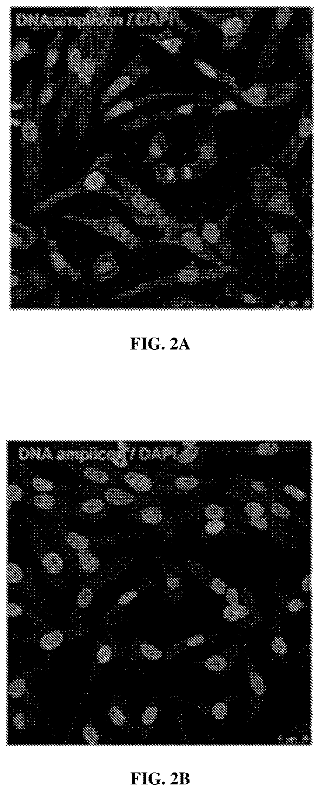

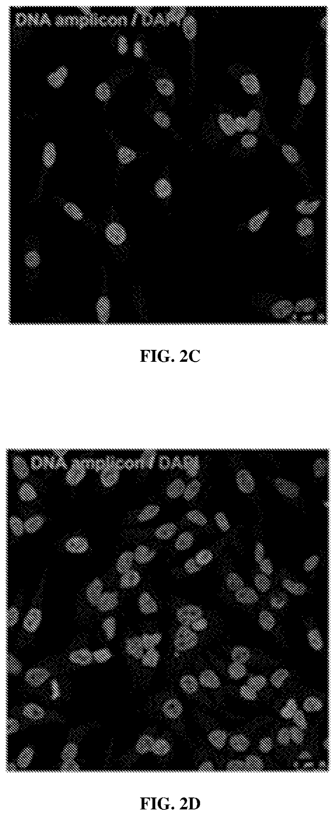

**FIGs. 2A-2D** are images of single-cell profiling of *in situ* RNA translation. **FIG. 2A** shows detection of actively transcribed beta-actin (ACTB) RNAs compared to negative controls in **FIG. 2B** (no primary antibody), **FIG. 2C** (secondary antibody with no DNA conjugated), and **FIG. 2D** (no secondary antibody).

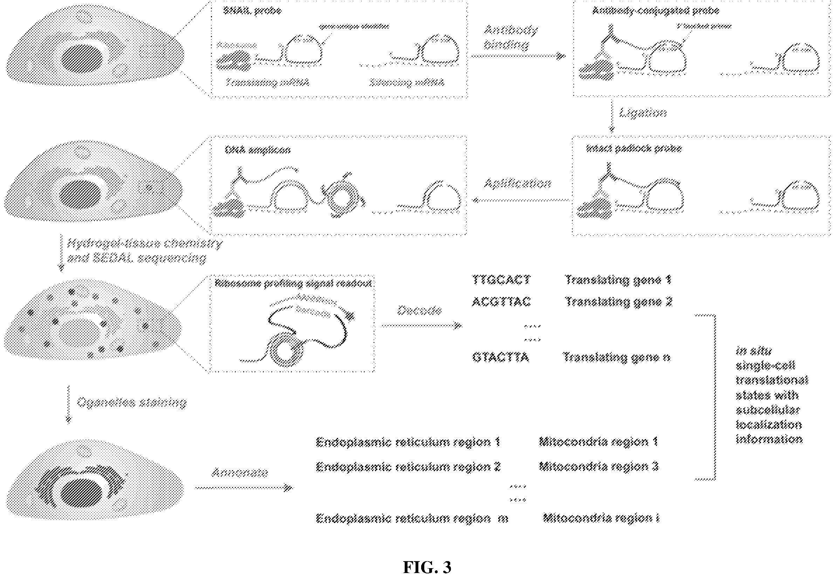

**FIG. 3** is a schematic of an *in situ* ribosome profiling method using ribosomal antibodies and three-part DNA probes. A DNA-conjugated secondary antibody is used to recognize padlock probes residing on the same RNA in close proximity, enabling ligation. Each padlock probe contains a unique barcode encoding the identity of the RNA site. The barcode sequence is amplified by rolling circle amplification to yield optimally measurable signals. The method is compatible with immunofluorescence staining to label subcellular organelles (e.g., the endoplasmic reticulum and mitochondria).

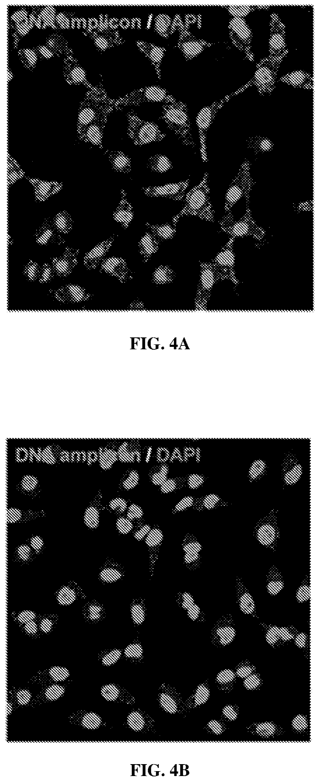

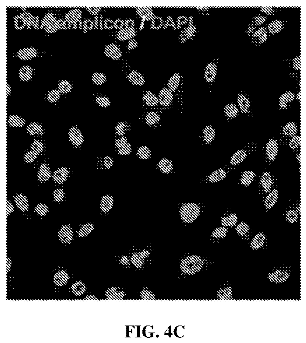

**FIGs. 4A-4C** are images of ribosomal protein antibody-based *in situ* ribosome profiling using an anti-40S ribosomal protein S3 (RPS3) antibody. **FIG. 4A** shows an *in situ* ribosome profiling signal for ACTB. **FIG. 4B** shows a negative control ribosome profiling signal using IgG in place of the anti-RPS3 antibody. **FIG. 4C** shows an *in situ* ribosome profiling signal for the non-coding RNA metastasis associated lung adenocarcinoma transcript 1 (MALAT1).

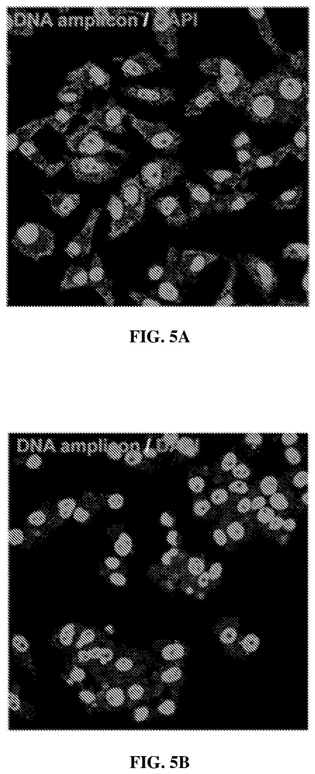

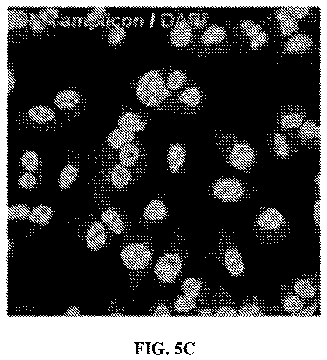

**FIGs. 5A-5C** are images of ribosomal protein antibody-based *in situ* ribosome profiling using an anti-60S ribosomal protein L4 (RPL4) antibody. **FIG. 5A** shows an *in situ* ribosome profiling signal for ACTB. **FIG. 5B** shows a negative control ribosome profiling signal using IgG in place of the anti-RPL4 antibody. **FIG. 5C** shows an *in situ* ribosome profiling signal for the non-coding RNA MALAT1.

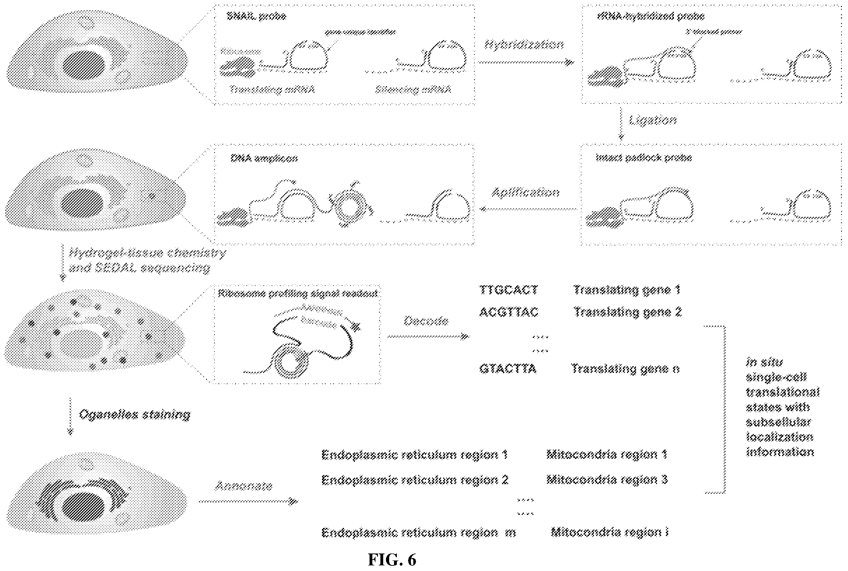

**FIG. 6** is a schematic of an *in situ* ribosome profiling method using three-part DNA probes that hybridize rRNAs and mRNAs. rRNA-hybridizing probes are used to recognize padlock probes residing on the same RNA in close proximity, enabling ligation. Each padlock probe contains a unique barcode encoding the identity of the RNA site. The barcode information is amplified by rolling circle amplification and decoded by SEDAL *in situ* sequencing. The method is compatible with immunofluorescence staining to label subcellular organelles (*e.g.*, the endoplasmic reticulum and mitochondria).

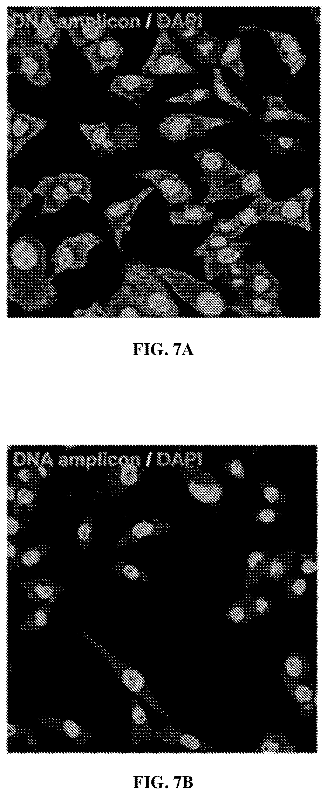

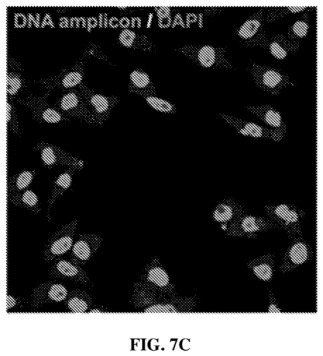

**FIGs. 7A-7C** are images of an rRNA-hybridized probe-based *in situ* ribosome profiling method. **FIG. 7A** shows an *in situ* ribosome profiling signal for ACTB. **FIG. 7B** shows a negative control ribosome profiling signal using probes without the portion that hybridizes to rRNA within the ribosome. **FIG. 7C** shows an *in situ* ribosome profiling signal for the non-coding RNA MALAT1.

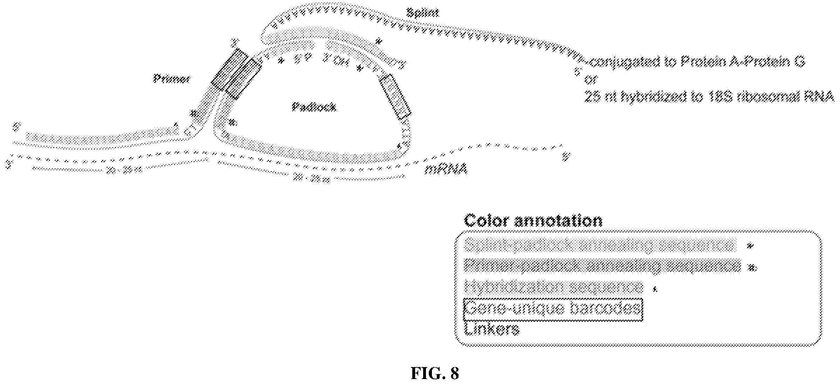

**FIG. 8** shows the detailed design of exemplary probes useful in the RNA profiling methods described herein.

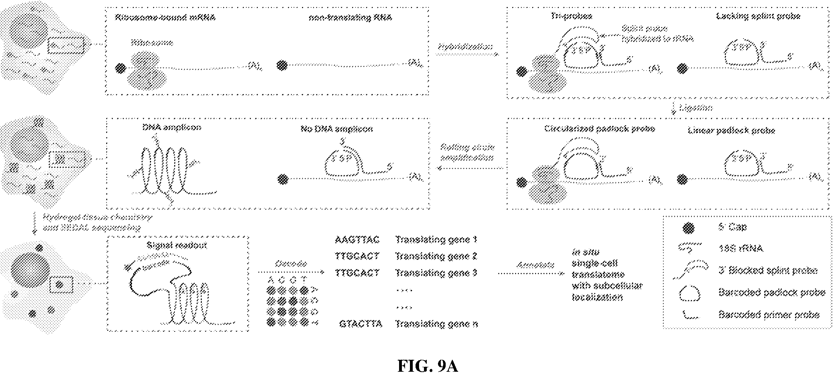

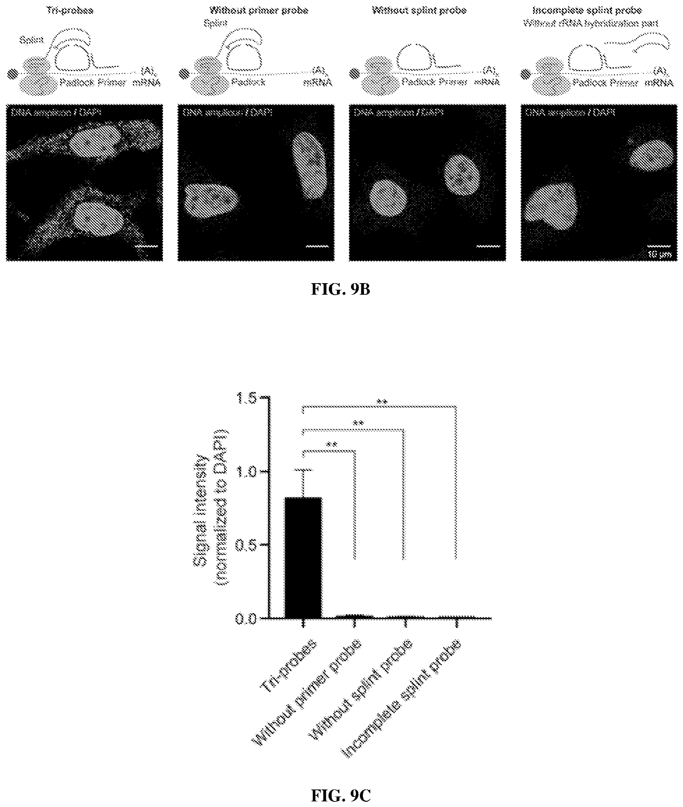

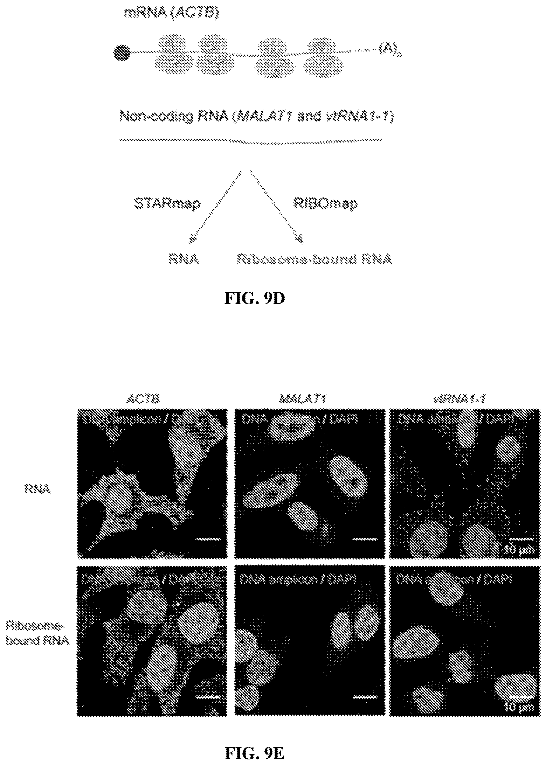

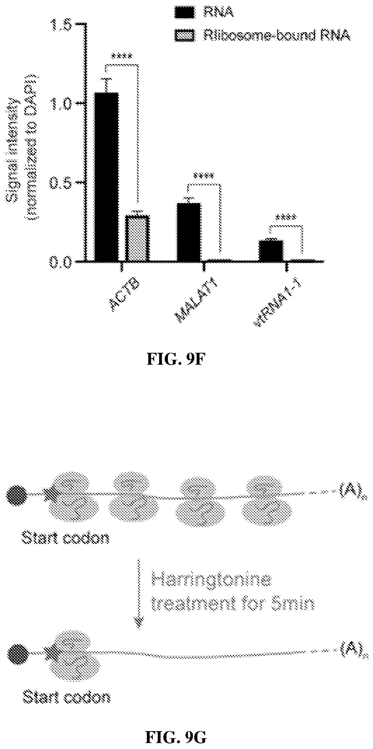

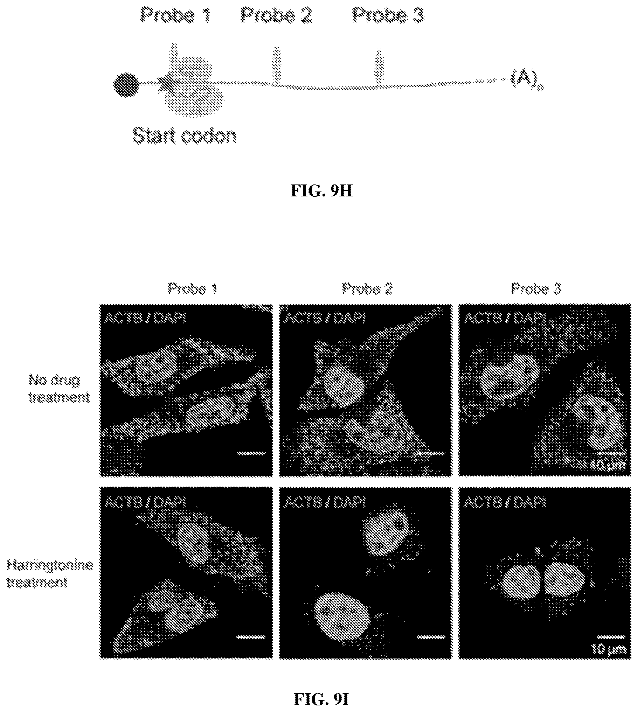

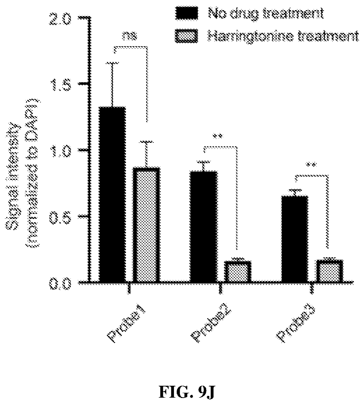

**FIGs. 9A-9J** show RIBOmap for *in situ* profiling of mRNA translation at subcellular resolution. **FIG. 9A** provides a schematic of RIBOmap. After the sample (cell line or tissue sample) is prepared, a pair of barcoded padlock probes and primers are hybridized to a targeted intracellular RNA, and the splint probe is hybridized to ribosomes via 18S rRNA. The splint probe is used as a template for the proximity ligation to circularize the padlock probe. The splint probe is designed with 3' Inverted dT modifications to block its extension during rolling circle amplification RCA. The intact padlock probe can then be amplified to generate amine-modified DNA amplicons *in situ.* Next, these DNA amplicons are copolymerized into a hydrogel via tissue-hydrogel chemistry for *in situ* mapping. The gene-specific identifier sequence (labeled "barcode") in the cDNA amplicons can then be read out through cyclic *in situ* sequencing with error reduction by dynamic annealing and ligation (SEDAL). **FIG. 9B** shows an example of the tri-probe strategy. (Left) Fluorescent images of tri-probe samples show the RIBOmap signal of *ACTB* mRNA in HeLa cells. (Middle) Fluorescent images of negative control samples without the primer or splint probe show minimum DNA amplicon signal. (Right) Fluorescent images of the control sample using splint probes without the rRNA hybridization segment show no DNA amplicon signal. Scale bar, 10 $\mu$m. **FIG. 9C** shows quantification of the DNA amplicon signal intensity normalized to the DAPI signal in FIG. 9B. Error bars, standard deviation. $n$ = 3 images per condition. Student's t-test, **P < 0.01. **FIG. 9D** provides a schematic representation of RIBOmap signal verification by targeting mRNA (*ACTB*) and non-coding RNA (*MALAT 1* and *vtRNA1-1*). **FIG. 9E** provides fluorescent images showing RNA detection results by STARmap and translation of RNA detection results by RIBOmap. Scale bar, 10 $\mu$m. **FIG. 9F** shows quantification of the DNA amplicon signal intensity that was normalized to the DAPI signal shown in FIG. 9E. Error bars, standard deviation. $n$ = 3 images per condition. Student's t-test, ****$P$ < 0.0001. **FIG. 9G** provides a schematic of translational regulation by Harringtonine. **FIG. 9H** shows three pairs of SNAIL probes targeting different sites of *ACTB* mRNA. Probe pair 1 is near the start codon, while Probe pair 2 and Probe pair 3 are 115 nt and 405 nt downstream of the start codon in the CDS region, respectively. **FIG. 9I** provides fluorescent images showing the RIBOmap signal of 3 sets of probes targeted to different regions *of ACTB* mRNA in HeLa cells before and after Harringtonine treatment. Scale bar, 10 $\mu$m. **FIG. 9J** shows quantification of the RIBOmap signal intensity that was normalized to the DAPI signal shown in FIG. 9I. Error bars, standard deviation. n = 3 images per condition. Student's t-test, **$P$ < 0.01.

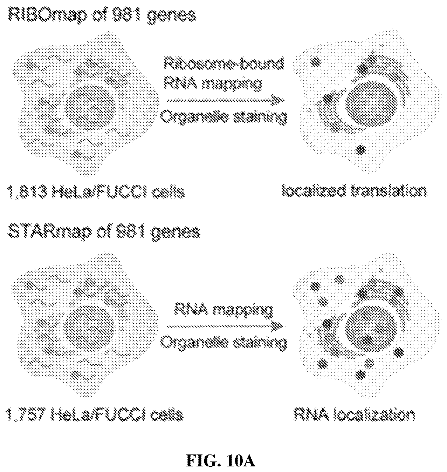

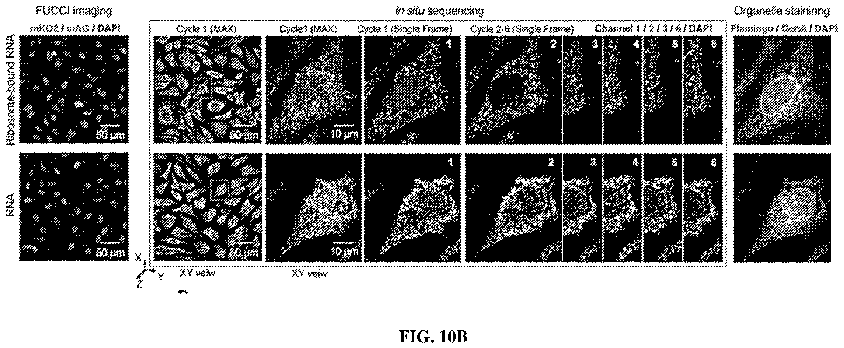

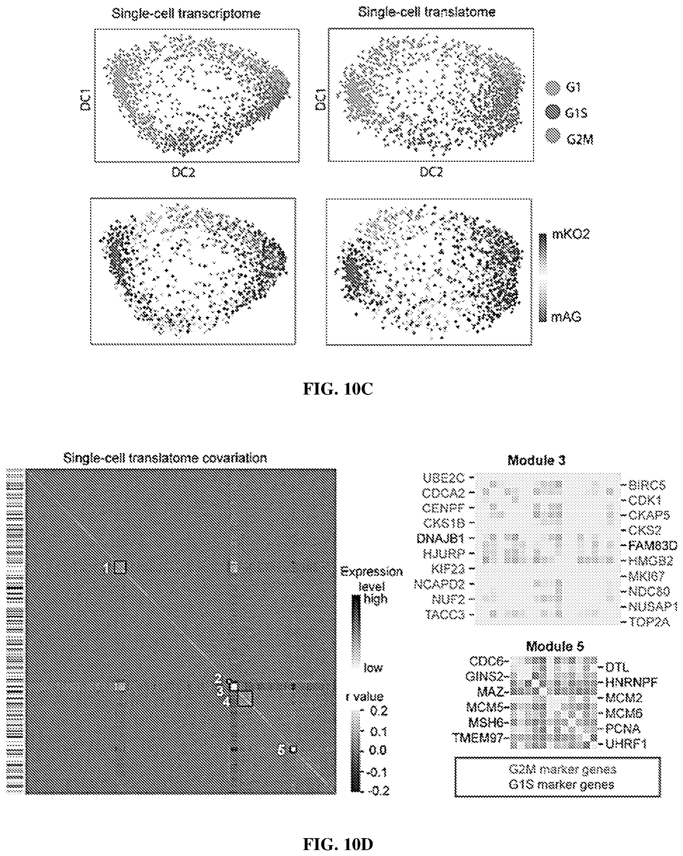

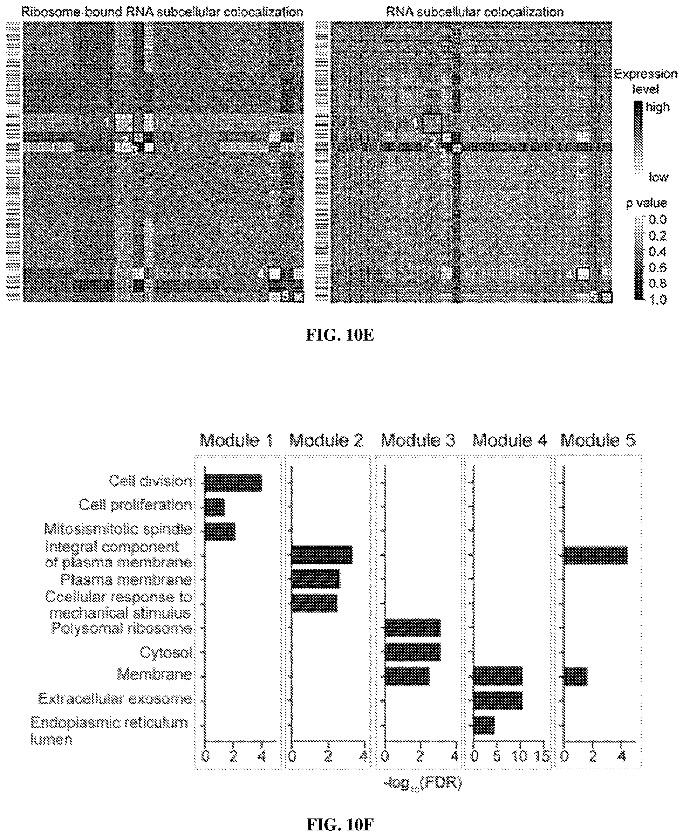

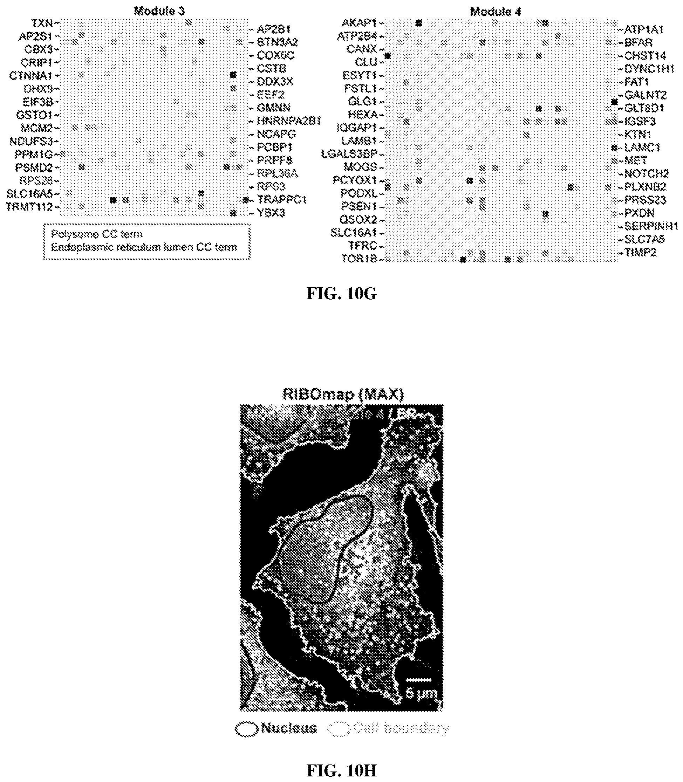

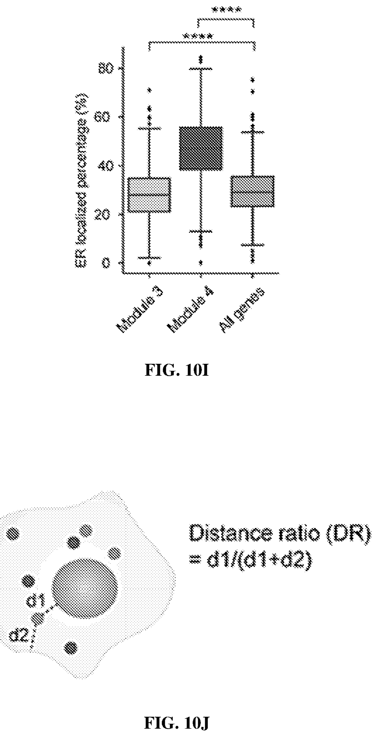

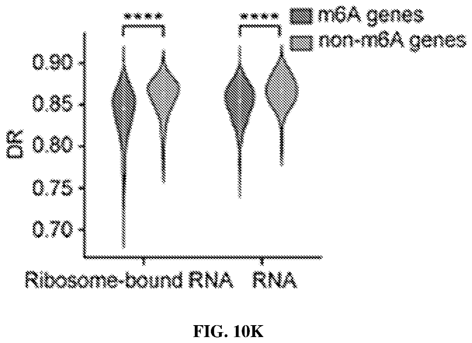

**FIGs. 10A-10K** show that RIBOmap simultaneously measures the subcellular translation of 981 genes in human HeLa cells. **FIG. 10A** provides schematics of RIBOmap detection in HeLa/FUCCI cells to measure localized mRNA translation. RIBOmap reads out ribosome-bound mRNA signals, and STARmap detects the targeted RNA. Organelle staining was combined in the procedure to visualize nucleus, ER, and cell morphology. **FIG. 10B** provides representative images showing the simultaneous mapping of FUCCI fluorescence signal, cDNA amplicons, and organelle staining signal in HeLa cells. Left, FUCCI fluorescence imaging results of the cells for RIBOmap (top) and STARmap (bottom). The fluorescent signals indicate the cell cycle stage of the HeLa cells. Middle, Representative images showing the maximum-intensity projections (MAX) of the first sequencing cycle for 981 genes with zoomed-in views of a representative cell and single-frame views of the representative cell across 6 sequencing cycles. Right: Zoomed-in view of the organelle staining signal of the representative cell. Alexa Fluor 594 conjugate of concanavalin A (ConA) staining of ER, flamingo fluorescent staining of cell morphology, and DAPI staining of the nucleus are shown according to the legend provided. **FIG. 10C** shows diffusion map embeddings of cell-cycle stage clusters determined using the single-cell expression profile of 41 cell-cycle marker genes for STARmap (upper left) and RIBOmap (upper right) measurements along with the corresponding single-cell protein fluorescence profiles of the monomeric Kusabira-Orange 2 (mKO2)-hCDT1 and monomeric Azami-Green (mAG)-hGEN FUCCI cell-cycle markers (STARmap, bottom left; RIBOmap, bottom right). **FIG. 10D** shows a single-cell translatome covariation matrix showing the pairwise Pearson's correlation coefficients of the cell-to-cell variation across 981 measured genes, shown together with their averaged expression levels. Five strongly correlating modules of genes are indicated by the gray boxes in the matrix and two of them, Modules 3 and 5, are enlarged on the right. The genes involved in translation complexes and identified as cell cycle markers are annotated in the enlarged matrix. **FIG. 10E** shows a matrix of the pairwise colocalization p-values describing the degree to which the reads of two genes tend to co-exist in a sphere of 3-um radius in the same cell in RIBOmap results (left) and STARmap results (right), shown together with the expression level of these genes. The STARmap matrix uses the same order of genes as the RIBOmap matrix. Five strongly correlating modules of genes are indicated by the gray boxes in the matrix. **FIG. 10F** shows bar plots visualizing the most significantly enriched GO terms (maximum 3) in each of the five gene modules. **FIG. 10G** shows an enlarged matrix of gene Module 3 and gene Module 4. **FIG. 10H** shows the spatial distribution of RIBOmap signals of Module 3 and Module 4 genes, overlaid on the ER image. The images are MAXs of the gene dots and ER signals. **FIG. 10I** shows quantification of the ER-localized percentage of Module 3 genes, Module 4 genes, and all the detected genes. Wilcoxon signed-rank test, ****$P$ < 0.0001. **FIG. 10J** provides a diagram showing the calculation of spatial parameter Distance Ratio (DR). **FIG. 10K** provides violin plots for the ribosome-bound RNA DR and RNA DR values for m$^6$A

genes and non-m$^6$A genes. Wilcoxon signed-rank test, ****$P < 0.0001$.

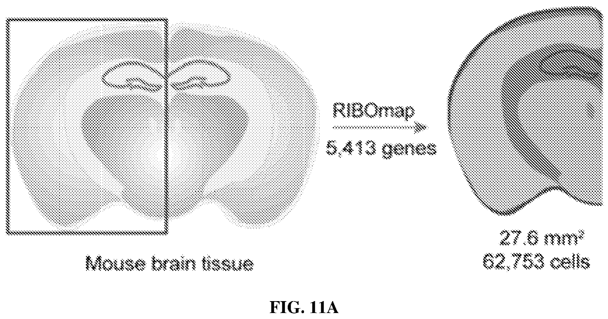

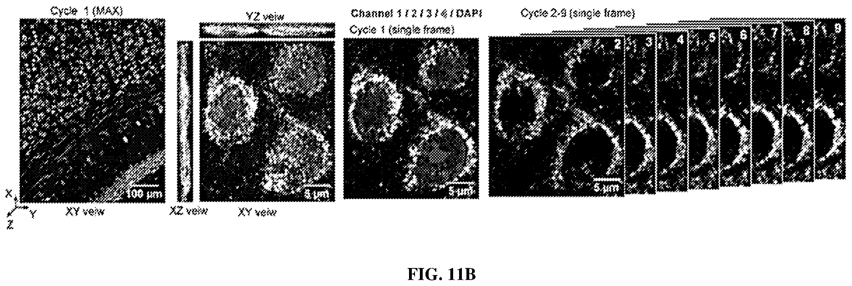

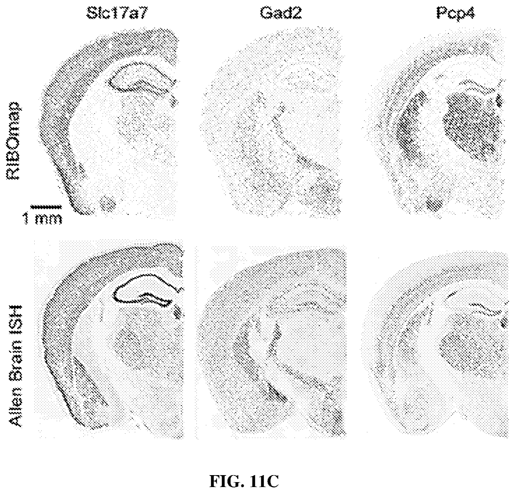

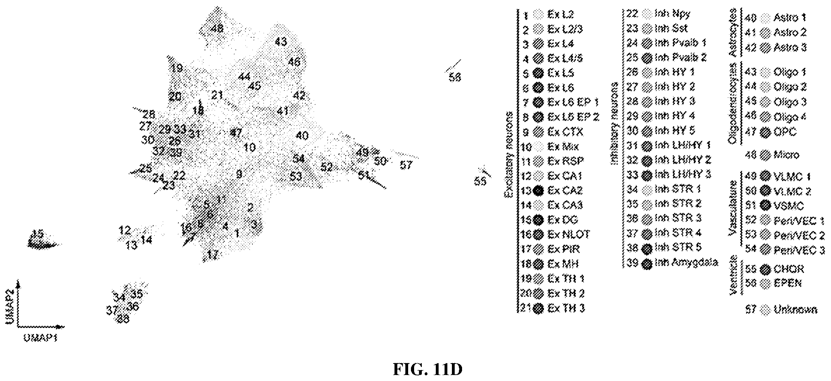

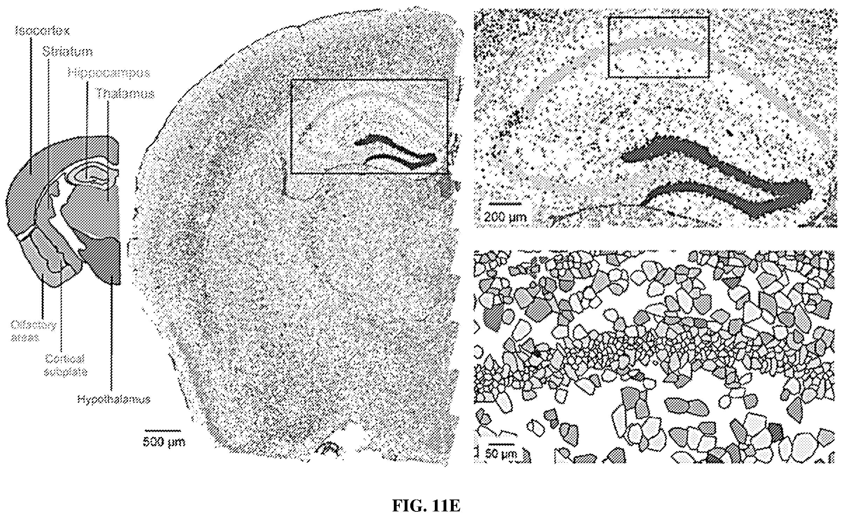

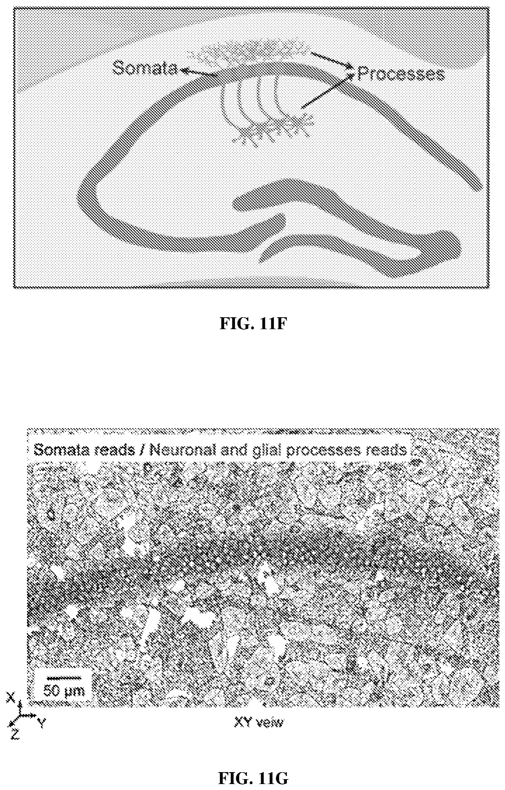

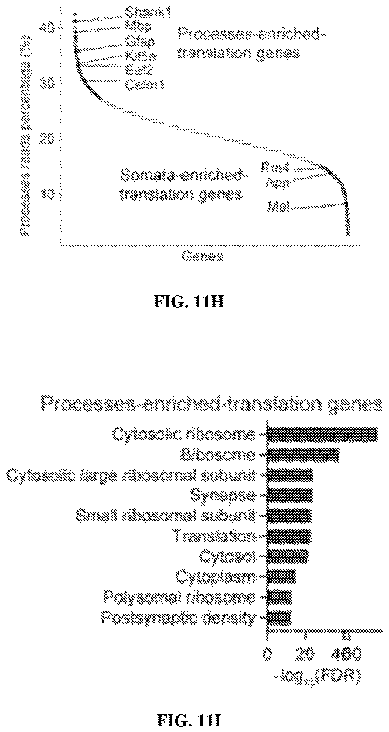

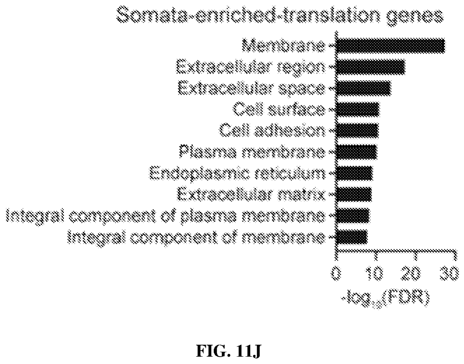

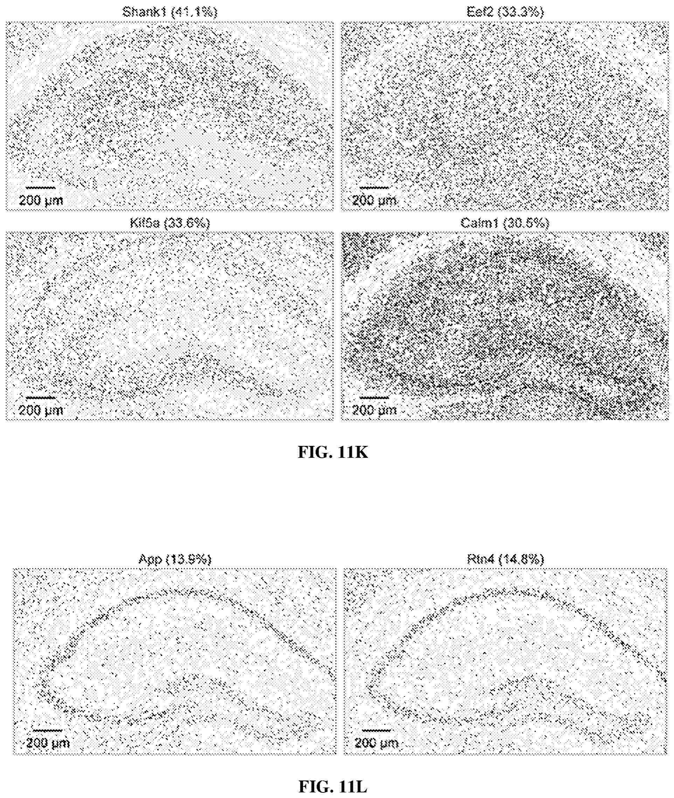

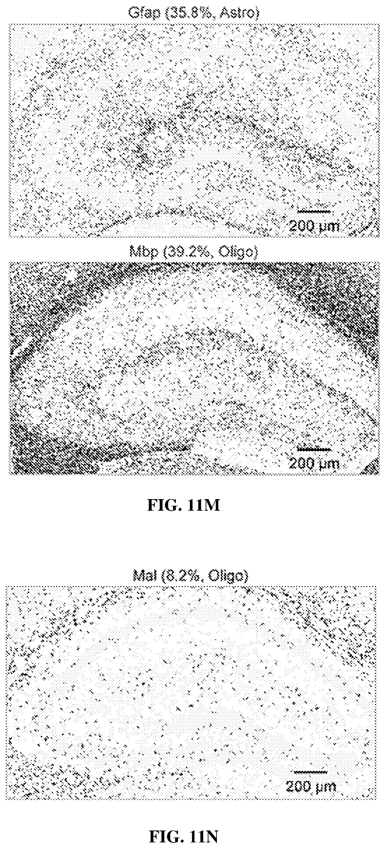

**FIGs. 11A-11N** show spatially resolved single-cell translatome profiling of 5,413 genes in the mouse brain. **FIG. 11A** provides a diagram of the targeted mouse coronal hemibrain region (marked in a box) for RIBOmap. **FIG. 11B** provides representative images showing the measurements of localized translation of 5,413 genes by RIBOmap in a mouse coronal hemibrain slice. (Left) Maximum-intensity projection of the first sequencing round of 5,413 genes, showing all five channels simultaneously. Square, zoomed-in region. (Middle) Zoomed-in view of three cells showing the MAX view of the first sequencing round. (Right) Zoomed-in view of three cells showing the spatial arrangement of amplicons in a single z-frame across nine sequencing rounds. **FIG. 11C** provides RIBOmap images of three cell-type marker genes in comparison with the Allen Brain ISH images showing the expression patterns of the corresponding genes. **FIG. 11D** shows a Uniform Manifold Approximation and Projection (UMAP) plot visualization of translational profiles of 62,753 cells collected from mouse coronal hemibrain. 57 cell types were identified using Leiden clustering. **FIG. 11E** provides a representative spatial cell-type atlas. (Left) Schematic highlighting different brain regions of the imaged coronal mouse brain slice. (Middle) Representative spatial cell-type atlas in the imaged coronal hemibrain region using the same code of FIG. 11D. Scale bar, 500 $\mu$m. (Right) Zoomed-in sections in the hippocampus and CA1 region showing the different cell types and the interspace. Scale bar, 200 $\mu$m. **FIG. 11F** provides a scheme of a hippocampal slice showing the somata and processes of hippocampal neurons. **FIG. 11G** shows a section in the CA1 region showing somata reads and neuronal and glial processes reads. Scale bar, 50 $\mu$m. **FIG. 11H** shows processes reads percentage of individual genes in the 5,413-gene RIBOmap measurements, with genes rank-ordered based on their processes reads percentage. Nine example genes were labeled inset. **FIG. 11I** show the top 10 significantly enriched GO terms for processes-enriched-translation genes. **FIG. 11J** shows the top 10 significantly enriched GO terms for somata-enriched-translation genes. **FIGs. 11K-11L** show the spatial translation map of example processes-enriched-translation genes (FIG. 11K) and somata-enriched-translation genes (FIG. 11L) in the hippocampus region, showing somata reads and processes reads. **FIGs. 11M-11N** show the spatial translation map of glial cell marker gene examples of processes-enriched-translation genes (M) and somata-enriched-translation genes (N) in the hippocampus region, showing somata reads and processes reads.

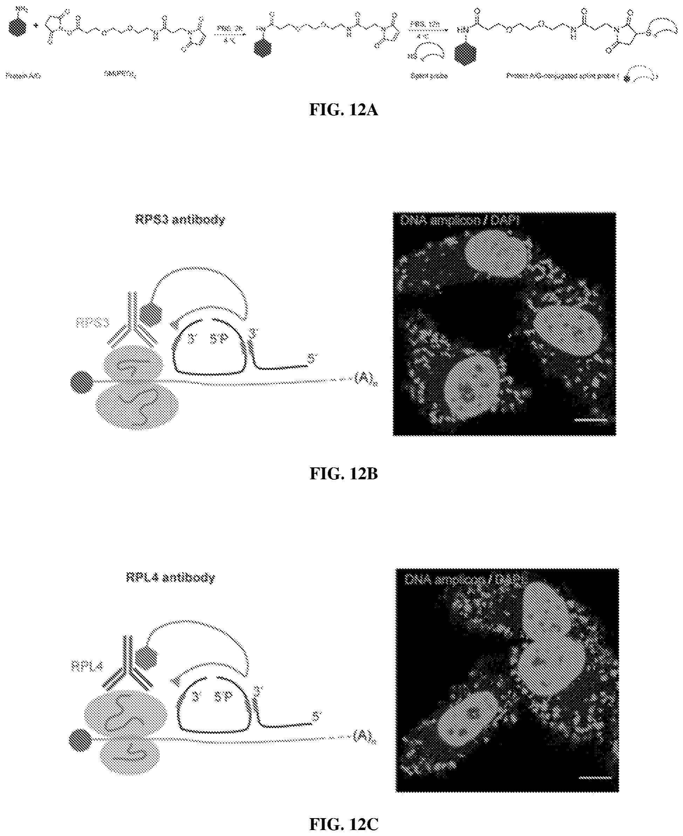

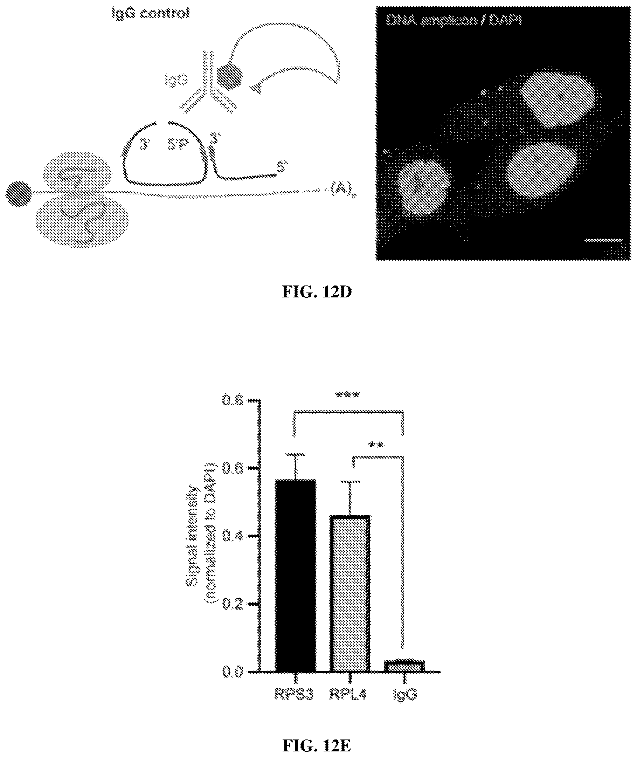

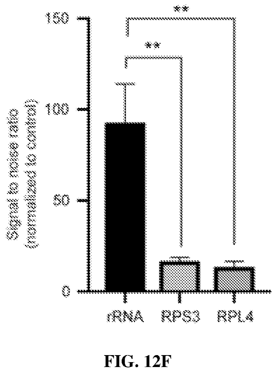

**FIGs. 12A-12F** show an example of the antibody-based RIBOmap strategy. **FIG. 12A** provides a synthetic scheme for splint probe-conjugated protein A/G preparation. **FIG. 12B** shows anti-ribosomal protein S3 (RPS3) antibody-based RIBOmap signal *of ACTB* mRNA in HeLa cells. RPS3 antibody binds to RPS3 protein in the small unit of the ribosome and brings in protein A/G-conjugated splint probe to the translating mRNA. The padlock probe targeted to the translating mRNA can then be ligated and amplified to generate amine-modified DNA amplicons *in situ*. **FIG. 12C** shows anti-ribosomal protein L4 (RPL4) antibody-based RIBOmap signal of *ACTB* mRNA in HeLa cells. **FIG. 12D** shows IgG control of the antibody-based RIBOmap signal of *ACTB* mRNA in HeLa cells. **FIG. 12E** shows quantification of the antibody-assisted RIBOmap signal intensity normalized to DAPI in FIGs. 12B-12D. Error bars, standard deviation. $n = 3$ images per condition. Student's t-test, **$P < 0.01$, ***$P < 0.001$. **FIG. 12F** shows the quantified comparison of the signal-to-noise ratio of rRNA probe-based RIBOmap strategy and antibody-based RIBOmap strategy. Error bars, standard deviation. $n = 3$ images per condition. Student's t-test, **$P < 0.01$.

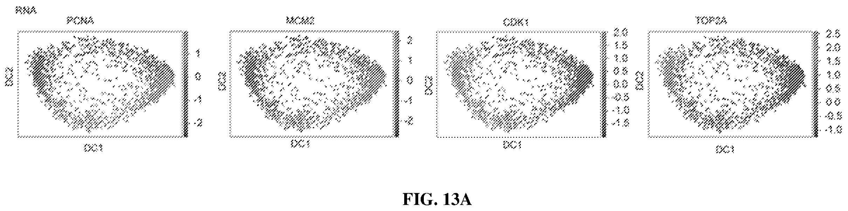

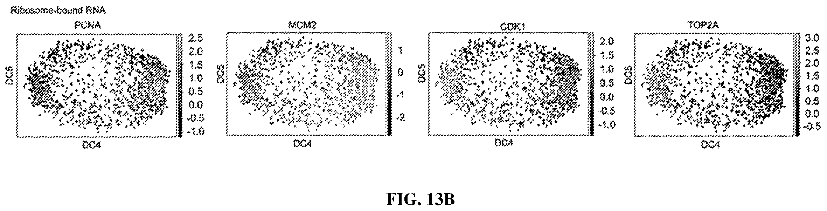

**FIGs. 13A-13B** show transcriptomic and translatomic dissection of cell-cycle-related variations. **FIG. 13A** shows cell-cycle marker gene expression on cell-cycle stage cluster embedding. **FIG. 13B** shows cell-cycle marker gene translation on cell-cycle stage cluster embedding.

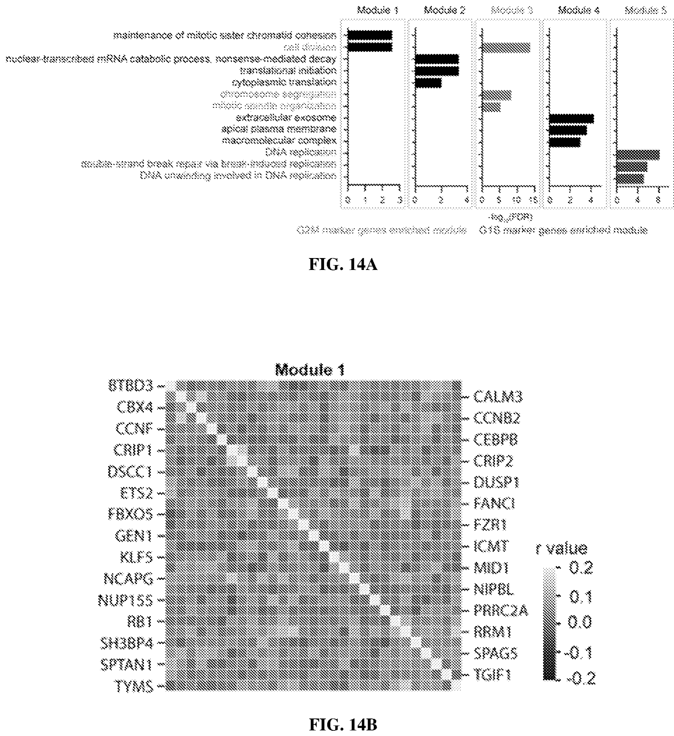

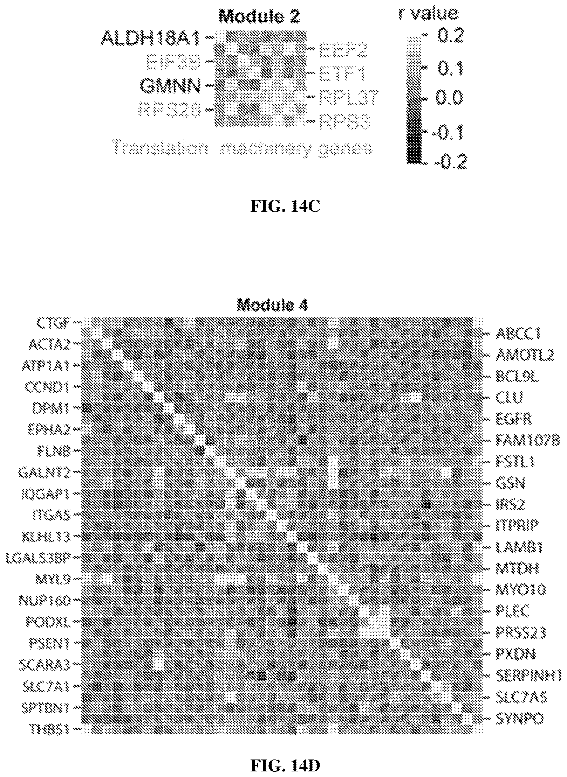

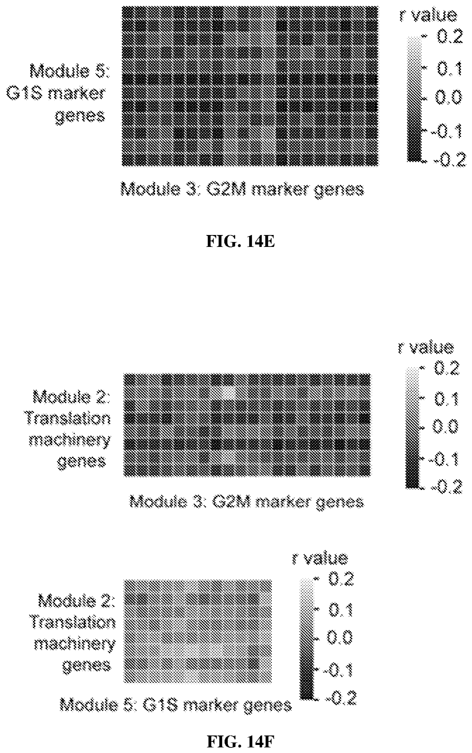

**FIGs 14A-14F** show covariation analysis of the single-cell translatome. **FIG. 14A** shows the top three significantly enriched GO terms in each of the five modules identified in the single-cell translatome covariation matrix. **FIGs. 14B-14D** show Enlarged Module 1 (FIG. 14B), Module 2 (FIG. 14C), and Module 4 (FIG. 14D) identified in the single-cell translatome covariation matrix. **FIG. 14E** provides a zoomed-in view showing the correlation between gene Module 3 (G2M marker genes) and gene Module 5 (G1S marker genes). **FIG. 14F** provides a zoomed-in view showing the correlation between cell-cycle marker gene modules and translation machinery gene modules.

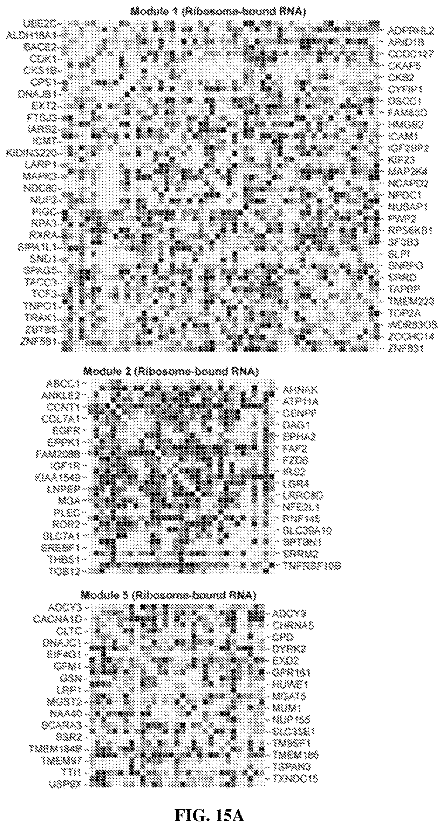

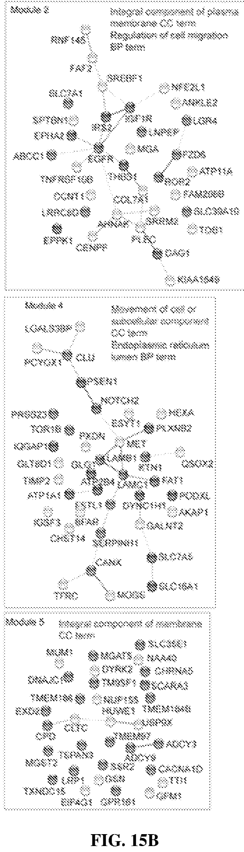

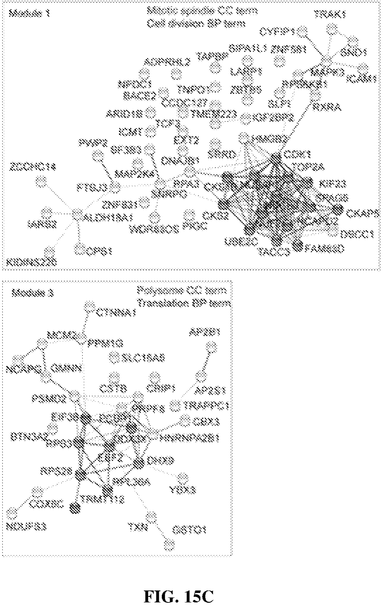

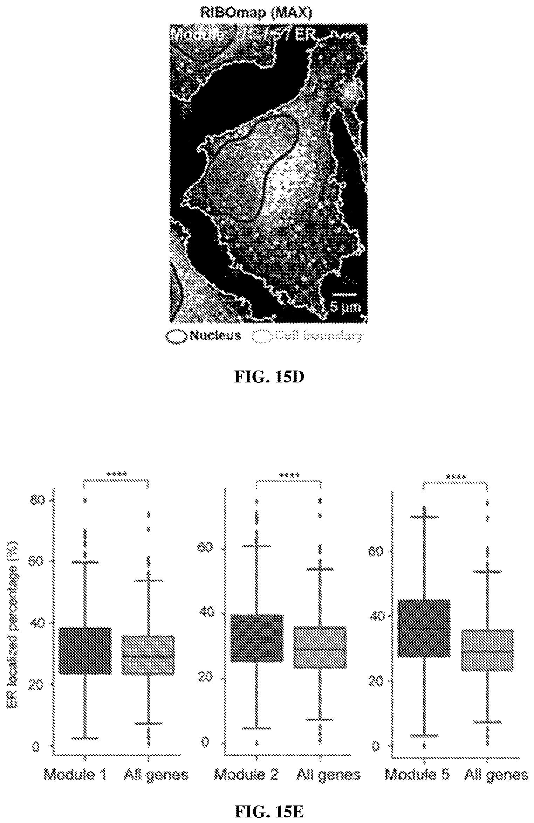

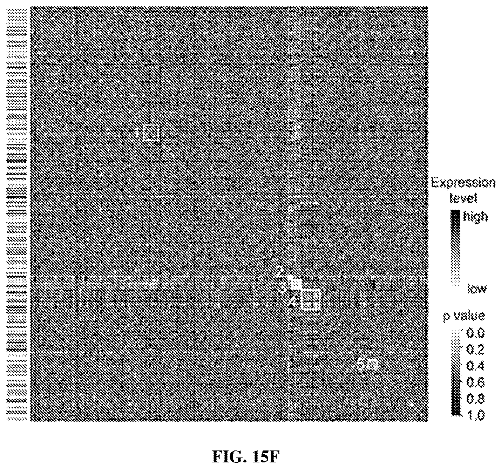

**FIGs. 15A-15F** show protein-protein interaction analysis and spatial distribution of co-localized gene modules. **FIG. 15A** shows String protein network analysis of the genes in Module 3 (left) and Module 4 (right). **FIG. 15B** shows the enlarged Module 1 (left), Module 2 (top right), and Module 5 (bottom right) in the ribosome-bound RNA subcellular colocalization matrix. **FIG. 15C** shows String protein network analysis of the genes in Module 1 (top), Module 2 (middle), and Module 5 (bottom). **FIG. 15D** shows the spatial distribution of RIBOmap signals of the Module 1, Module 2, and Module 5 genes, overlaid on the ER image. **FIG. 15E** shows quantification of the ER-localized percentage of Module 1 genes, Module 2 genes, Module 5 genes, and all the detected genes. Wilcoxon signed-rank test, ****$P < 0.0001$. **FIG. 15F** shows colocalization pairwise correlation p-value on the hierarchical clustering matrix of covariation analysis.

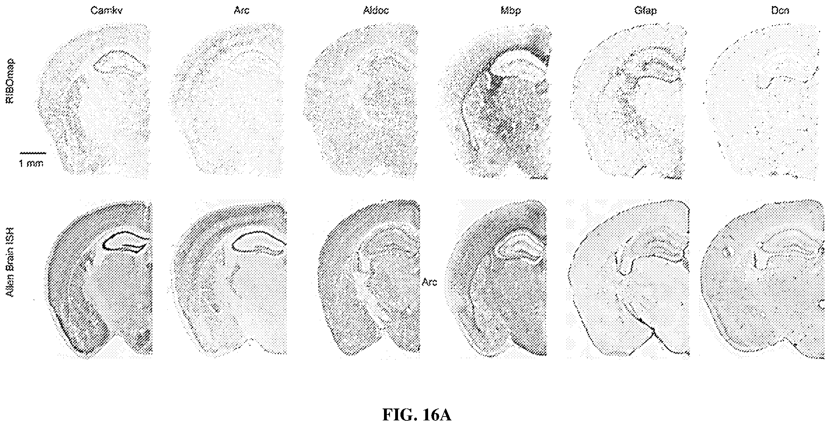

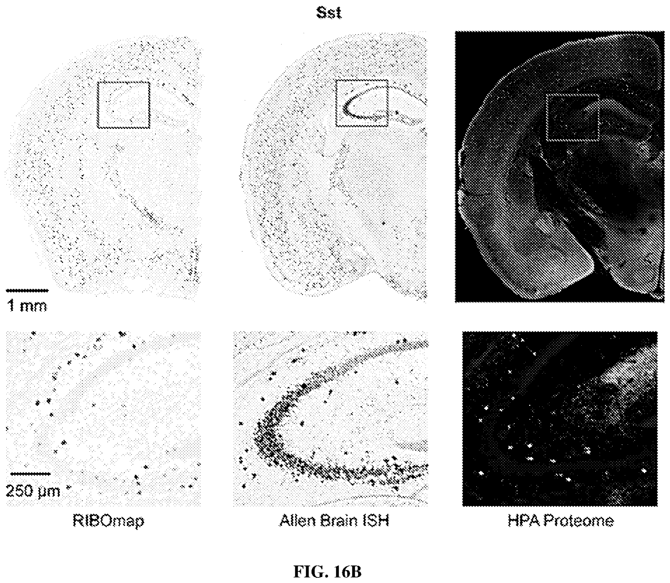

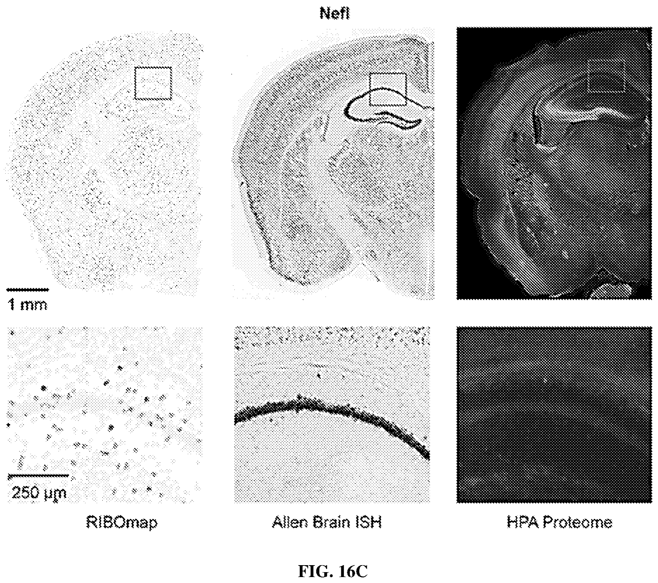

**FIGs 16A-16C** show cross-validation of RIBOmap measurements with *in situ* hybridization and immunofluorescence. **FIG. 16A** provides RIBOmap images of six example cell marker genes in comparison with the Allen Brain ISH images showing the expression patterns of the corresponding genes. **FIGs. 16B-16C** show translation profiles obtained from RIBOmap, *in situ* hybridization (ISH) data from the Allen Brain Atlas, and antibody-based immunofluorescence (IF) profiling from the HPA Brain Atlas of Sst (FIG. 16B) and Nefl (FIG. 16C).

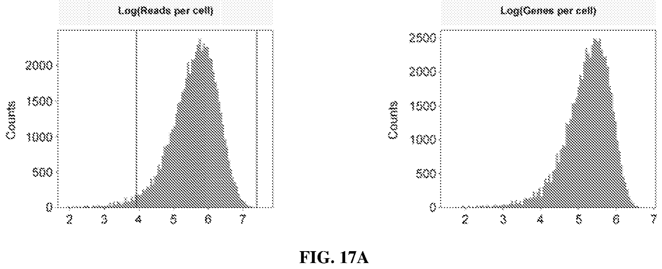

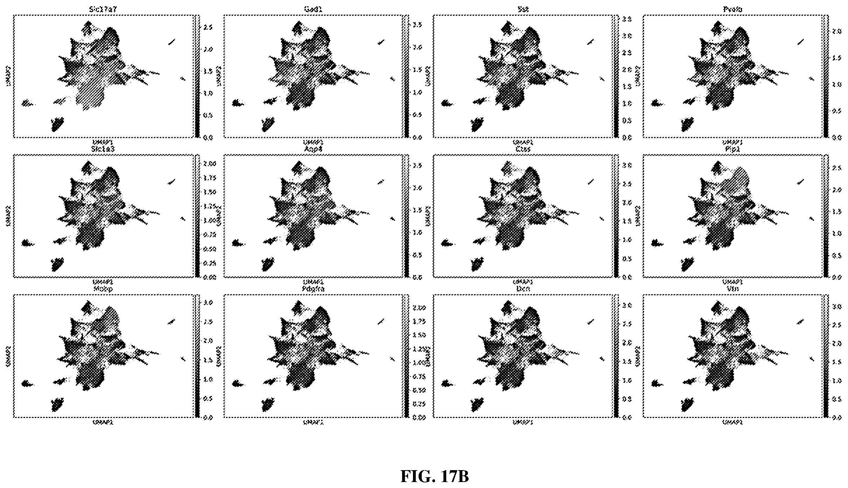

**FIGs. 17A-17B** show quality control and cell type marker expression level of RIBOmap mouse brain data. **FIG. 17A** provides a histogram showing the number of transcripts and genes in each cell after logarithmic transformation of the RIBOmap mouse brain dataset. Vertical lines represent the filtering thresholds estimated by median absolute deviation (MAD). **FIG. 17B** provides UMAPs showing expression levels of canonical markers for major cell types of mouse brain in the RIBOmap dataset.

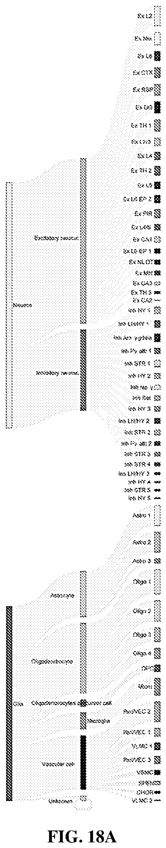

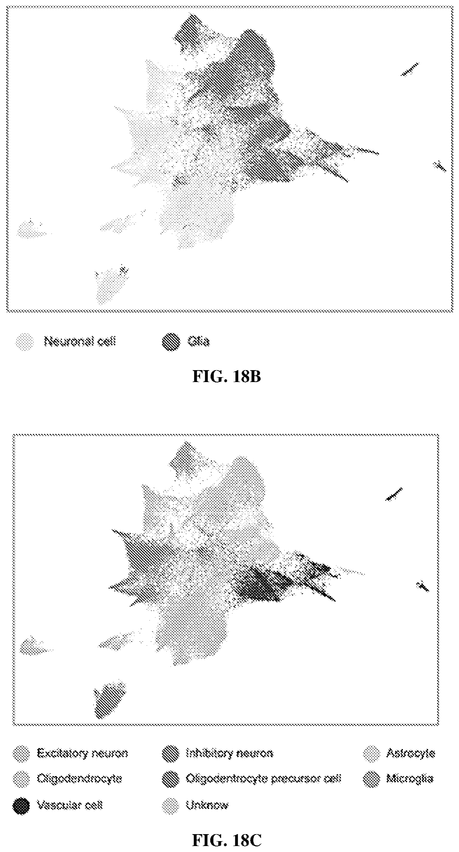

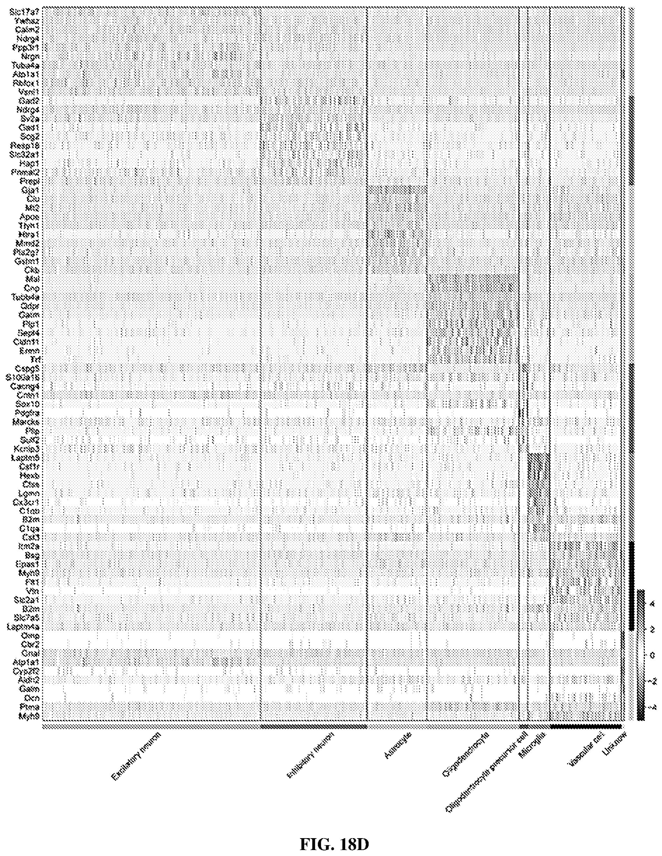

**FIGs. 18A-18D** show cell-type clustering based on single-cell translation profiling. **FIG. 18A** shows the hierarchical taxonomy of cell types showing level 1-3 cell clustering. **FIG. 18B** provides a Uniform Manifold Approximation and Projection (UMAP) plot visualization of the level 1 clustering to classify cells into neuronal cells and glial cells. FIG. 18C provides a UMAP plot visualization of the level 2 clustering to classify cells into excitatory neurons, inhibitory neurons, astrocytes, oligodendrocytes, oligodendrocyte precursor cells, microglia, and vascular cells. **FIG. 18D** provides gene expression heatmaps for representative markers aligned with level 2 clustering-identified cell types. Expression for each gene is z-scored across all genes in each cell.

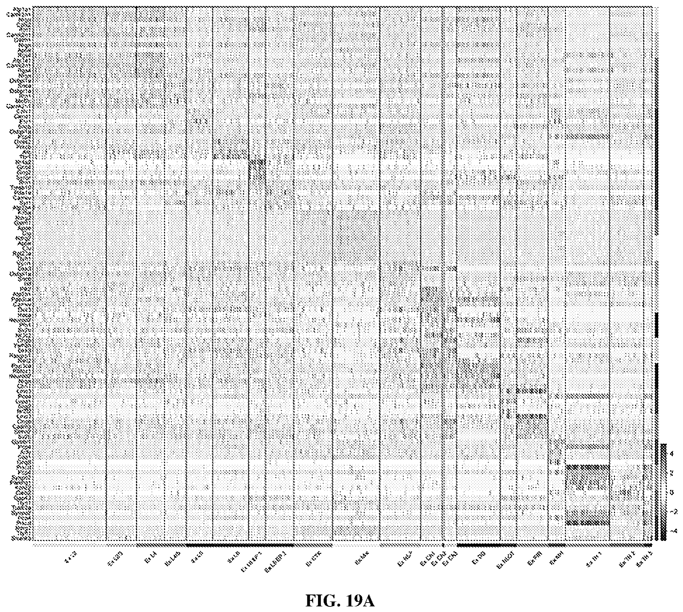

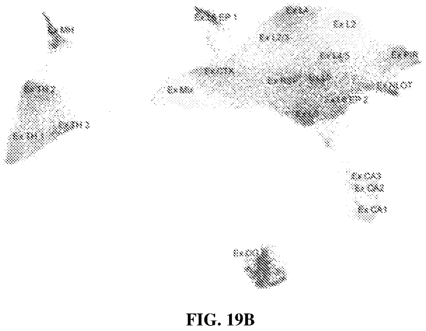

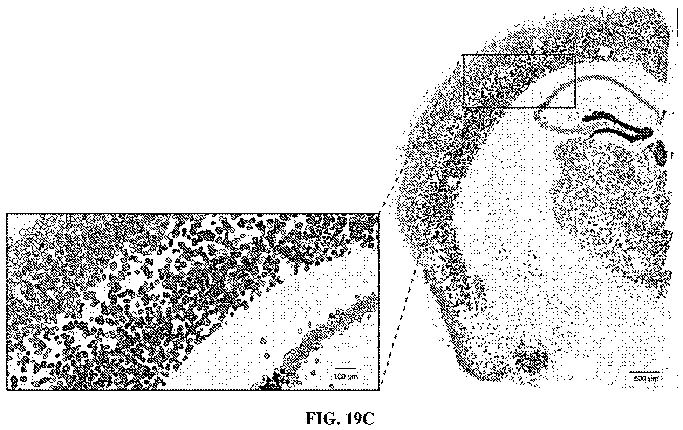

**FIGs. 19A-19C** provide gene expression and spatial maps of excitatory neurons. **FIG. 19A** provides gene expression heatmaps for representative markers aligned with excitatory neuron subtypes. Expression for each gene is z-scored across all genes in each cell. **FIG. 19B** shows UMAP plot visualization of the 21 excitatory neuron subtypes. **FIG. 19C** provides a spatial cell map of excitatory neuron subtypes in the imaged coronal hemibrain region with a zoomed-in view showing the spatial cell map in the cortex and hippocampus region.

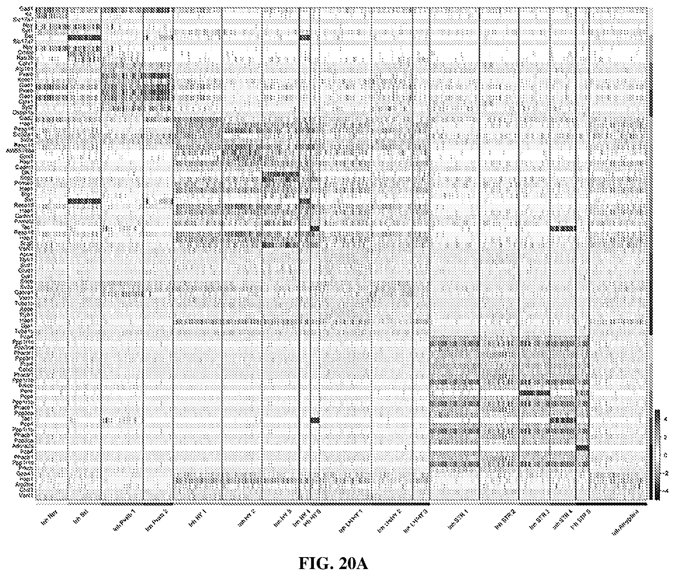

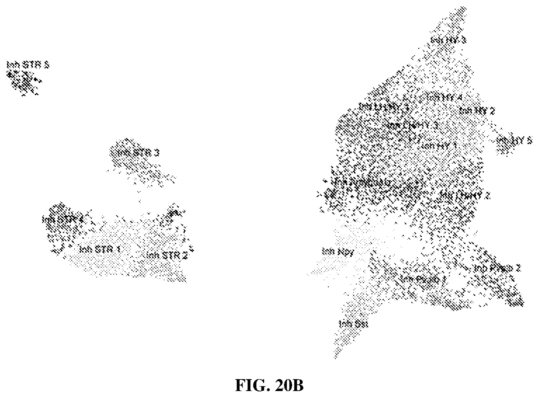

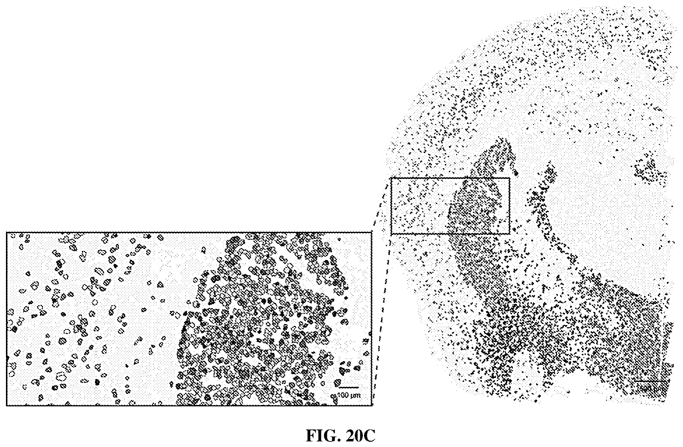

**FIGs. 20A-20C** provide gene expression and spatial maps of inhibitory neurons. **FIG. 20A** provides gene expression heatmaps for representative markers aligned with inhibitory neuron subtypes. Expression for each gene is z-scored across all genes in each cell. **FIG. 20B** shows UMAP plot visualization of the 18 inhibitory neuron subtypes. **FIG. 20C** shows the spatial cell map of inhibitory neuron subtypes in the imaged coronal hemibrain region with a zoomed-in view showing the spatial cell map in the cortex and striatum region.

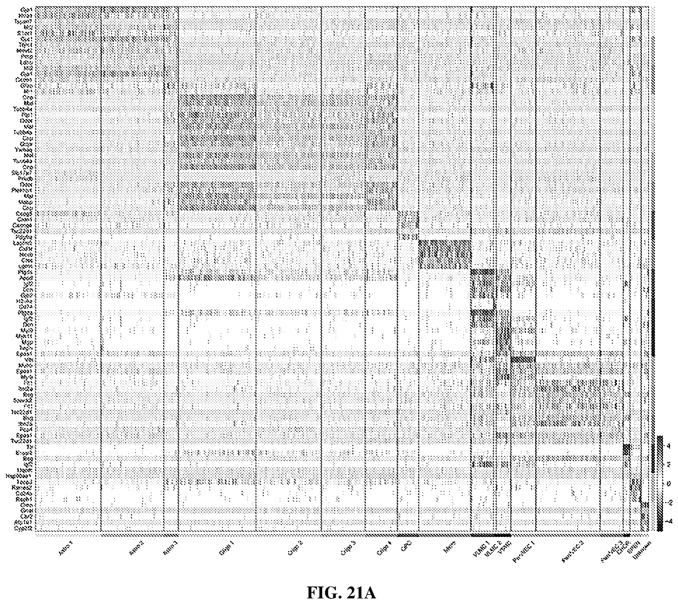

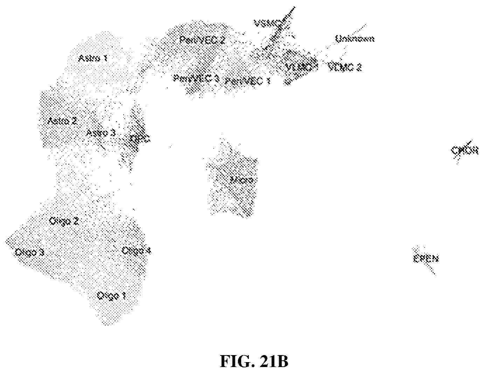

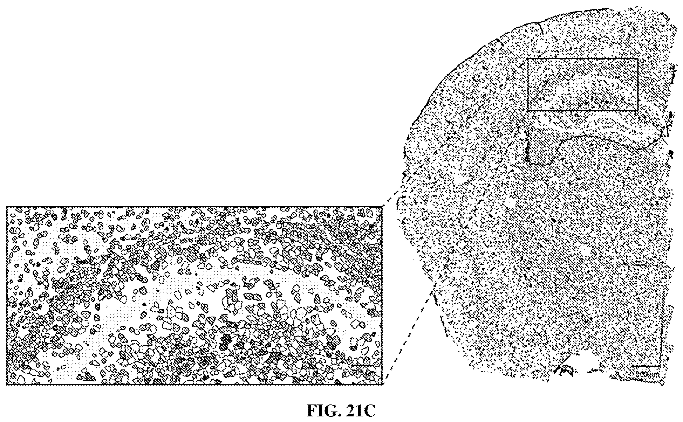

**FIGs. 21A-21C** provide gene expression and spatial maps of glial cells. **FIG. 21A** provides gene expression heatmaps for representative markers aligned with glial cell subtypes. Expression for each gene is z-scored across all genes in each cell. **FIG. 21B** shows UMAP plot visualization of the 18 glial cell subtypes. **FIG. 21C** provides a spatial cell map of glial cell subtypes in the imaged coronal hemibrain region with a zoomed-in view showing the spatial cell map in the cortex and hippocampus regions.

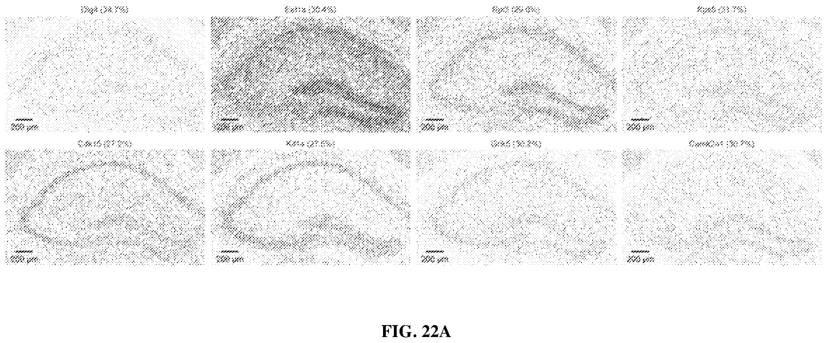

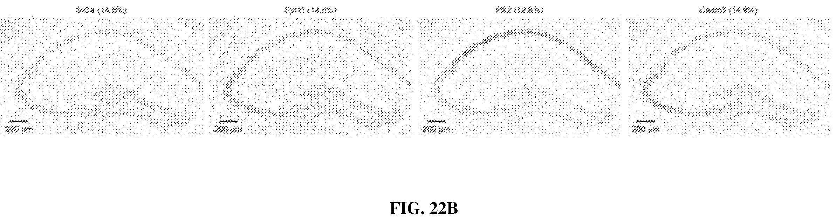

**FIGs. 22A-22D** provide spatial maps of examples of processes-enriched-translation genes. **FIGs. 22A-22B** show translation spatial maps of additional examples of processes-enriched-translation genes (FIG. 22A) and somata-enriched-translation genes (FIG. 22B) in the hippocampus region. Somata reads and processes reads are shown.

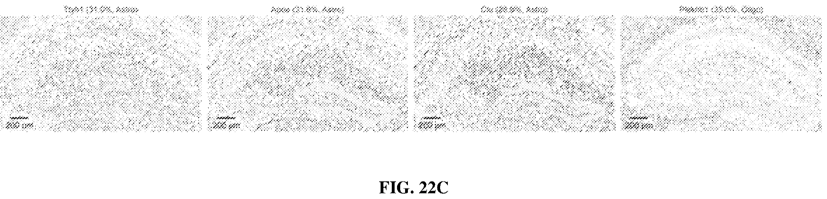

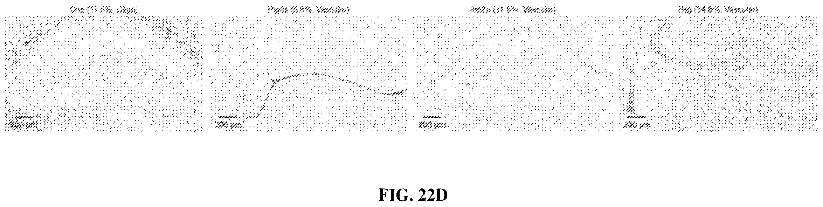

**FIGs. 22C-22D** provide translation spatial maps of additional glial cell marker gene examples of processes-enriched-translation genes (FIG. 22C) and somata-enriched-translation genes (FIG. 22D) in the hippocampus region. Somata reads and processes reads are shown.

## DEFINITIONS

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them unless specified otherwise.

The terms "administer," "administering," and "administration" refer to implanting, absorbing, ingesting, injecting, inhaling, or otherwise introducing a treatment or therapeutic agent, or a composition of treatments or therapeutic agents, in or on a subject.

The term "amplicon" as used herein refers to a nucleic acid (e.g., RNA) that is the product of an amplification reaction (i.e., the production of one or more copies of a genetic fragment or target sequence) or replication reaction. Amplicons can be formed artificially using, for example, PCR or other polymerization reactions. The term "concatenated amplicons" refers to multiple amplicons that are joined together to form a single nucleic acid molecule. Concatenated amplicons can be formed, for example, by rolling circle amplification (RCA), in which a circular oligonucleotide is amplified to produce multiple linear copies of the oligonucleotide as a single nucleic acid molecule comprising multiple amplicons that are concatenated.

An "antibody" refers to a glycoprotein belonging to the immunoglobulin superfamily. The terms antibody and immunoglobulin are used interchangeably. With some exceptions, mammalian antibodies are typically made of basic structural units each with two large heavy chains and two small light chains. There are several different types of antibody heavy chains, and several different kinds of antibodies, which are grouped together into different isotypes based on which heavy chain they possess. Five different antibody isotypes are known in mammals (IgG, IgA, IgE, IgD, and IgM, which perform different roles, and help direct the appropriate immune response for each different type of foreign object they encounter. The term "antibody" as used herein also encompasses antibody fragments and nanobodies, as well as variants of antibodies and variants of antibody fragments and nanobodies. In some embodiments, an antibody is administered as a treatment for a disease or disorder *(e.g.,* one that is associated with a change in the profile of RNAs being translated in a cell taken from a subject). In some embodiments, an antibody is conjugated to an oligonucleotide probe as described herein. In certain embodiments, the antibody binds to a ribosome (e.g., the antibody is an anti-40S ribosomal protein S3 (RPS3) antibody or an anti-60S ribosomal protein L4 (RPL4) antibody).

The term "cancer" (including cancers that may be studied, characterized, diagnosed, and/or treated using the methods described herein) refers to a class of diseases characterized by the development of abnormal cells that proliferate uncontrollably and have the ability to infiltrate and destroy normal body tissues. *See e.g.,* Stedman's Medical Dictionary, 25th ed.; Hensyl ed.; Williams & Wilkins: Philadelphia, 1990. Cancer is one example of a proliferative disease. Exemplary cancers include, but are not limited to, acoustic neuroma; adenocarcinoma; adrenal gland cancer; anal cancer; angiosarcoma (*e.g.*, lymphangiosarcoma, lymphangioendotheliosarcoma, hemangiosarcoma); appendix cancer; benign monoclonal gammopathy; biliary cancer (*e.g.,* cholangiocarcinoma); bladder cancer; breast cancer (*e.g.,* adenocarcinoma of the breast, papillary carcinoma of the breast, mammary cancer, medullary carcinoma of the breast); brain cancer (*e.g.,* meningioma, glioblastomas, glioma (*e.g.,* astrocytoma, oligodendroglioma), medulloblastoma); bronchus cancer; carcinoid tumor; cervical cancer (*e.g.,* cervical adenocarcinoma); choriocarcinoma; chordoma; craniopharyngioma; colorectal cancer (*e.g.,* colon cancer, rectal cancer, colorectal adenocarcinoma); connective tissue cancer; epithelial carcinoma; ependymoma; endotheliosarcoma (*e.g.,* Kaposi's sarcoma, multiple idiopathic hemorrhagic sarcoma); endometrial cancer (*e.g.,* uterine cancer, uterine sarcoma); esophageal cancer (*e.g.,* adenocarcinoma of the esophagus, Barrett's adenocarcinoma); Ewing's sarcoma; ocular cancer (*e.g.,* intraocular melanoma, retinoblastoma); familiar hypereosinophilia; gall bladder cancer; gastric cancer (*e.g.,* stomach adenocarcinoma); gastrointestinal stromal tumor (GIST); germ cell cancer; head and neck cancer (*e.g.,* head and neck squamous cell carcinoma, oral cancer (*e.g.,* oral squamous cell carcinoma), throat cancer (*e.g.,* laryngeal cancer, pharyngeal cancer, nasopharyngeal cancer, oropharyngeal cancer)); hematopoietic cancers (*e.g.,* leukemia such as acute lymphocytic leukemia (ALL) (*e.g.,* B-cell ALL, T-cell ALL), acute myelocytic leukemia (AML) (*e.g.,* B-cell AML, T-cell AML), chronic myelocytic leukemia (CML) (*e.g.,* B-cell CML, T-cell CML), and chronic lymphocytic leukemia (CLL) (*e.g.,* B-cell CLL, T-cell CLL)); lymphoma such as Hodgkin lymphoma (HL) (*e.g.,* B-cell HL, T-cell HL) and non-Hodgkin lymphoma (NHL) (*e.g.,* B-cell NHL such as diffuse large cell lymphoma (DLCL) (*e.g.,* diffuse large B-cell lymphoma), follicular lymphoma, chronic lymphocytic leukemia/small lymphocytic lymphoma (CLL/SLL), mantle cell lymphoma (MCL), marginal zone B-cell lymphomas (*e.g.,* mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (*i.e.,* Waldenstrom's macroglobulinemia), hairy cell leukemia (HCL), immunoblastic large cell lymphoma, precursor B-lymphoblastic lymphoma and primary central nervous system (CNS) lymphoma; and T-cell NHL such as precursor T-lymphoblastic lymphoma/leukemia, peripheral T-cell lymphoma (PTCL) (*e.g.,* cutaneous T-cell lymphoma (CTCL) (*e.g.,* mycosis fungoides, Sezary syndrome), angioimmunoblastic T-cell lymphoma, extranodal natural killer T-cell lymphoma, enteropathy type T-cell lymphoma, subcutaneous panniculitis-like T-cell lymphoma, and anaplastic large cell lymphoma); a mixture of one or more leukemia/lymphoma as described above; and multiple myeloma (MM)), heavy chain disease (*e.g.,* alpha chain disease, gamma chain disease, mu chain disease); hemangioblastoma; hypopharynx cancer; inflammatory myofibroblastic tumors; immunocytic amyloidosis; kidney cancer (*e.g.,* nephroblastoma *a.k.a.* Wilms' tumor, renal cell carcinoma); liver cancer (*e.g.,* hepatocellular cancer (HCC), malignant hepatoma); lung cancer (*e.g.,* bronchogenic carcinoma, small cell lung cancer (SCLC), non-small cell lung cancer (NSCLC), adenocarcinoma of the lung); leiomyosarcoma (LMS); mastocytosis (*e.g.,* systemic mastocytosis); muscle cancer; myelodysplastic syndrome (MDS); mesothelioma; myeloproliferative disorder (MPD) (e.g., polycythemia vera (PV), essential thrombocytosis (ET), agnogenic myeloid metaplasia (AMM) *a.k.a.* myelofibrosis (MF), chronic idiopathic myelofibrosis, chronic myelocytic leukemia (CML), chronic neutrophilic leukemia (CNL), hypereosinophilic syndrome (HES)); neuroblastoma; neurofibroma (*e.g.,* neurofibromatosis (NF) type 1 or type 2, schwannomatosis); neuroendocrine cancer (*e.g.,* gastroenteropancreatic neuroendoctrine tumor (GEP-NET), carcinoid tumor); osteosarcoma (*e.g.,* bone cancer); ovarian cancer (*e.g.,* cystadenocarcinoma, ovarian embryonal carcinoma, ovarian adenocarcinoma); papillary adenocarcinoma; pancreatic cancer (*e.g.,* pancreatic adenocarcinoma, intraductal papillary mucinous neoplasm (IPMN), Islet cell tumors); penile cancer (*e.g.,* Paget's disease of the penis and scrotum); pinealoma; primitive neuroectodermal tumor (PNT); plasma cell neoplasia; paraneoplastic syndromes; intraepithelial neoplasms; prostate cancer (*e.g.,* prostate adenocarcinoma); rectal cancer; rhabdomyosarcoma; salivary gland cancer; skin cancer (*e.g.,* squamous cell carcinoma (SCC), keratoacanthoma (KA), melanoma, basal cell carcinoma (BCC)); small bowel cancer (*e.g.,* appendix cancer); soft tissue sarcoma (*e.g.,* malignant fibrous histiocytoma (MFH), liposarcoma, malignant peripheral nerve sheath tumor (MPNST), chondrosarcoma, fibrosarcoma, myosarcoma); sebaceous gland carcinoma; small intestine cancer; sweat gland carcinoma; synovioma; testicular cancer (*e.g.,* seminoma, testicular embryonal carcinoma); thyroid cancer (*e.g.,* papillary carcinoma of the thyroid, papillary thyroid carcinoma (PTC), medullary thyroid cancer); urethral cancer; vaginal cancer; and vulvar cancer (*e.g.,* Paget's disease of the vulva).

A "cell," as used herein, may be present in a population of cells (*e.g.,* in a tissue, a sample, a biopsy, an organ, or an organoid). In some embodiments, a population of cells is composed of a plurality of different cell types. Cells for use in the methods of the present disclosure can be present within an organism, a single cell type derived from an organism, or a mixture of cell types. Included are naturally occurring cells and cell populations, genetically engineered cell lines, cells derived from transgenic animals, cells from a subject, etc. Virtually any cell type and size can be accommodated in the methods and systems described herein. In some embodiments, the cells are mammalian cells (*e.g.,* complex cell populations such as naturally occurring tissues). In some embodiments, the cells are from a human. In certain embodiments, the cells are collected from a subject (*e.g.*, a human) through a medical procedure such as a biopsy. Alternatively, the cells may be a cultured population (*e.g.,* a culture derived from a complex population, or a culture derived from a single cell type where the cells have differentiated into multiple lineages). The cells may also be provided *in situ* in a tissue sample.

Cell types contemplated for use in the methods of the present disclosure include, but are not limited to, stem and progenitor cells (*e.g.*, embryonic stem cells, hematopoietic stem cells, mesenchymal stem cells, neural crest cells, *etc.*), endothelial cells, muscle cells, myocardial cells, smooth and skeletal muscle cells, mesenchymal cells, epithelial cells, hematopoietic cells, lymphocytes such as T-cells (*e.g.,* Th1 T cells, Th2 T cells, ThO T cells, cytotoxic T cells) and B cells (*e.g.,* pre-B cells), monocytes, dendritic cells, neutrophils, macrophages, natural killer cells, mast cells, adipocytes, immune cells, neurons, hepatocytes, and cells involved with particular organs (*e.g.*, thymus, endocrine glands, pancreas, brain, neurons, glia, astrocytes, dendrocytes, and genetically modified cells thereof). The cells may also be transformed or neoplastic cells of different types (*e.g.*, carcinomas of different cell origins, lymphomas of different cell types, *etc.)* or cancerous cells of any kind (*e.g.,* from any of the cancers disclosed herein). Cells of different origins (*e.g.*, ectodermal, mesodermal, and endodermal) are also contemplated for use in the methods of the present disclosure. In some embodiments, the cells are microglia, astrocytes, oligodendrocytes, excitatory neurons, or inhibitory neurons. In some embodiments, cells of multiple cell types are present within the same sample.

The term "complementary" is used herein to refer to two oligonucleotide sequences (*e.g.,* DNA or RNA) comprising bases that hydrogen bond to one another. The degree of complementarity between two oligonucleotide sequences can vary, from complete complementarity to no complementarity. For example, two oligonucleotide sequences may be only partially complementary to one another (*e.g.*, in the probes described herein, wherein only a portion of the probe is complementary to another probe, or to an RNA of interest). Two oligonucleotide sequences may be, *e.g.,* 70% or more complementary to one another, 75% or more complementary to one another, 80% or more complementary to one another, 85% or more complementary to one another, 90% or more complementary to one another, 95% or more complementary to one another, 96% or more complementary to one another, 97% or more complementary to one another, 98% or more complementary to one another, 99% or more complementary to one another, or 100% complementary to one another.

The terms "polynucleotide", "nucleotide sequence", "nucleic acid", "nucleic acid molecule", "nucleic acid sequence", and "oligonucleotide" refer to a series of nucleotide bases (also called "nucleotides") in DNA and RNA and mean any chain of two or more nucleotides. The polynucleotides can be chimeric mixtures or derivatives or modified versions thereof, and single-stranded or double-stranded. The oligonucleotide can be modified at the base moiety, sugar moiety, or phosphate backbone, for example, to improve stability of the molecule, its hybridization parameters, *etc.*

A "protein," "peptide," or "polypeptide" comprises a polymer of amino acid residues linked together by peptide bonds. The term refers to proteins, polypeptides, and peptides of any size, structure, or function. Typically, a protein will be at least three amino acids long. A protein may refer to an individual protein or a collection of proteins. Inventive proteins preferably contain only natural amino acids, although non-natural amino acids (*i.e.,* compounds that do not occur in nature but that can be incorporated into a polypeptide chain) and/or amino acid analogs as are known in the art may alternatively be employed. Also, one or more of the amino acids in a protein may be modified, for example, by the addition of a chemical entity such as a carbohydrate group, a hydroxyl group, a phosphate group, a farnesyl group, an isofarnesyl group, a fatty acid group, a linker for conjugation or functionalization, or other modification. A protein may also be a single molecule or may be a multi-molecular complex. A protein may be a fragment of a naturally occurring protein or peptide. A protein may be naturally occurring, recombinant, synthetic, or any combination of these. A protein may also be a therapeutic protein administered as a treatment for a disease or disorder (*e.g.*, one that is associated with a change in the profile of RNAs being translated in a cell taken from a subject). In certain embodiments, the protein is an antibody.

A "transcript" or "RNA transcript" is the product resulting from RNA polymerase-catalyzed transcription of a DNA sequence. When the RNA transcript is a complimentary copy of the DNA sequence, it is referred to as the primary transcript, or it may be an RNA sequence derived from post-transcriptional processing of the primary transcript and is referred to as the mature RNA. "Messenger RNA (mRNA)" refers to the RNA that is without introns and can be translated into polypeptides by the cell. "cRNA" refers to complementary RNA, transcribed from a recombinant cDNA template. "cDNA" refers to DNA that is complementary to and derived from an mRNA template.

The term "sample" or "biological sample" refers to any sample including tissue samples (such as tissue sections, surgical biopsies, and needle biopsies of a tissue); cell samples (*e.g.*, cytological smears (such as Pap or blood smears) or samples of cells obtained by microdissection); or cell fractions, fragments, or organelles (such as obtained by lysing cells and separating the components thereof by centrifugation or otherwise). Other examples of biological samples include, but are not limited to, blood, serum, urine, semen, fecal matter, cerebrospinal fluid, interstitial fluid, mucous, tears, sweat, pus, biopsied tissue (*e.g.*, obtained by a surgical biopsy or needle biopsy), nipple aspirates, milk, vaginal fluid, saliva, swabs (such as buccal swabs), or any material containing biomolecules that is derived from a first biological sample. In some embodiments, a biological sample is a surgical biopsy taken from a subject, for example, a biopsy of any of the tissues described herein. In certain embodiments, a biological sample is a tumor biopsy (*e.g.*, from a subject diagnosed with, suspected of having, or thought to have cancer). In some embodiments, the tumor biopsy captures primary tumor cells and tissue for direct study. In some embodiments, liquid biopsy can be used on tumor cells that have been captured and/or sorted via traditional methods (*e.g.*, FACS, affinity capture, *etc.*) from blood or other bodily fluids (CNS fluid, lymph, *etc.*). In some embodiments, the sample is brain tissue. In some embodiments, the tissue is cardiac tissue. In some embodiments, the tissue is muscle tissue.

The term "small molecule" refers to molecules, whether naturally-occurring or artificially created (*e.g.*, via chemical synthesis) that have a relatively low molecular weight. Typically, a small molecule is an organic compound (*e.g.*, it contains carbon). The small molecule may contain multiple carbon-carbon bonds, stereocenters, and other functional groups (*e.g.*, amines, hydroxyl, carbonyls, and heterocyclic rings, *etc.*). In certain embodiments, the molecular weight of a small molecule is not more than about 1,000 g/mol, not more than about 900 g/mol, not more than about 800 g/mol, not more than about 700 g/mol, not more than about 600 g/mol, not more than about 500 g/mol, not more than about 400 g/mol, not more than about 300 g/mol, not more than about 200 g/mol, or not more than about 100 g/mol. In certain embodiments, the molecular weight of a small molecule is at least about 100 g/mol, at least about 200 g/mol, at least about 300 g/mol, at least about 400 g/mol, at least about 500 g/mol, at least about 600 g/mol, at least about 700 g/mol, at least about 800 g/mol, at least about 900 g/mol, or at least about 1,000 g/mol. Combinations of the above ranges (*e.g.*, at least about 200 g/mol and not more than about 500 g/mol) are also possible. In certain embodiments, the small molecule is a therapeutically active agent such as a drug (*e.g.*, a molecule approved by the U.S. Food and Drug Administration. The small molecule may also be complexed with one or more metal atoms and/or metal ions. Preferred small molecules are biologically active in that they produce a biological effect in animals, preferably mammals, and more preferably humans. In certain embodiments, the small molecule is a drug. Preferably, though not necessarily, the drug is one that has already been deemed safe and effective for use in humans or animals by the appropriate governmental agency or regulatory body.

A "subject" to which administration is contemplated refers to a human (*i.e.*, male or female of any age group, *e.g.*, pediatric subject (*e.g.*, infant, child, or adolescent) or adult subject (*e.g.*, young adult, middle-aged adult, or senior adult)) or non-human animal. In some embodiments, the non-human animal is a mammal (*e.g.*, primate (*e.g.*, cynomolgus monkey or rhesus monkey) or mouse). The term "patient" refers to a subject in need of treatment of a disease. In some embodiments, the subject is human. In some embodiments, the patient is human. The human may be a male or female at any stage of development. A subject or patient "in need" of treatment of a disease or disorder includes, without limitation, those who exhibit any risk factors or symptoms of a disease or disorder. In some embodiments, a subject is a non-human experimental animal (*e.g.*, a mouse, rat, dog, or non-human primate).

A "therapeutically effective amount" of a treatment or therapeutic agent is an amount sufficient to provide a therapeutic benefit in the treatment of a condition or to delay or minimize one or more symptoms associated with the condition. A therapeutically effective amount of a treatment or therapeutic agent means an amount of the therapy, alone or in combination with other therapies, that provides a therapeutic benefit in the treatment of the condition. The term "therapeutically effective amount" can encompass an amount that improves overall therapy, reduces or avoids symptoms, signs, or causes of the condition, and/or enhances the therapeutic efficacy of another therapeutic agent.

As used herein, a "tissue" is a group of cells and their extracellular matrix from the same origin. Together, the cells carry out a specific function. The association of multiple tissue types together forms an organ. The cells may be of different cell types. In some embodiments, a tissue is an epithelial tissue. Epithelial tissues are formed by cells that cover an organ surface (e.g., the surface of the skin, airways, soft organs, reproductive tract, and inner lining of the digestive tract). Epithelial tissues perform protective functions and are also involved in secretion, excretion, and absorption. Examples of epithelial tissues include, but are not limited to, simple squamous epithelium, stratified squamous epithelium, simple cuboidal epithelium, transitional epithelium, pseudo stratified epithelium, columnar epithelium, and glandular epithelium. In some embodiments, a tissue is a connective tissue. Connective tissues are fibrous tissues made up of cells separated by non-living material (e.g., an extracellular matrix). Connective tissues provide shape to organs and hold organs in place. Connective tissues include fibrous connective tissue, skeletal connective tissue, and fluid connective tissue. Examples of connective tissues include, but are not limited to, blood, bone, tendon, ligament, adipose, and areolar tissues. In some embodiments, a tissue is a muscular tissue. Muscular tissue is an active contractile tissue formed from muscle cells. Muscle tissue functions to produce force and cause motion. Muscle tissue includes smooth muscle (*e.g.*, as found in the inner linings of organs), skeletal muscle (*e.g.*, as typically attached to bones), and cardiac muscle (*e.g.*, as found in the heart, where it contracts to pump blood throughout an organism). In some embodiments, a tissue is a nervous tissue. Nervous tissue includes cells comprising the central nervous system and peripheral nervous system. Nervous tissue forms the brain, spinal cord, cranial nerves, and spinal nerves (e.g., motor neurons). In certain embodiments, a tissue is brain tissue. In certain embodiments, a tissue is placental tissue. In some embodiments, a tissue is heart tissue.

The term "translatome" refers to all of the open reading frames (*i.e.,* DNA sequences that fall between start and stop codons) in a particular cell or organism. In some embodiments, the methods, probes, and kits provided herein are useful for studying and/or profiling the translatome.

The terms "treatment," "treat," and "treating" refer to reversing, alleviating, delaying the onset of, or inhibiting the progress of a disease described herein (*e.g.*, cancer). In some embodiments, treatment may be administered after one or more signs or symptoms of the disease have developed or have been observed (*e.g.*, prophylactically (as may be further described herein) or upon suspicion or risk of disease). In other embodiments, treatment may be administered in the absence of signs or symptoms of the disease. For example, treatment may be administered to a susceptible subject prior to the onset of symptoms (*e.g.*, in light of a history of symptoms in the subject, or family members of the subject). Treatment may also be continued after symptoms have resolved, for example, to delay or prevent recurrence. In some embodiments, treatment may be administered after using the methods disclosed herein and observing a change in the profile of RNAs being expressed in a cell or tissue in comparison to a healthy cell or tissue.

## DETAILED DESCRIPTION OF CERTAIN EMBODIMENTS

The aspects described herein are not limited to specific embodiments, systems, compositions, methods, or configurations, and as such can, of course, vary. The terminology used herein is for the purpose of describing particular aspects only and, unless specifically defined herein, is not intended to be limiting.

The present disclosure provides methods, compositions, and systems for profiling RNAs being translated in a cell. The present disclosure also provides methods for diagnosing a disease or disorder in a subject based on a profile of the RNAs being translated in a cell, including cells within an intact tissue. Methods of screening for or testing a candidate agent capable of modulating translation of one or more RNAs are also provided by the present disclosure. The present disclosure also provides methods for treating a disease or disorder in a subject in need thereof. Pairs and sets of oligonucleotide probes, which may be useful for performing the methods described herein, are also described by the present disclosure, as well as kits comprising any of the oligonucleotide probes described herein.

*Methods for Profiling RNAs being Translated in a Cell*

In one aspect, the present disclosure provides methods for profiling RNAs being translated in a cell. In the methods disclosed herein, a cell may be contacted with one or more pairs of probes or sets of probes, which are described further herein and may be used to amplify RNAs that are actively being translated by a ribosome to produce one or more concatenated amplicons. The one or more concatenated amplicons may then be embedded in a polymeric matrix and sequenced to determine the identity of the transcripts and their location within the polymeric matrix (*e.g.*, through SEDAL sequencing as described further herein). Using the locations of the transcripts of interest, RNAs being translated in a cell may be profiled. The methods provided herein have several advantages over previously disclosed methods and systems, including, but not limited to, the ability to profile RNA translation in a sample without disrupting the spatial information of subcellular structures, cell morphology, and/or tissue organization in the sample.

In some embodiments, the present disclosure provides a method for profiling RNAs being translated in a cell comprising the steps of:

a) contacting the cell with one or more pairs of probes, wherein each pair of probes comprises a first probe and a second probe, wherein

i) the first probe comprises an oligonucleotide portion that is complementary to the second probe, an oligonucleotide portion that is complementary to an RNA of interest, and an oligonucleotide barcode sequence; and
ii) the second probe comprises a portion that recognizes the ribosome and an oligonucleotide portion that is complementary to a portion of the first probe;

b) ligating the 5' end and the 3' end of the first probe together to produce a circular oligonucleotide;
c) performing rolling circle amplification to amplify the circular oligonucleotide using the second probe as a primer to produce one or more concatenated amplicons;
d) embedding the one or more concatenated amplicons in a polymeric matrix; and
e) sequencing the concatenated amplicons embedded in the polymeric matrix to determine the identity and location of each RNA of interest in the cell.

The methods disclosed herein contemplate the use of a pair of probes comprising a first probe and a second probe. The second probe (also referred to herein as the "primer probe") comprises a portion that recognizes a ribosome (*i.e.,* a ribosome that is bound to and is actively translating the RNA of interest). The portion of the probe that recognizes a ribosome may be a protein, peptide, nucleic acid, or small molecule. In some embodiments, the portion of the second probe that recognizes a ribosome is an agent that binds an antibody, or an antibody variant or fragment. In certain embodiments, the portion of the second probe that recognizes the ribosome comprises an antibody (e.g., a secondary antibody), or an antibody variant or fragment. When the portion of the second probe that recognizes the ribosome is a secondary antibody, the method may optionally further comprise contacting the cell with a primary antibody that recognizes a ribosome and is recognized by the secondary antibody of the second probe. The primary antibody may recognize any portion of the ribosome, for example, any protein, nucleic acid (*e.g.,* rRNA), or combination thereof of the ribosome. In some embodiments, the primary antibody is an anti-40S ribosomal protein S3 (RPS3) antibody (*e.g.*, an anti-RPS3 monoclonal antibody). In some embodiments, the antibody is an anti-60S ribosomal protein L4 (RPL4) antibody (*e.g.*, an anti-RPL4 polyclonal antibody). In place of an antibody, the present disclosure also contemplates the use of any agent capable of recognizing the ribosome on the probes described herein. In some embodiments, the portion of the second probe that recognizes the ribosome comprises an oligonucleotide that is complementary to a portion of the ribosomal RNA (rRNA) within the ribosome. In some embodiments, the portion of the second probe that recognizes the ribosome comprises an oligonucleotide that is complementary to a portion of the 40S small ribosomal subunit (including, *e.g.*, the 18S rRNA) or to a portion of the 60S large ribosomal subunit (including, *e.g.*, the 5S rRNA, the 28S rRNA, and the 5.8S rRNA). In some embodiments, the portion of the second probe that recognizes the ribosome comprises an oligonucleotide that is complementary to a portion of the 18S rRNA. In certain embodiments, the oligonucleotide complementary to a portion of rRNA is 5, 6, 7, 8, 9, 10, 11, 12, 13, 14, 15, 16, 17, 18, 19, 20, 21, 22, 23, 24, 25, 26, 27, 28, 29, 30, or more than 30 nucleotides in length. In certain embodiments, the oligonucleotide complementary to a portion of rRNA is about 25 nucleotides in length.

In addition to the portion that recognizes the ribosome, the second probe also comprises a portion that is complementary to a portion of the first probe. In some embodiments, the portion of the second probe that is complementary to a portion of the first probe is 3, 4, 5, 6, 7, 8, 9, 10, 11, 12, 13, 14, 15, 16, 17, 18, 19, or 20 nucleotides in length. The present disclosure contemplates any arrangement of the portions of the second probe. In some embodiments, the second probe used in the methods described herein comprises the structure:

5'-[portion recognizing ribosome]-[portion complementary to first probe]-3' or
5'-[portion complementary to first probe]-[portion recognizing ribosome]-3';

wherein ]-[ comprises an optional linker (e.g., nucleotide linker). In some embodiments, ]-[ represents a direct linkage between two portions of the second oligonucleotide probe (*i.e.*, a phosphodiester bond).

The first probe used in the methods described herein (also referred to herein as the "padlock" probe) includes an oligonucleotide barcode sequence made up of a specific sequence of nucleotides. In some embodiments, the oligonucleotide barcode sequence of the first probe is about 3 to about 20, about 5 to about 15, about 6 to about 14, about 7 to about 13, about 8 to about 12, or about 9 to about 11 nucleotides in length. In some embodiments, the oligonucleotide barcode sequence of the first probe is 3, 4, 5, 6, 7, 8, 9, 10, 11, 12, 13, 14, or 15 nucleotides in length. The barcodes of the oligonucleotide probes described herein may comprise gene-specific sequences used to identify RNAs of interest that are being translated (*i.e.,* each barcode sequence is associated with a specific gene or transcript). The use of the barcodes on probes analogous to those described herein is further described in, for example, International Patent Application Publication No. WO 2019/199579, published October 17, 2019, and Wang et al., Science 2018, 361, 380, both of which are incorporated herein by reference in their entireties.

The first probe also comprises an oligonucleotide portion that is complementary to the second probe, and an oligonucleotide portion that is complementary to the RNA of interest. In some embodiments, the portion of the first probe that is complementary to an RNA of interest is about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, or about 30 nucleotides long. In some embodiments, the first probe is about 20, about 21, about 22, about 23, about 24, about 25, about 26, about 27, about 28, about 29, about 30, about 31, about 32, about 33, about 34, about 35, about 36, about 37, about 38, about 39, or about 40 nucleotides long or longer. The arrangement of the portions of the first oligonucleotide probe in any order is contemplated by the present disclosure. In some embodiments, the first probe comprises the structure:

5'-[portion complementary to second probe]-[portion complementary to RNA of interest]-[barcode sequence]-3' or
5'-[portion complementary to RNA of interest]-[portion complementary to second probe]-[barcode sequence]-3' or
5'-[barcode sequence]-[portion complementary to RNA of interest]-[portion complementary to second probe]-3' or
5'-[barcode sequence]-[portion complementary to second probe]-[portion complementary to RNA of interest]-3' or
5'-[portion complementary to RNA of interest]-[barcode sequence]-[portion complementary to second probe]-3' or

5'-[portion complementary to second probe]-[barcode sequence]-[portion complementary to RNA of interest]-3';

wherein ]-[ comprises an optional linker (*e.g.,* a nucleotide linker). In some embodiments, ]-[ represents a direct linkage between two portions of the first probe (*i.e.,* a phosphodiester bond).

In some embodiments, the present disclosure provides a method for profiling RNAs being translated in a cell comprising the steps of:

a) contacting the cell with one or more sets of probes, wherein each set of probes comprises a first probe, a second probe, and a third probe, wherein

i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;

ii) the second probe comprises a portion that recognizes the ribosome and an oligonucleotide portion that is complementary to a portion of the first probe; and

iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe;

b) ligating the 5' end and the 3' end of the first probe together to produce a circular oligonucleotide;

c) performing rolling circle amplification to amplify the circular oligonucleotide using the third probe as a primer to produce one or more concatenated amplicons;

d) embedding the one or more concatenated amplicons in a polymeric matrix; and

e) sequencing the concatenated amplicons embedded in the polymeric matrix to determine the identity and location of each RNA of interest in the cell.

The methods disclosed herein contemplate the use of a set of probes comprising a first probe, a second probe, and a third probe. The addition of the third probe (also referred to herein as a "primer probe") may result in increased specificity and/or reduced off target amplification compared to embodiments of the methods where a third probe is not used.

As described herein, the second probe (also referred to herein as a "splint probe" or as a "blocked probe") comprises a portion that recognizes a ribosome (*i.e.,* a ribosome that is bound to and is actively translating the RNA of interest). In some embodiments, the second probe further comprises a polymerization blocker. The addition of a polymerization blocker prevents the second probe from being used as a primer in the amplification of step (c), ensuring that the third probe will be used as the primer during amplification. The polymerization blocker on the second probe can be any moiety capable of preventing the use of the second probe as a primer in the amplification of step (c) of the methods described herein. In some embodiments, the polymerization blocker is at the 3' end of the second probe. The polymerization blocker can be, for example, any chemical moiety that prevents a polymerase from using the second probe as a primer for polymerization. In some embodiments, the polymerization blocker is a nucleic acid residue comprising a blocked 3' hydroxyl group (e.g., comprising an oxygen protecting group on the 3' hydroxyl group). In some embodiments, the polymerization blocker comprises a hydrogen in place of the 3' hydroxyl group. In some embodiments, the polymerization blocker comprises any chemical moiety in place of the 3' hydroxyl group that prevents an additional nucleotide from being added. In some embodiments, the polymerization blocker comprises an inverted nucleic acid residue. In some embodiments, the polymerization blocker is an inverted adenosine, thymine, cytosine, guanosine, or uridine residue. In certain embodiments, the polymerization blocker is an inverted thymine residue.

In addition to the portion that recognizes the ribosome, the second probe also comprises a portion that is complementary to a portion of the first probe. In some embodiments, the portion of the second probe that is complementary to a portion of the first probe is 4-20, 5-19, 6-18, 7-17, 8-16, 9-15, 10-14, or 11-13 nucleotides in length. In some embodiments, the portion of the second probe that is complementary to a portion of the first probe is 4, 5, 6, 7, 8, 9, 10, 11, 12, 13, 14, 15, 16, 17, 18, 19, or 20 nucleotides in length. In certain embodiments, the portion of the second probe that recognizes the ribosome and the portion of the second probe that is complementary to the first probe are joined by a poly-A nucleotide linker. In some embodiments, the poly-A nucleotide linker is 20-80, 30-70, or 40-60 nucleotides in length (e.g., about 50 nucleotides in length).

The present disclosure contemplates any arrangement of the portions of the second probe. In some embodiments, the second probe used in the methods described herein comprises the structure:

5'-[portion recognizing ribosome]-[portion complementary to first probe]-3' or
5'-[portion complementary to first probe]-[portion recognizing ribosome]-3';

wherein ]-[ comprises an optional linker (*e.g.*, nucleotide linker). In some embodiments, ]-[ represents a direct linkage between two portions of the second oligonucleotide probe.
In some embodiments, the second probe comprises the structure:
5'-[portion recognizing ribosome]-[portion complementary to first probe]-[polymerization blocker]-3';
wherein ]-[ comprises an optional nucleotide linker.

The first probe used in the methods described herein (also referred to herein as the "padlock" probe) includes a first oligonucleotide barcode sequence and a second oligonucleotide barcode sequence made up of a specific sequence of nucleotides. In some embodiments, the first and second oligonucleotide barcode sequences on the first probe comprise the same nucleotide sequence. The presence of the second barcode sequence of the first oligonucleotide probe may increase the specificity of the detection of the RNA of interest in the methods described herein (*i.e.,* as compared to if the method were performed using a first oligonucleotide probe that did not comprise a second barcode sequence). The use of an additional oligonucleotide barcode sequence of the first oligonucleotide probe may also play a role in reducing non-specific amplification of the RNA of interest in the methods described herein. This is accomplished because the oligonucleotide barcode sequence of the third probe used in the methods described herein is complementary to the first oligonucleotide barcode sequence on the first probe, adding an additional layer of specificity that is required before amplification can occur using the third probe as a primer. In some embodiments, the portion of the first probe that is complementary to the third probe is 5-15, 6-14, 7-13, 8-12, or 9-11 nucleotides in length. In some embodiments, the portion of the first probe that is complementary to the third probe is 5, 6, 7, 8, 9, 10, 11, 12, 13, 14, or 15 nucleotides in length.

The first probe also comprises an oligonucleotide portion that is complementary to the second probe and a portion that is complementary to an RNA of interest. In some embodiments, the portion of the first probe that is complementary to the second probe is 4-20, 5-19, 6-18, 7-17, 8-16, 9-15, 10-14, or 11-13 nucleotides in length. In some embodiments, the portion of the first probe that is complementary to the second probe is 4, 5, 6, 7, 8, 9, 10, 11, 12, 13, 14, 15, 16, 17, 18, 19, or 20 nucleotides in length. In certain embodiments, the oligonucleotide portion of the first probe that is complementary to the second probe is split between the 5' end and the 3' end of the first probe. In some embodiments, the portion of the first probe that is complementary to the RNA of interest is 10-30, 11-29, 12-28, 13-27, 14-26, 15-25, 16-24, 17-23, 18-22, or 19-21 nucleotides in length. In some embodiments, the portion of the first probe that is complementary to an RNA of interest is about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, or about 30 nucleotides long. In some embodiments, the first probe is about 20, about 21, about 22, about 23, about 24, about 25, about 26, about 27, about 28, about 29, about 30, about 31, about 32, about 33, about 34, about 35, about 36, about 37, about 38, about 39, or about 40 nucleotides long or longer.

In some embodiments, the first probe comprises the structure:
5'-[portion complementary to second probe]-[first barcode sequence]-[portion complementary to portion of third probe]-[portion complementary to RNA of interest]-[second barcode sequence]-3';
wherein ]-[ comprises an optional linker (*e.g.*, nucleotide linker). In some embodiments, ]-[ represents a direct linkage between two portions of the first probe (*i.e.*, a phosphodiester bond).

The third probe used in the methods disclosed herein (also referred to herein as the "primer" probe) includes a barcode sequence made up of a specific sequence of nucleotides. In some embodiments, the barcode sequence of the third probe is about 3 to about 20, about 5 to about 15, about 6 to about 14, about 7 to about 13, about 8 to about 12, or about 9 to about 11 nucleotides in length. In some embodiments, the barcode sequence of the third probe is 3, 4, 5, 6, 7, 8, 9, 10, 11, 12, 13, 14, or 15 nucleotides in length. In certain embodiments, the barcode sequence of the third probe is 10 nucleotides in length.

The third probe also comprises a portion that is complementary to an RNA of interest and a portion that is complementary to a portion of the first probe. In some embodiments, the first and the third probes are complementary to and bind different portions of the RNA of interest. In some embodiments, the portion of the third probe that is complementary to the RNA of interest is 10-30, 11-29, 12-28, 13-27, 14-26, 15-25, 16-24, 17-23, 18-22, or 19-21 nucleotides in length. In some embodiments, the portion of the third probe that is complementary to an RNA of interest is about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, or about 30 nucleotides long. In some embodiments, the third probe is about 20, about 21, about 22, about 23, about 24, about 25, about 26, about 27, about 28, about 29, about 30, about 31, about 32, about 33, about 34, about 35, about 36, about 37, about 38, about 39, or about 40 nucleotides long or longer. In some embodiments, the portion of the third probe that is complementary to the first probe is 5-15, 6-14, 7-13, 8-12, or 9-11 nucleotides in length. In some embodiments, the portion of the third probe that is complementary to the first probe is 5, 6, 7, 8, 9, 10, 11, 12, 13, 14, or 15 nucleotides in length.

In some embodiments, the third probe comprises the structure:

5'-[portion complementary to RNA of interest]-[portion complementary to first probe]-[barcode sequence]-3'
wherein ]-[ comprises an optional linker (*e.g.*, nucleotide linker). In some embodiments, ]-[ represents a direct linkage between two portions of the third probe. Any arrangement of the portions of the third probe is contemplated by the present disclosure.

The use of any type of cell in the methods disclosed herein is contemplated by the present disclosure (*e.g.*, any of the cell types described herein). In some embodiments, the cell is a mammalian cell. In certain embodiments, the cell is a human cell. The present disclosure also contemplates performing the methods described herein on multiple cells simultaneously (*e.g.*, more than 100 cells, more than 200 cells, more than 300 cells, more than 400 cells, more than 500 cells, more than 1000 cells, more than 10,000 cells, more than 20,000 cells, more than 30,000 cells, more than 40,000 cells, or more than 50,000 cells simultaneously). In some embodiments, the method is performed on multiple cells of the same cell type. In some embodiments, the method is performed on multiple cells comprising cells of different cell types. The cell types in which RNAs being translated may be profiled using the methods disclosed herein include, but are not limited to, stem cells, progenitor cells, neuronal cells, astrocytes, dendritic cells, endothelial cells, microglia, oligodendrocytes, muscle cells, myocardial cells, mesenchymal cells, epithelial cells, immune cells, hepatic cells, smooth and skeletal muscle cells, hematopoietic cells, lymphocytes, monocytes, neutrophils, macrophages, natural killer cells, mast cells, adipocytes, and neurons. In certain embodiments, the cell or cells are present within an intact tissue (*e.g.*, of any of the tissue types described herein). In certain embodiments, the intact tissue is a fixed tissue sample. In some embodiments, the intact tissue comprises multiple cell types. In certain embodiments, the tissue is cardiac tissue, lymph node tissue, liver tissue, muscle tissue, bone tissue, eye tissue, or ear tissue. In certain embodiments, the tissue is brain tissue.

The RNAs of interest for which gene expression is profiled in the methods described herein may be transcripts that have been expressed from the genomic DNA of the cell. In some embodiments, the RNAs of interest are mRNA. The methods described herein may be used to profile one RNA being translated in a cell at a time, or multiple RNAs of interest simultaneously. In some embodiments, RNA translation in a cell, or multiple cells, is profiled for more than 1, more than 2, more than 3, more than 4, more than 5, more than 10, more than 20, more than 30, more than 40, more than 50, more than 100, more than 200, more than 500, more than 1000, more than 2000, more than 3000 RNAs, more than 4000 RNAs, or more than 5000 RNAs simultaneously.

A polymeric matrix is used in the methods described herein following rolling circle amplification to facilitate sequencing and imaging of the RNAs of interest being translated in the cell. The use of various polymeric matrices is contemplated by the present disclosure, and any polymeric matrix in which the one or more concatenated amplicons can be embedded is suitable for use in the methods described herein. In some embodiments, the polymeric matrix is a hydrogel (*i.e.*, a network of crosslinked polymers that are hydrophilic). In some embodiments, the hydrogel is a polyvinyl alcohol hydrogel, a polyethylene glycol hydrogel, a sodium polyacrylate hydrogel, an acrylate polymer hydrogel, or a polyacrylamide hydrogel. In certain embodiments, the hydrogel is a polyacrylamide hydrogel. Such a hydrogel may be prepared, for example, by incubating the sample in a buffer comprising acrylamide and bisacrylamide, removing the buffer, and incubating the sample in a polymerization mixture (comprising, *e.g.*, ammonium persulfate and tetramethylethylenediamine).

In some embodiments, the step of performing rolling circle amplification to amplify the circular oligonucleotide to produce one or more concatenated amplicons further comprises providing nucleotides modified with reactive chemical groups (*e.g.*, amine modified nucleotides such as 5-(3-aminoallyl)-dUTP). In some embodiments, the nucleotides modified with reactive chemical groups make up about 5%, about 6%, about 7%, about 8%, about 9%, or about 10% of the nucleotides used in the amplification reaction. For example, the step of performing rolling circle amplification to amplify the circular oligonucleotide to produce one or more concatenated amplicons may further comprise providing amine-modified nucleotides such as 5-(3-aminoallyl)-dUTP. During the amplification process, the amine-modified nucleotides are incorporated into the one or more concatenated amplicons as they are produced. The resulting amplicons are functionalized with primary amines, which can be further reacted with another compatible chemical moiety (*e.g.,* N-hydroxysuccinimide) to facilitate the step of embedding the concatenated amplicons in the polymeric matrix. In some embodiments, the step of embedding the one or more concatenated amplicons in a polymeric matrix comprises reacting the amine-modified nucleotides of the one or more concatenated amplicons with acrylic acid *N*-hydroxysuccinimide ester and co-polymerizing the one or more concatenated amplicons and the polymer matrix.

The methods disclosed herein also include a step of sequencing the concatenated amplicons embedded in the polymeric matrix. In some embodiments, the step of sequencing comprises performing "sequencing with error-reduction by dynamic annealing and ligation" (SEDAL sequencing). In some embodiments, SEDAL is performed two, three, four, five, or more than five times on a particular sample. SEDAL sequencing is described further in Wang, X. et al., Three-dimensional intact-tissue sequencing of single-cell transcriptional states. Science 2018, 361, 380, and International Patent Application Publication No. WO 2019/199579, published October 17, 2019, each of which is incorporated herein by reference. In brief, an oligonucleotide probe comprising a detectable label (*i.e.,* any label that can be used to visualize the location of the additional oligonucleotide probe, for example, through imaging) is provided to the cell. In certain embodiments, the detectable label is fluorescent (*e.g.,* a fluorophore). The additional oligonucleotide probe is complementary to the oligonucleotide barcode sequence of the first probe and is thus linked to the identity of an RNA of interest and can be used to identify the location of an RNA of interest within the cell.

The additional oligonucleotide probe used in the methods described herein (*e.g.*, as used in SEDAL sequencing) may be read out using any suitable imaging technique known in the art. For example, in embodiments where the additional oligonucleotide probe comprises a fluorophore, the fluorophore may be read out using imaging to identify the RNA of interest. As discussed above, the additional oligonucleotide probe comprises a sequence complementary to a barcode sequence on the first oligonucleotide probe, which is used to detect a specific RNA of interest. By imaging the location of the additional oligonucleotide probe comprising a fluorophore, the location of that specific RNA of interest within the sample can be determined. In some embodiments, the step of imaging comprises fluorescent imaging. In certain embodiments, the step of imaging comprises confocal microscopy. In certain embodiments, the step of imaging comprises epifluorescence microscopy. In certain embodiments, two rounds of imaging are performed. In certain embodiments, three rounds of imaging are performed. In certain embodiments, four rounds of imaging are performed. In certain embodiments, five or more rounds of imaging are performed.

In some embodiments, the methods for profiling RNA translation described herein may be combined with methods for profiling additional molecules within the cell. For example, expression of non-translating RNAs may be profiled alongside RNA translation using probes that do not comprise a portion that recognizes a ribosome. RNAs in other subcellular locations that are not actively being translated may be profiled alongside RNA translation as well. Profiling of protein expression (*e.g.*, using traditional proteomics methods) may also be combined with the methods described herein, as well as profiling of the transcriptome, lipids, and/or small molecules. In some embodiments, any of the methods provided herein further comprise a step of overexpressing or knocking out one or more genes in the cell to determine whether the one or more genes are involved in regulating the translation of the RNA of interest.

Any of the methods described herein may also be used to determine the cell type and/or cell state of one or more cells. In some embodiments, any of the methods provided herein further comprise a step of determining the cell type of the profiled cell, or the cell types of multiple profiled cells, by comparing the RNA translation profile of the cell or cells to reference data comprising RNA translation profiles of cells of various cell types. In some embodiments, any of the methods provided herein further comprise a step of determining the cell state of the profiled cell, or the cell states of multiple profiled cells, by comparing the RNA translation profile of the cell or cells to reference data comprising RNA translation profiles of cells of various cell states.

*Methods for Diagnosing a Disease or Disorder in a Subject*

In another aspect, the present disclosure provides methods for diagnosing a disease or disorder in a subject. For example, the methods for profiling RNAs being translated described herein may be performed on a cell or multiple cells taken from a subject (*e.g.*, a subject who is thought to have or is at risk of having a disease or disorder, or a subject who is healthy or thought to be healthy). The expression of various RNAs of interest in the cell can then be compared to the expression of the same RNAs of interest in a non-diseased cell or a cell from a non-diseased tissue sample (*e.g.*, a cell from a healthy individual, or multiple cells from a population of healthy individuals). Any difference in the RNA translation profile of the cell (including of a single RNA or of multiple RNAs of interest, *e.g.*, a specific disease signature) relative to one or more non-diseased cells may indicate that the subject has the disease or disorder. RNA translation in one or more non-diseased cells may be profiled alongside expression in a diseased cell as a control experiment. RNA translation in one or more non-diseased cells may have also been profiled previously, and expression in a diseased cell may be compared to this reference data for a non-diseased cell.

In some embodiments, a method for diagnosing a disease or disorder in a subject comprises the steps of:

a) contacting a cell taken from the subject with one or more pairs of probes, wherein each pair of probes comprises a first probe and a second probe, wherein

i) the first probe comprises an oligonucleotide portion that is complementary to the second probe, an oligonucleotide portion that is complementary to an RNA of interest, and an oligonucleotide barcode sequence; and
ii) the second probe comprises a portion that recognizes the ribosome and an oligonucleotide portion that is complementary to a portion of the first probe;

b) ligating the 5' end and the 3' end of the first probe together to produce a circular oligonucleotide;
c) performing rolling circle amplification to amplify the circular oligonucleotide using the second probe as a primer to produce one or more concatenated amplicons;
d) embedding the one or more concatenated amplicons in a polymeric matrix; and
e) sequencing the concatenated amplicons embedded in the polymeric matrix to determine the identity and location of each RNA of interest to profile RNAs being translated in the cell,

wherein a difference in the RNA translation profile of the cell relative to one or more non-diseased cells indicates that the subject has the disease or disorder.

In some embodiments, a method for diagnosing a disease or disorder in a subject comprises the steps of:

a) contacting a cell taken from the subject with one or more sets of probes, wherein each set of probes comprises a first probe, a second probe, and a third probe, wherein

i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;
ii) the second probe comprises a portion that recognizes the ribosome and an oligonucleotide portion that is complementary to a portion of the first probe; and
iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe;

b) ligating the 5' end and the 3' end of the first probe together to produce a circular oligonucleotide;
c) performing rolling circle amplification to amplify the circular oligonucleotide using the third probe as a primer to produce one or more concatenated amplicons;
d) embedding the one or more concatenated amplicons in a polymeric matrix; and
e) sequencing the concatenated amplicons embedded in the polymeric matrix to determine the identity and location of each RNA of interest in the cell;

wherein a difference in the RNA translation profile of the cell relative to one or more non-diseased cells indicates that the subject has the disease or disorder.

In some embodiments, RNAs being translated in one or more non-diseased cells are profiled simultaneously alongside the cell taken from a subject using the methods disclosed herein as a control experiment. In some embodiments, the profile of RNAs being translated in one or more non-diseased cells that is compared to expression in a diseased cell comprises reference data from when the method was performed on one or more non-diseased cells previously.

Diagnosis of any disease or disorder is contemplated by the methods described herein. In some embodiments, the disease or disorder is a genetic disease, a proliferative disease, an inflammatory disease, an autoimmune disease, a liver disease, a spleen disease, a lung disease, a hematological disease, a neurological disease, a psychiatric disease, a gastrointestinal (GI) tract disease, a genitourinary disease, an infectious disease, a musculoskeletal disease, an endocrine disease, a metabolic disorder, an immune disorder, a central nervous system (CNS) disorder, or a cardiovascular disease. In certain embodiments, the disease is cancer.

In some embodiments, the cell is present in a tissue (*e.g.*, epithelial tissue, connective tissue, muscular tissue, or nervous tissue). In some embodiments, the tissue is a tissue sample from a subject. In some embodiments, the subject is a non-human experimental animal (*e.g.,* a mouse, a rat, a dog, a pig, or a non-human primate). In some embodiments, the subject is a domesticated animal. In some embodiments, the subject is a human. In some embodiments, the tissue sample comprises a fixed tissue sample. In certain embodiments, the tissue sample is a biopsy (*e.g.,* bone, bone marrow, breast, gastrointestinal tract, lung, liver, pancreas, prostate, brain, nerve, renal, endometrial, cervical, lymph node, muscle, or skin biopsy). In certain embodiments, the biopsy is a tumor biopsy. In certain embodiments, the tissue is brain tissue. In certain embodiments, the tissue is from the central nervous system.

*Methods of Screening for an Agent Capable of Modulating Translation of One or More RNAs*

In another aspect, the present disclosure provides methods for screening for an agent capable of modulating translation of one or more RNAs of interest. For example, the methods for profiling RNAs being translated described herein may be performed in a cell in the presence of one or more candidate agents. The expression of various RNAs of interest being translated in the cell (*e.g.,* a normal cell, or a diseased cell) can then be compared to the expression of the same RNAs of interest in a cell that was not exposed to the one or more candidate agents. Any difference in the RNA translation profile relative to translation in the cell that was not exposed to the candidate agent(s) may indicate that translation of the one or more RNAs of interest is modulated by the candidate agent(s). In some embodiments, a particular signature (*e.g.*, of multiple RNAs of interest being translated) that is known to be associated with treatment of the disease may be used to identify a candidate agent capable of modulating translation in a desired manner. The methods described herein may also be used to identify drugs that have certain side effects, for example, by looking for specific RNA translation signatures when one or more cells is treated with a candidate agent or known drug.

In some embodiments, the present disclosure provides a method for screening for an agent capable of modulating translation of one or more RNAs comprises the steps of:

a) contacting a cell that is being treated with or has been treated with a candidate agent with one or more pairs of probes, wherein each pair of probes comprises a first probe and a second probe, wherein

i) the first probe comprises an oligonucleotide portion that is complementary to the second probe, an oligonucleotide portion that is complementary to an RNA of interest, and an oligonucleotide barcode sequence; and
ii) the second probe comprises a portion that recognizes the ribosome and an oligonucleotide portion that is complementary to a portion of the first probe;

b) ligating the 5' end and the 3' end of the first probe together to produce a circular oligonucleotide;
c) performing rolling circle amplification to amplify the circular oligonucleotide using the second probe as a primer to produce one or more concatenated amplicons;
d) embedding the one or more concatenated amplicons in a polymeric matrix; and
e) sequencing the concatenated amplicons embedded in the polymeric matrix to determine the identity and location of each RNA of interest to profile RNAs being translated in the cell;

wherein a difference in the profile of RNAs being translated in the presence of the candidate agent relative to in the absence of the candidate agent indicates that the candidate agent modulates translation of one or more RNAs.

In some embodiments, the present disclosure provides a method for screening for an agent capable of modulating translation of one or more RNAs comprises the steps of:

a) contacting a cell that is being treated with or has been treated with a candidate agent with one or more sets of probes, wherein each set of probes comprises a first probe, a second probe, and a third probe, wherein

i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;
ii) the second probe comprises a portion that recognizes the ribosome and an oligonucleotide portion that is complementary to a portion of the first probe; and
iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe;

b) ligating the 5' end and the 3' end of the first probe together to produce a circular oligonucleotide;
c) performing rolling circle amplification to amplify the circular oligonucleotide using the third probe as a primer to produce one or more concatenated amplicons;
d) embedding the one or more concatenated amplicons in a polymeric matrix; and
e) sequencing the concatenated amplicons embedded in the polymeric matrix to determine the identity and location of each RNA of interest bound to a ribosome in the cell,

wherein a difference in the profile of RNAs being translated in the presence of the candidate agent relative to in the absence of the candidate agent indicates that the candidate agent modulates translation of one or more RNAs.

In some embodiments, the candidate agent is a small molecule, a protein, a peptide, a nucleic acid, a lipid, or a carbohydrate. In some embodiments, the candidate agent comprises a known drug or an FDA-approved drug. In certain embodiments, the protein is an antibody. In certain embodiments, the protein is an antibody fragment or an antibody variant. In certain embodiments, the protein is a receptor. In certain embodiments, the protein is a cytokine. In certain embodiments, the nucleic acid is an mRNA, an antisense RNA, an miRNA, an siRNA, an RNA aptamer, a double stranded RNA (dsRNA), a short hairpin RNA (shRNA), or an antisense oligonucleotide (ASO). Any candidate agent may be screened using the methods described herein. In particular, any candidate agents thought to be capable of modulating translation of one or more RNAs may be screened using the methods described herein. In some embodiments, modulation of translation of one or more RNAs of interest by the candidate agent is associated with reducing, relieving, or eliminating the symptoms of a disease or disorder, or preventing the development or progression of the disease or disorder. In some embodiments, the disease or disorder modulated by the candidate agent is a genetic disease, a proliferative disease, an inflammatory disease, an autoimmune disease, a liver disease, a spleen disease, a lung disease, a hematological disease, a neurological disease, a psychiatric disease, a gastrointestinal (GI) tract disease, a genitourinary disease, an infectious disease, a musculoskeletal disease, an endocrine disease, a metabolic disorder, an immune disorder, a central nervous system (CNS) disorder, or a cardiovascular disease. In certain embodiments, the disease or disorder modulated by the candidate agent is cancer.

*Methods for Treating a Disease or Disorder in a Subject*

In another aspect, the present disclosure provides methods for treating a disease or disorder in a subject. For example, the methods for profiling RNAs being translated described herein may be performed in a cell from a sample taken from a subject (e.g., a subject who is thought to have or is at risk of having a disease or disorder). The profile of one or more RNAs being translated in the cell can then be compared to the translation status of the same RNAs of interest in a cell from a non-diseased tissue sample. A treatment for the disease or disorder may then be administered to the subject if any difference in the RNA translation profile relative to a non-diseased cell is observed. RNA translation in one or more non-diseased cells may be profiled alongside RNA translation in a diseased cell as a control experiment. RNA translation in one or more non-diseased cells may have also been profiled previously, and translation in a diseased cell may be compared to this reference data for a non-diseased cell.

In some embodiments, the present disclosure provides a method for treating a disease or disorder in a subject comprising the steps of:

a) contacting a cell that is being treated with or has been treated with a candidate agent with one or more pairs of probes, wherein each pair of probes comprises a first probe and a second probe, wherein

i) the first probe comprises an oligonucleotide portion that is complementary to the second probe, an oligonucleotide portion that is complementary to an RNA of interest, and an oligonucleotide barcode sequence; and
ii) the second probe comprises a portion that recognizes the ribosome and an oligonucleotide portion that is complementary to a portion of the first probe;

b) ligating the 5' end and the 3' end of the first probe together to produce a circular oligonucleotide;
c) performing rolling circle amplification to amplify the circular oligonucleotide using the second probe as a primer to produce one or more concatenated amplicons;
d) embedding the one or more concatenated amplicons in a polymeric matrix; and
e) sequencing the concatenated amplicons embedded in the polymeric matrix to determine the identity and location of each RNA of interest to profile RNAs being translated in the cell;

wherein a difference in the profile of RNAs being translated in the presence of the candidate agent relative to in the absence of the candidate agent indicates that the candidate agent modulates translation of one or more RNAs.

In some embodiments, the present disclosure provides a method for treating a disease or disorder in a subject comprising the steps of:

a) contacting a cell that is being treated with or has been treated with a candidate agent with one or more sets of probes, wherein each set of probes comprises a first probe, a second probe, and a third probe, wherein

i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;
ii) the second probe comprises a portion that recognizes the ribosome and an oligonucleotide portion that is complementary to a portion of the first probe; and
iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe;

b) ligating the 5' end and the 3' end of the first probe together to produce a circular oligonucleotide;
c) performing rolling circle amplification to amplify the circular oligonucleotide using the third probe as a primer to produce one or more concatenated amplicons;
d) embedding the one or more concatenated amplicons in a polymeric matrix; and

e) sequencing the concatenated amplicons embedded in the polymeric matrix to determine the identity and location of each RNA of interest bound to a ribosome in the cell;

wherein a difference in the profile of RNAs being translated in the presence of the candidate agent relative to in the absence of the candidate agent indicates that the candidate agent modulates translation of one or more RNAs.

In some embodiments, RNA translation in one or more non-diseased cells is profiled simultaneously using the methods disclosed herein as a control experiment. In some embodiments, the RNA translation data in one or more non-diseased cells that is compared to translation in a diseased cell comprises reference data from a time the method was performed on a non-diseased cell previously.

Any suitable treatment for a disease or disorder may be administered to the subject. In some embodiments, the treatment comprises administering a therapeutic agent. In some embodiments, the treatment comprises surgery. In some embodiments, the treatment comprises imaging. In some embodiments, the treatment comprises performing further diagnostic methods. In some embodiments, the treatment comprises radiation therapy. In some embodiments, the therapeutic agent is a small molecule, a protein, a peptide, a nucleic acid, a lipid, or a carbohydrate. In some embodiments, the therapeutic agent is a known drug and/or an FDA-approved drug. In certain embodiments, the protein is an antibody. In certain embodiments, the protein is an antibody fragment or antibody variant. In certain embodiments, the protein is a receptor, or a fragment or variant thereof. In certain embodiments, the protein is a cytokine. In certain embodiments, the nucleic acid is an mRNA, an antisense RNA, an miRNA, an siRNA, an RNA aptamer, a double stranded RNA (dsRNA), a short hairpin RNA (shRNA), or an antisense oligonucleotide (ASO).

Treatment of any disease or disorder is contemplated by the methods described herein. In some embodiments, the disease or disorder is a genetic disease, a proliferative disease, an inflammatory disease, an autoimmune disease, a liver disease, a spleen disease, a lung disease, a hematological disease, a neurological disease, a gastrointestinal (GI) tract disease, a genitourinary disease, an infectious disease, a musculoskeletal disease, an endocrine disease, a metabolic disorder, an immune disorder, a central nervous system (CNS) disorder, a neurological disorder, an ophthalmic disease, or a cardiovascular disease. In certain embodiments, the disease is cancer.

In some embodiments, the subject is a human. In some embodiments, the sample comprises a biological sample. In some embodiments, the sample comprises a tissue sample. In certain embodiments, the tissue sample is a biopsy (e.g., bone, bone marrow, breast, gastrointestinal tract, lung, liver, pancreas, prostate, brain, nerve, renal, endometrial, cervical, lymph node, muscle, or skin biopsy). In certain embodiments, the biopsy is a tumor biopsy. In certain embodiments, the biopsy is a solid tumor biopsy. In some embodiments, the tissue sample is a brain tissue sample. In certain embodiments, the tissue sample is a central nervous system tissue sample.

*Probes*

The present disclosure also provides pairs and sets of probes for use in the methods and systems described herein. In one aspect, the present disclosure provides pairs of probes comprising a first probe (also referred to herein as the "padlock" probe) and a second probe (also referred to herein as the "primer" probe), wherein:

i) the first probe comprises an oligonucleotide portion that is complementary to the second probe, an oligonucleotide portion that is complementary to an RNA of interest, and an oligonucleotide barcode sequence; and
ii) the second probe comprises a portion that recognizes the ribosome and an oligonucleotide portion that is complementary to a portion of the first probe.

All of the probes described herein may optionally have spacers or linkers of various nucleotide lengths in between each of the recited components, or the components of the oligonucleotide probes may be joined directly to one another (*i.e.*, by a phosphodiester bond). All of the probes described herein may comprise standard nucleotides, or some of the standard nucleotides may be substituted for any modified nucleotides known in the art.

The pair of probes described herein comprises a first probe and a second probe. The second probe comprises a portion that recognizes a ribosome (*i.e.,* a ribosome that is bound to and is actively translating the RNA of interest). In some embodiments, the portion of the second probe that recognizes a ribosome is an agent that binds an antibody, or an antibody variant or fragment. In certain embodiments, the portion of the second probe that recognizes the ribosome comprises an antibody (*e.g.*, a secondary antibody), or an antibody variant or fragment. When the portion of the second probe that recognizes the ribosome is a secondary antibody, the secondary antibody may bind to a primary antibody that recognizes the ribosome. In some embodiments, the primary antibody is an anti-40S ribosomal protein S3 (RPS3) antibody. In some embodiments, the primary antibody is an anti-60S ribosomal protein L4 (RPL4) antibody. In place of an antibody, the present disclosure also contemplates the use of any agent capable of recognizing the ribosome on the probes described herein. In some embodiments, the portion of the second probe that recognizes the ribosome comprises an oligonucleotide that is complementary to a portion of the ribosomal RNA (rRNA) within the ribosome. In some embodiments, the portion of the second probe that recognizes the ribosome comprises an oligonucleotide that is complementary to a portion of the 40S small ribosomal subunit (including, e.g., the 18S rRNA) or to a portion of the 60S large ribosomal subunit (including, e.g., the 5S rRNA, the 28S rRNA, and the 5.8S rRNA). In some embodiments, the portion of the second probe that recognizes the ribosome comprises an oligonucleotide that is complementary to a portion of the 18S rRNA. In certain embodiments, the oligonucleotide complementary to a portion of rRNA is 5, 6, 7, 8, 9, 10, 11, 12, 13, 14, 15, 16, 17, 18, 19, 20, 21, 22, 23, 24, 25, 26, 27, 28, 29, 30, or more than 30 nucleotides in length. In certain embodiments, the oligonucleotide complementary to a portion of rRNA is about 25 nucleotide in length.

The first probe of the pair of probes described herein (also referred to herein as the "padlock" probe) includes an oligonucleotide barcode sequence made up of a specific sequence of nucleotides. In some embodiments, the oligonucleotide barcode sequence of the first probe is about 3 to about 20, about 5 to about 15, about 6 to about 14, about 7 to about 13, about 8 to about 12, or about 9 to about 11 nucleotides in length. In some embodiments, the oligonucleotide barcode sequence of the first probe is 3, 4, 5, 6, 7, 8, 9, 10, 11, 12, 13, 14, or 15 nucleotides in length. The barcodes of the oligonucleotide probes described herein may comprise gene-specific sequences used to identify RNAs of interest (i.e., RNAs that are being translated by a ribosome).

The first probe also comprises a portion that is complementary to the second probe, and a portion that is complementary to the RNA of interest. The arrangement of the portions of the first oligonucleotide probe in any order is contemplated by the present disclosure. In some embodiments, the first probe comprises the structure:
5'-[portion complementary to second probe]-[portion complementary to RNA of interest]-[barcode sequence]-3'; wherein ]-[ comprises an optional nucleotide linker. In some embodiments, ]-[ represents a direct linkage between two portions of the first probe (i.e., a phosphodiester bond).

In addition to the portion that recognizes the ribosome, the second probe also comprises a portion that is complementary to a portion of the first probe. The present disclosure contemplates any arrangement of the portions of the second probe. In some embodiments, the second probe of the pair of probes described herein comprises the structure:
5'-[portion recognizing ribosome]-[portion complementary to first probe]-3'; wherein ]-[ comprises an optional linker (e.g., nucleotide linker). In some embodiments, ]-[ represents a direct linkage between two portions of the second oligonucleotide probe.

In another aspect, the present disclosure provides sets of oligonucleotide probes comprising a first probe (also referred to herein as a "padlock probe"), a second probe (also referred to herein as a "splint probe" or as a "blocked probe"), and a third probe (also referred to herein as a "primer probe"), wherein:

i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;
ii) the second probe comprises a portion that recognizes the ribosome and an oligonucleotide portion that is complementary to a portion of the first probe; and
iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe.

In comparison to the pairs of probes described herein, the sets of probes contemplated by the present disclosure comprise a third probe. The addition of the third probe may result in increased specificity and/or reduced off target amplification compared to embodiments where a third probe is not used. As described herein, the second probe comprises a portion that recognizes a ribosome (i.e., a ribosome that is bound to and is actively translating the RNA of interest). In some embodiments, the second probe further comprises a polymerization blocker. The addition of a polymerization blocker prevents the second probe from being used as a primer in the amplification of step (c), ensuring that the third probe will be used as the primer during amplification. The polymerization blocker on the second probe can be any moiety capable of preventing the use of the second probe as a primer in the amplification of step (c) of the methods described herein. In some embodiments, the polymerization blocker is at the 3' end of the second probe. The polymerization blocker can be, for example, any chemical moiety that prevents a polymerase from using the second probe as a primer for polymerization. In some embodiments, the polymerization blocker is a nucleic acid residue comprising a blocked 3' hydroxyl group (e.g., comprising an oxygen protecting group on the 3' hydroxyl group). In some embodiments, the polymerization blocker comprises a hydrogen in place of the 3' hydroxyl group. In some embodiments, the polymerization blocker comprises any chemical moiety in place of the 3' hydroxyl group that prevents an additional nucleotide from being added. In some embodiments, the polymerization blocker comprises an inverted nucleic acid residue. In some embodiments, the polymerization blocker is an inverted adenosine, thymine, cytosine, guanosine, or uridine residue. In certain embodiments, the polymerization blocker is an inverted thymine residue.

In addition to the portion that recognizes the ribosome, the second probe also comprises a portion that is complementary to a portion of the first probe. In some embodiments, the portion of the second probe that is complementary to a portion of the first probe is 4-20, 5-19, 6-18, 7-17, 8-16, 9-15, 10-14, or 11-13 nucleotides in length. In some embodiments, the portion of the second probe that is complementary to a portion of the first probe is 4, 5, 6, 7, 8, 9, 10, 11, 12, 13, 14, 15, 16, 17, 18, 19, or 20 nucleotides in length. In certain embodiments, the portion of the second probe that recognizes the ribosome and the portion of the second probe that is complementary to the first probe are joined by a poly-A nucleotide linker. In some embodiments, the poly-A nucleotide linker is 20-80, 30-70, or 40-60 nucleotides in length (*e.g.*, about 50 nucleotides in length).

The present disclosure contemplates any arrangement of the portions of the second probe. In some embodiments, the second probe comprises the structure:

5'-[portion recognizing ribosome]-[portion complementary to first probe]-3'; wherein ]-[ comprises an optional nucleotide linker. In some embodiments, ]-[ represents a direct linkage between two portions of the second oligonucleotide probe. In some embodiments, the second probe comprises the structure:

5'-[portion recognizing ribosome]-[portion complementary to first probe]-[polymerization blocker]-3';

wherein ]-[ comprises an optional linker (*e.g.*, nucleotide linker).

The first probe of the sets of probes described herein (also referred to herein as the "padlock" probe) includes a first oligonucleotide barcode sequence and a second oligonucleotide barcode sequence made up of a specific sequence of nucleotides. In some embodiments, the first and second oligonucleotide barcode sequences on the first probe comprise the same nucleotide sequence. The second barcode sequence of the first oligonucleotide probe may increase the specificity of the detection of the RNA of interest in the methods described herein (*i.e.,* as compared to if the method were performed using a first probe that did not comprise a second barcode sequence). The use of an additional oligonucleotide barcode sequence of the first oligonucleotide probe may also play a role in reducing non-specific amplification of the RNA of interest being translated by a ribosome. This is accomplished because the oligonucleotide barcode sequence of the third probe is complementary to the first oligonucleotide barcode sequence on the first probe, adding an additional layer of specificity that is required before amplification can occur, using the third probe as a primer. In some embodiments, the portion of the first probe that is complementary to a portion of the third probe is 5-15, 6-14, 7-13, 8-12, or 9-11 nucleotides in length. In some embodiments, the portion of the first probe that is complementary to a portion of the third probe is 5, 6, 7, 8, 9, 10, 11, 12, 13, 14, or 15 nucleotides in length.

The first probe also comprises an oligonucleotide portion that is complementary to the second probe and a portion that is complementary to an RNA of interest. In some embodiments, the portion of the first probe that is complementary to a portion of the second probe is 4-20, 5-19, 6-18, 7-17, 8-16, 9-15, 10-14, or 11-13 nucleotides in length. In some embodiments, the portion of the first probe that is complementary to a portion of the second probe is 4, 5, 6, 7, 8, 9, 10, 11, 12, 13, 14, 15, 16, 17, 18, 19, or 20 nucleotides in length. In certain embodiments, the oligonucleotide portion of the first probe that is complementary to the second probe is split between the 5' end and the 3' end of the first probe. In some embodiments, the portion of the first probe that is complementary to the RNA of interest is 10-30, 11-29, 12-28, 13-27, 14-26, 15-25, 16-24, 17-23, 18-22, or 19-21 nucleotides in length. In some embodiments, the portion of the first probe that is complementary to an RNA of interest is about 10, about 11, about 12, about 13, about 14, about 15, about 16, about 17, about 18, about 19, about 20, about 21, about 22, about 23, about 24, about 25, about 26, about 27, about 28, about 29, or about 30 nucleotides long. In some embodiments, the first probe is about 20, about 21, about 22, about 23, about 24, about 25, about 26, about 27, about 28, about 29, about 30, about 31, about 32, about 33, about 34, about 35, about 36, about 37, about 38, about 39, or about 40 nucleotides long. In some embodiments, the first probe comprises the structure:

5'-[portion complementary to second probe]-[first barcode sequence]-[portion complementary to portion of third probe]-[portion complementary to RNA of interest]-[second barcode sequence]-3';

wherein ]-[ comprises an optional linker (*e.g.*, nucleotide linker). In some embodiments, ]-[ represents a direct linkage between two portions of the first probe (*i.e.,* a phosphodiester bond).

The third probe of the sets of probes disclosed herein (also referred to herein as the "primer" probe) includes a barcode sequence made up of a specific sequence of nucleotides. In some embodiments, the barcode sequence of the third probe is about 3 to about 20, about 5 to about 15, about 6 to about 14, about 7 to about 13, about 8 to about 12, or about 9 to about 11 nucleotides in length. In some embodiments, the barcode sequence of the third probe is 3, 4, 5, 6, 7, 8, 9, 10, 11, 12, 13, 14, or 15 nucleotides in length. In certain embodiments, the barcode sequence of the third probe is 10 nucleotides in length.

The third probe also comprises a portion that is complementary to an RNA of interest and a portion that is complementary to a portion of the first probe. In some embodiments, the first and the third probes are complementary to and bind different portions of the RNA of interest. In some embodiments, the portion of the third probe that is complementary to the RNA of interest is 10-30, 11-29, 12-28, 13-27, 14-26, 15-25, 16-24, 17-23, 18-22, or 19-21 nucleotides in length. In some embodiments, the portion of the third probe that is complementary to an RNA of interest is about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, or about 30 nucleotides long. In some embodiments, the third probe is about 20, about 21, about 22, about 23, about 24, about 25, about 26, about 27, about 28, about 29, about 30, about 31, about 32, about 33, about 34, about 35, about 36, about 37, about 38, about 39, or about 40 nucleotides long. In some embodiments, the portion of the third probe that is complementary to a portion of the first probe is 5-15, 6-14, 7-13, 8-12, or 9-11 nucleotides in length. In some embodiments, the portion of the third probe that is complementary to a portion of the first probe is 5, 6, 7, 8, 9, 10, 11, 12, 13, 14, or 15 nucleotides in length.

In some embodiments, the third probe comprises the structure:

5'-[portion complementary to RNA of interest]-[portion complementary to first probe]-[barcode sequence]-3'

wherein ]-[ comprises an optional nucleotide linker. In some embodiments, ]-[ represents a direct linkage between two portions of the third probe. Any arrangement of the portions of the third probe is contemplated by the present disclosure.

In some embodiments, the present disclosure provides a plurality of probes comprising multiple pairs of probes as described herein. In some embodiments, the present disclosure provides a plurality of probes comprising multiple sets of probes as described herein. In certain embodiments, each pair or set of probes in the plurality of probes comprises oligonucleotide portions that are complementary to a different RNA of interest.

*Kits*

Also provided by the disclosure are kits. In one aspect, the kits provided may comprise one or more of the probes as described herein. In some embodiments, the kits comprise any of the pairs of probes described herein, or multiple pairs of probes. In some embodiments, the kits comprise any of the sets of probes described herein, or multiple sets of probes. In some embodiments, the kits may further comprise a container (*e.g.*, a vial, ampule, bottle, and/or dispenser package, or other suitable container). The kits may also comprise cells for performing control experiments. In some embodiments, the kits may further comprise other reagents for performing the methods disclosed herein (*e.g.*, enzymes such as a ligase or a polymerase, amine-modified nucleotides as described herein, primary antibodies, secondary antibodies, buffers, and/or reagents and monomers for making a polymeric matrix (*e.g.,* a polyacrylamide matrix)). In some embodiments, the kits are useful for profiling gene and protein expression in a cell. In some embodiments, the kits are useful for diagnosing a disease in a subject. In some embodiments, the kits are useful for screening for an agent capable of modulating RNA translation. In some embodiments, the kits are useful for diagnosing a disease or disorder in a subject. In some embodiments, the kits are useful for treating a disease or disorder in a subject. In certain embodiments, a kit described herein further includes instructions for using the kit.

*Systems*

In one aspect, the present disclosure provides systems for profiling RNAs being translated in a cell. In some embodiments, such a system comprises:

a) a cell;
b) one or more pairs of probes comprising a first probe and a second probe, wherein

i) the first probe comprises an oligonucleotide portion that is complementary to the second probe, an oligonucleotide portion that is complementary to an RNA of interest, and an oligonucleotide barcode sequence; and
ii) the second probe comprises a portion that recognizes the ribosome and an oligonucleotide portion that is complementary to a portion of the first probe;

c) a microscope; and
d) a computer.

In some embodiments, such a system comprises:

a) a cell;
b) one or more sets of probes comprising a first probe, a second probe, and a third probe, wherein

i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;
ii) the second probe comprises a portion that recognizes the ribosome and an oligonucleotide portion that is complementary to a portion of the first probe; and

iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe;

c) a microscope; and
d) a computer.

Any of the probes (*i.e.,* the pairs of probes or sets of probes) described herein may be used in the systems contemplated by the present disclosure. In some embodiments, the microscope is a confocal microscope. In some embodiments, the system further comprises a CPU. In some embodiments, the system further comprises computer storage and/or memory, or a storage device. In some embodiments, the system further comprises a camera. In some embodiments, the system further comprises a CCD. In some embodiments, the system further comprises software for performing microscopy and/or for image analysis. In some embodiments, the system further comprises a ligase. In some embodiments, the system further comprises a polymerase. In some embodiments, the system further comprises amine-modified nucleotides. In some embodiments, the system further comprises reagents for making a polymeric matrix (*e.g.*, a polyacrylamide matrix). The cell in the systems of the present disclosure may be of any of the cell types disclosed herein. In some embodiments, the system comprises multiple cells. In some embodiments, the cells are of different cell types. In certain embodiments, the cells are present in a tissue. In some embodiments, the tissue is a tissue sample provided by or from a subject. In certain embodiments, the subject is a human.

## EXAMPLES

*Example 1: Single-cell profiling of RNA translation status* in situ

*In situ* ribosome profiling was used to visualize active translation of beta-actin (ACTB) mRNAs (FIGs. 2A-2D). An oligonucleotide-conjugated ribosome antibody converts the binding of ribosomes to an amplifiable primer, which can be readily amplified and detected by fluorescent methods. A pool of padlock probes was designed to convert a targeted segment of sequence into a unique barcode. Combinatorial and sequential SEDAL sequencing rounds were then performed to read out the barcode and decipher the identity of the mRNA and the number of ribosomes on that mRNA.
To further improve the detection specificity of *in situ* ribosome profiling, a three-part probe strategy was developed and utilized (FIG. 3). The anti-ribosomal antibody-conjugated DNA probes are blocked at the 3' end and can thus only be used for padlock probe ligation. The intact padlock probe can then be amplified to generate DNA amplicons using a primer probe that hybridizes to a neighboring site. Thus, only ribosome-bound RNA will generate cDNA amplicons, while mRNA that is not bound by a ribosome cannot be ligated or amplified. The identity of each gene is encoded in the padlock and primer probes and read out by SEDAL *in situ* sequencing. To obtain subcellular localization information, organelle staining was incorporated into the *in situ* ribosome profiling procedure (FIG. 3). Overall, high specificity *in situ* single-cell translational states with subcellular localization information can be obtained using this improved strategy.
Eukaryotic ribosomes consist of a small 40S subunit and a large 60S subunit, which together contain approximately 80 structurally distinct proteins in total. Antibodies targeting both small subunit protein RPS3 (ribosomal protein S3) and large subunit protein RPL4 (ribosomal protein L4) were utilized to test the three-probe *in situ* ribosome profiling method. Both anti-RPS3 antibody and anti-RPL4 antibody were shown to work in this method (FIGs. 4A-4C and 5A-5C). A strong *in situ* ribosome profiling signal for ACTB using anti-RPS3 antibody or anti-RPL4 antibody (FIGs. 4A and 5A) was observed, while the negative control samples that used Immunoglobulin G (IgG) as the antibody showed weak signal (FIGs. 4B and 5B). Non-coding metastasis associated lung adenocarcinoma transcript 1 (MALAT1) RNA was also tested, and few signals were observed for MALAT1 using either of the anti-RPS3 or anti-RPL4 antibodies (FIGs. 4C and 5C), supporting the hypothesis that this approach would specifically detect translating mRNAs and not non-coding RNAs.
The antibody-based *in situ* ribosome profiling method described here can accurately detect ribosome-bound mRNA; however, the procedure relies on the specificity of anti-ribosome antibodies and requires extra steps of antibody-DNA conjugation. Since the ribosome consists of ribosomal RNA (rRNA) and ribosomal proteins, a procedure that utilizes rRNA-hybridized probes for padlock probe ligation was developed to avoid the antibody binding steps (FIG. 6). The procedure utilized the three-probe strategy described herein, and it is also compatible with the two-probe strategy described herein as well. Similar to the antibody-based approach, the rRNA-hybridized probe is blocked at the 3' end and cannot be used as the primer for rolling-circle amplification. Probes that hybridize to 18S rRNA were used to test the *in situ* ribosome profiling method. This procedure was found to give the best signal-to-noise contrast in comparison with the aforementioned approaches (FIGs. 7A-7C). A strong *in situ* ribosome profiling signal for ACTB was observed near the cell membrane (FIG. 7A), which is consistent with the localized translation of ACTB RNA. The negative control samples utilizing probes without the rRNA hybridization portion or that targeted the non-coding MALAT1 gene both showed minimal signal (FIGs. 9B and 9C).

*Example 2: Spatially Resolved Single-cell Translatomics at Subcellular Resolution*

The precise spatial control of mRNA translation is an integral part of post-transcriptional and translational gene regulation of cellular physiology. However, it remains a major challenge to systematically study localized mRNA translation at the transcriptomic scale in single cells. Thus, the development of RIBOmap is described herein. RIBOmap is a three-dimensional (3D) *in situ* profiling method to detect mRNA translation for thousands of genes simultaneously at subcellular resolution in intact cells and tissues. Using RIBOmap, 981 genes were mapped in human HeLa cells, revealing cell-cycle dependent and subcellular localized translation. Furthermore, single-cell spatial translatomes of 5,413 genes in the adult mouse brain tissue were created with a spatial cell atlas of 62,753 cells. Local translation in neuronal and glial cells was also detected. Together, RIBOmap presents the first spatially resolved single-cell translatomics technology, accelerating the understanding of localized protein synthesis in the context of subcellular architecture, cell states, and tissue anatomy. Overall, RIBOmap enables single-cell *in situ* profiling of mRNA translation in 3D for thousands of genes simultaneously in intact cells and tissues.

Cellular proteins are the final products of gene expression to execute cellular functions and shape cell types and states, and measuring genome-wide translational patterns with single-cell and spatial resolution is a paramount goal that could transform the understanding of translational regulation in heterogeneous cell types and states. While large-scale single-cell or spatially resolved proteome profiling remains challenging (*1*), it has been a common practice to use mRNA levels to infer the corresponding protein abundances in single cells. However, gene expression is regulated at both the transcription and translation levels, and numerous studies have concluded poor correlations between mRNA and protein levels (*2-5*). Moreover, many genes undergo localized translation at specific subcellular loci (*6*). Therefore, methods for scalable single-cell and spatially-resolved profiling of protein synthesis are needed for a comprehensive understanding of translational control.

Existing bulk and single-cell ribosome profiling methods have enabled analysis of protein translation at the transcriptome (*7-12*). However, these methods cannot preserve the spatial information of subcellular structure, cell morphology, or tissue organization, limiting the ability to chart protein synthesis in space. In contrast, imaging-based methods that can trace mRNA translation with their physical coordinates (*13-19*) are limited to one gene at a time. Therefore, it remains challenging to achieve highly multiplexed spatial ribosome profiling in single cells with subcellular resolution. To fill this gap, a new 3D *in situ* mapping method (RIBOmap) for highly multiplexed charting of protein and gene expression with subcellular resolution is described herein. The protein synthesis of 981 genes was profiled in intact cells with RIBOmap, and computational pipelines were developed to analyze the subcellular patterning of local translation. RIBOmap was also applied to profile 5,413 genes in intact mouse brain tissues at subcellular resolution, revealing spatial patterns of protein synthesis across cell types and tissue regions.

RIBOmap is built upon a targeted-sequencing strategy that uses a unique design of tri-probes, which selectively detects and amplifies ribosome-bound mRNAs, followed by hydrogel-tissue embedding and highly multiplexed *in situ* sequencing readout (*20*). In particular, the RIBOmap tri-probe set includes: (1) a splint DNA probe that hybridizes to ribosomal RNAs (rRNAs) and serves as the splint to circularize nearby padlock probes (see below); (2) a padlock probe that targets specific mRNA species of interest and encodes a gene-unique identifier; and (3) a primer that targets the adjacent site of the padlock probe on the same mRNA and serves as the primer to enable amplification of the circularized padlock probes through rolling circle amplification (RCA), producing a DNA nanoball (amplicon). Importantly, the gene-specific hybridization pair of primer-padlock probes exclude false-positive signals arising from the nonspecific hybridization of a single probe (*20*) (FIG. 9A). Together, the signal of RNAs will only be amplified to DNA amplicons when all three probes are present in proximity (FIGs. 9B and 9C). The DNA amplicons are subsequently embedded *in situ* in a polyacrylamide hydrogel matrix using tissue-hydrogel conjugation chemistry (*20*). The gene-unique barcodes in the DNA amplicons are then decoded through *in situ* sequencing with error-reduction by dynamic annealing and ligation (SEDAL, FIG. 9A) (*20*).

An alternative strategy for RIBOmap workflow was also designed, utilizing primary antibodies targeting ribosomal proteins, which are subsequently detected by splint-conjugated Protein A/G for tri-probe amplification (FIG. 12A). Specifically, this strategy was evaluated using anti-RPS3 (small 40S subunit ribosomal protein 3) antibody and anti-RPL4 (large 80S subunit ribosome protein 4) antibody (FIGs. 12B-12E). The signal-to-noise ratio (SNR) of the antibody-based strategy was in the range of 13.6-16.7. The SNR of the rRNA-targeting strategy was $93\pm17$ (FIG. 12F).

Next, the specificity of the RIBOmap tri-probe amplification for ribosome-bound mRNAs was evaluated. First, the detection specificity was tested using non-translating long non-coding RNAs (nuclear-localized *MALAT1* RNA and cytoplasmic localized *vtRNA1-1* RNA) as negative controls. RIBOmap and previously reported STARmap (20) imaging were performed for *ACTB* and negative control RNAs in HeLa cells to detect ribosome-bound RNAs, and total RNAs, respectively (FIG. 9D). It was found that RIBOmap only detected *ACTB* mRNAs, while STARmap detected both mRNA and non-coding RNAs (FIGs. 9E and 9F). Next, the sub-transcript specificity of RIBOmap in detecting ribosome-loading sites was validated. Here, Harringtonine was used, which is a translation inhibitor that traps the translation-initiating ribosomes at the start codon and allows the elongating ribosomes to run off the transcript (FIG. 9G). Three tri-probe sets were designed to target sub-transcript regions at different distances from the start codon (-16, 115, and 405 nt) (FIG. 1H). After 5 min of Harringtonine treatment (2 $\mu$g/ml final concentration), the *ACTB* mRNA showed a significant decrease in RIBOmap signal intensity when detected by the probes targeting 115 or 405 nt downstream of the start codon ($p$ = 7.5 X $10^{-5}$, 4.7 X $10^{-5}$ respectively, student's *t*-test), while signals generated by the probes targeting the -16 nt from the start codon showed little decrease (FIGs. 9I and 9J). Altogether, it was demonstrated that RIBOmap can specifically detect ribosome-bound mRNAs.

After benchmarking the SNR and specificity of RIBOmap, cell-cycle dependent and localized mRNA translation was profiled at subcellular resolution in single cells by performing a highly multiplexed 981-gene RIBOmap experiment in HeLa cells (FIG. 10A). The 981 genes represent a curated list composed of cell-cycle gene markers, genes with diverse subcellular patterns, and genes of varying RNA stabilities (*21-24*). A multi-modal RIBOmap experiment was designed that further incorporates the information of cell-cycle stages and subcellular organelles: (1) the cell-cycle phase of each cell was captured by the fluorescent ubiquitination-based cell cycle indicators (FUCCI) (*25*, *26*); (2) ribosome-bound mRNAs (981 genes) were *in situ* sequenced after FUCCI imaging (*20*); (3) finally, nucleus, endoplasmic reticulum, and cell shape were stained and imaged (Fig. 2B). All three imaging modalities were registered by the shared channel of nuclear (DAPI) staining. To compare spatial translatome versus spatial transcriptome, a paired total RNA mapping of the same 981 genes was conducted using STARmap (with identical RNA hybridization sequences) for the same batch of HeLa FUCCI cells (FIG. 10A). In total, 1,813 cells were sequenced by RIBOmap (1,500 ribosome-bound RNA reads per cell on average) and 1,757 cells by STARmap (2,349 RNA reads per cell on average) after quality-control filtration.

First, it was evaluated whether RIBOmap could dissect cell cycle-dependent mRNA translation. To this end, the single-cell translatomic profiles generated by RIBOmap and the single-cell transcriptomic profiles generated by STARmap were subjected to single-cell trajectory analysis (*21*). In both RIBOmap and STARmap samples, the single-cell profiles of known cell-cycle gene markers (*21*) were used to embed single cells on diffusion maps with pseudotime values to delineate cell-cycle progression (FIG. 10C and FIGs. 13A and 13B). For both datasets, the RNA-defined G1, S, and G2/M cell-cycle phases were consistent with the expected cell-cycle-dependent patterns of FUCCI protein fluorescence (FIG. 10C), demonstrating the capability of RIBOmap in detecting cell states.

After confirming the accuracy of RIBOmap in delineating cell cycle progression, the co-regulated gene modules were identified by covariation analysis. The pairwise correlations for all gene pairs based on single-cell translatomic expression were calculated, and hierarchical clustering was used to identify genes with high correlation coefficients across single cells. Notably, five gene modules were found with substantial intra-module correlation and enrichment of distinct functional pathways (Module 1-5, FIG. 10D and FIGs. 14A to 14D). In particular, genes in Modules 3 and 5 were enriched with G2/M and G1/S cell-cycle marker genes, respectively (FIG. 10E). These two gene modules are also negatively correlated (FIG. 14E). This result provided single-cell level evidence supporting the previous finding that these cell-cycle marker genes are co-regulated during cell-cycle progression for synchronized execution of their gene functions (*27*). Moreover, Module 2 contains genes encoding protein translation machinery such as ribosomal proteins (RPS3, RPS28, and RPL37) and translational factors (EEF2, EIF3B, and ETF1) (FIG. 14C). These genes show a positive correlation with Module 5 (G1/S marker genes) and a negative correlation with Module 3 (G2/M marker genes) (FIG. 14F). This implies that the protein translation machinery is more actively produced in the G1/S phase to supply the protein production for the physical expanding and replicating of subcellular organelles.

Leveraging the high spatial resolution of the dataset, it was explored whether RIBOmap could detect subcellular patterns of translation. To identify translating genes that show a tendency to co-localize (exhibit spatial proximity) in the subcellular space, the nearest neighbor profile for each RNA in RIBOmap was generated, and the significance of gene colocalization was evaluated by comparing against a randomized control. With subsequent hierarchical clustering of the gene co-localization matrix, five gene modules were identified with highly correlated subcellular spatial organizations and distinct functional enrichment (Modules 1-5, FIGs. 10E-10G and FIGs. 15A-15C). Among them, Modules 2, 4, and 5 are functionally enriched with membrane protein and secretion pathway (FIGs. 10F and 10G and FIGs. 15A and 15B) and physically co-localized with ER staining (FIGs. 10H and 10I and FIGs. 14D and 14E), suggesting they are ER-translated genes. While Module 1 and 3 genes are enriched in big protein complexes of mitotic spindle (*e.g., CDK1, NUSAPI, KIF23, SPAG5, TACC3, FAM83D)* and translation machinery (*e.g., RPS3, RPS28, RPL36A, EEF2, DHX9),* respectively (FIGs. 10F and 10G and FIGs. 15A and 15C). Such an observation indicates that subunits of protein complexes may be produced in spatial proximity for efficient assembly. Finally, it was explored whether the co-regulated genes tended to be co-localized for translation. To this end, the co-localization *p*-values from the subcellular colocalization analysis were plotted into the single-cell covariation gene matrix. It was found that the five gene modules with high single-cell covariation correlation also show strong subcellular colocalization (FIG. 16F). These results imply that functionally related gene groups can be co-regulated through subcellular co-localized translation.

Next, it was determined whether RNAs with $N^6$-Methyladenosine modification (m$^6$A), a critical post-transcrip-

tional RNA modification (28), would have distinct spatial translation patterns. To this end, the translation distribution of each gene was quantified with a distance-ratio (DR) metric, which estimated the relative position of each amplicon between nuclear and cytoplasmic membranes (FIG. 10J). Notably, it was observed that the DR values of $m^6A$ genes were significantly lower than those of the non-$m^6A$ genes (FIG. 10K), indicating that $m^6A$ genes are physically translated closer to the nuclear membrane. It was hypothesized that this difference could be explained by the shorter lifetime and thus shorter trafficking time of $m^6A$-modified RNA in the cytosol (23). Together, the capability of RIBOmap for integrative analysis of spatial translatomics at subcellular levels was demonstrated, providing an unprecedented approach for future studies on RNA processing and translation.

To further demonstrate the spatial profiling power of RIBOmap in analyzing tissue samples, this approach was applied to mouse brain slices to reveal *in situ* single-cell translatomic profiles. Here, a targeted gene list of 5,413 genes curated from previously published single-cell sequencing studies of mouse cell atlas (*29-35*) was mapped. Through nine rounds of *in situ* sequencing, a 10 um-thick coronal section of the mouse left hemisphere (27.6 mm², 62,753 cells) containing multiple brain regions was imaged at 90 nm X 90 nm X 300 nm voxel size (FIG. 1A). As expected, minimal RIBOmap signals were observed in the nuclei in contrast to the dense signals in the cytosol, where protein translation occurs (FIG. 11B).

First, tissue RIBOmap data was benchmarked by comparing the spatial translation pattern of well-known cell-type marker genes (*e.g., Slc17a7, Gad2,* and *Pcp4*) with *in situ* hybridization (ISH) images of the corresponding genes from the Allen Brain database (36) (FIG. 11C and FIG. 16A), where the comparison showed consistent spatial patterns. Strikingly, it was observed that RIBOmap reads of specific genes correlated better with the protein signals than the RNA signals (ISH) in specific brain regions, such as *Sst* in CA3and *Nefl* in CA1 of the hippocampus (FIGs. 16B and 16C). This result is consistent with previous reports that the ribosome profiling results correlated better with protein abundance than RNA-seq data (*7, 11, 12*). Next, all 62,753 cells were subjected to brain cell-type identifications (FIG. 17). Here, a hierarchical clustering strategy was adopted (*20, 31*): Level-1 clustering classified cells into neuronal cells and glial cells (FIGs. 18A and 18B); Level-2 clustering identified excitatory neurons, inhibitory neurons, astrocytes, oligodendrocytes, oligodendrocyte precursor cells, microglia, and vascular cells (FIGs. 18A, 18C, and 18D); Level-3 clustering identified 57 distinct subtypes (FIGs. 11D and FIGs. 19-21). Based on the cell typing results, a spatial cell map from the imaged hemibrain region was generated (FIG. 11E). The spatial distribution of these translatomically defined cell types is largely as expected. For example, the cortex is enriched with layer specific excitatory neurons (Layer 2/3: *Cplx2+,* Layer 4/5: *Dkk3+,* Layer 6: *Nr4a2+,* Layer 6: *Pcp4+*), corpus callosum with oligodendrocytes (*Mbp+, Mal+, Cnp+, Plp*1+), striatum with medium spiny neurons (*Tac1+, Adora2a+, Ppp1r1b+, Penk+, Rasd2+*), thalamus with excitatory neurons (*Prkcd+, Synpo2+*), and hypothalamus with peptidergic neurons (*Hap1+, Tac1+, Dlk+*). Region-specific excitatory neurons in the pyramidal layer of CA1-3 and granular layer of DG, respectively, were also observed. Thus, the potential of RIBOmap to identify diverse brain cell types based on the single-cell translatomes was demonstrated as an alternative strategy for generating spatial tissue atlases.

Subcellular localized mRNA translation is crucial for both neuronal cell (*37-39*) and glial cell functions (*40-42*). Notably, the subcellular resolution of RIBOmap enabled investigation of localized translation in the somata and processes of neuronal and glial cells. To this end, the RIBOmap reads were divided into somata-reads (*i.e.,* inside the cell body area identified by ClusterMap (*43*)) and processes-reads (*i.e.,* the rest of the reads) (FIGs. 11F and 11G). Next, the top 10% of genes with the highest processes-to-somata ratios were defined as processes-enriched-translation genes, whereas the top 10% of genes with the lowest processes-to-somata ratios were defined as somata-enriched-translation genes (FIG. 11H). Gene Ontology (GO) analysis showed that processes-enriched-translation genes are associated with translation, synapse, and postsynaptic density (FIG. 11I). In contrast, somata-enriched-translation genes are associated with the plasma membrane, extracellular matrix, and endoplasmic reticulum (FIG. 11J). This result further validated the capability of RIBOmap in detecting subcellular localized translation of tissue samples, as membrane and secreted proteins are expected to be translated at ER in the somata region. Notably, an exceptionally high abundance of processes-enriched-translation signals was observed in hippocampal neuropils, such as postsynaptic density proteins (*e.g., Shank1, Dlg4,* and *Grik5*), translation machinery protein (*e.g., Eef2, Eef1a, Rpl3,* and *Rps5*), motor proteins (*e.g., Kif5a* and *Kif1a*), and calcium sensor and signaling proteins (*e.g., Calm1* and *Camk2n1*) (FIG. 11K and FIG. 22A). As a comparison, the somata-enriched-translation genes showed sparse RIBOmap signals in hippocampal neuropils (FIG. 11L and FIG. 22B), such as *App,* which encodes a transmembrane precursor protein and is associated with Alzheimer's Disease, and *Rtn4,* which is known to be associated with the endoplasmic reticulum. Besides these example genes that are translated in neurons, the localized translation of glial cell marker genes was also observed (FIGs. 11M and 11N and FIGs. 22C and 22D). For oligodendrocytes, the marker genes *Mbp* and *Plekhb1* were identified as processes-enriched-translation genes, while *Mal* and *Cnp* were somata-enriched-translation genes (FIGs. 11M and 11N and FIGs. 22C and 22D). Astrocytes have many processes, and their marker genes were enriched in the processes-enriched-translation category, like *Gfap, Ttyh1, Apoe,* and *Clu* (FIG. 11M and FIG. 22C). For vascular cells, their marker genes are enriched in the somata-enriched-translation gene group, like *Ptgds, Itm2a,* and *Bsg* (FIG. 22C). Overall, it was demonstrated that RIBOmap is a powerful tool to study localized translation in processes of both neuronal and glial cells in the mouse brain tissue.

With the development of RIBOmap, a novel spatial-omic method, spatial mRNA translation can be mapped at the single-cell and subcellular levels. It was demonstrated herein that RIBOmap enables a critical step toward a more comprehensive understanding of mRNA regulation and protein synthesis by examining the translation of 981 genes during the cell cycle and revealing the distinct subcellular distribution patterns of translating RNAs in human cells. To illustrate this, the present disclosure demonstrates the spatial profiling of translatomic states *in situ* in intact tissue samples with high 3D spatial resolution, generating a translatome-defined spatial cell map in mouse brain tissue. Moreover, compared to the Ribo-STAMP and TRAP methods, RIBOmap does not rely on genetic manipulation.

**Materials and Methods**

*Cell culture*

Human HeLa cells were obtained from ATCC (CCL-2) and were cultured in DMEM supplemented with 10% FBS at 37°C and 5% $CO_2$. Harringtonine treatment was performed by adding the drug to a final concentration of 2 $\mu$g/ml and incubating at 37°C for 5 min.

The HeLa FUCCI cells were generated using lentiviral particles as previously described (26). Briefly, HEK 293T cells were transfected with pBOB-EF1-FastFUCCI-Puro (obtained from Addgene, #86849) and packaging plasmids (psPAX2 and VSVG). Supernatant from the culture medium containing the virus was collected 48 hours post-transfection. The supernatant was filtered through a 0.45 $\mu$m PES filter, and the viral supernatant was used to transduce HeLa cells. Following puromycin selection, the stable cell line was expanded.

*Mice*

All animal procedures followed animal care guidelines approved by the Institutional Animal Care and Use Committee (IACUC) of the Broad Institute of MIT and Harvard under animal protocol # 0255-08-19. Animal experiments were conducted in compliance with IACUC policies and NIH guidelines. The C57BL/6 (male, 16 weeks old) mouse used for RIBOmap in this study is purchased from The Jackson Laboratory (JAX).

*Chemicals and enzymes*

Chemicals and enzymes are listed as name (vendor, catalog number): DMEM (Gibco, 11995), Harringtonine (Abcam, ab141941). Lipofectamine MessengerMAX Transfection Reagent (Thermo Scientific, LMRNA003). Tissue-Tek O.C.T. Compound (SAKURA, 4583). Glass bottom 24-well plates (Cellvis, P24-1.5H-N). Glass bottom 12-well and 24-well plates (MatTek, P12G-1.5-14-F). 3-(Trimethoxysilyl)propyl methacrylate (Sigma, M6514). poly-D-lysine (Sigma-Aldrich, A-003-M). 16% PFA (Electron Microscope Sciences, 15710-S). Methanol (Sigma-Aldrich, 34860-1L-R). PBS (Gibco, 10010-023). Tween-20, 10% solution (Calbiochem, 655206). Yeast tRNA (Thermo Scientific, AM7119). SUPERase·In RNase Inhibitor (Thermo Scientific, AM2696). 20xSSC (Sigma-Aldrich, S6639). Formamide (Calbiochem, 655206). Ribonucleoside vanadyl complex (New England Biolabs, S1402S). T4 DNA ligase, 5 Weiss U/$\mu$L (Thermo Scientific, EL0012). Phi29 DNA polymerase (Thermo Scientific, EP0094). 10 mM dNTP mix (Thermo Scientific, 18427089). UltraPure BSA (Thermo Scientific, AM2618). 5-(3-aminoallyl)-dUTP (Thermo Scientific, AM8439). Methacrylic acid NHS ester, 98% (Sigma-Aldrich, 730300). DMSO, anhydrous (Molecular Probes, D12345). Acrylamide solution, 40% (Bio-Rad, 161-0140). Bis Solution, 2% (Bio-Rad, 161-0142). Ammonium persulfate (Sigma-Aldrich, A3678). N,N,N',N'-Tetramethylethylenediamine (Sigma-Aldrich, T9281). OminiPur SDS, 20% (Calbiochem, 7991). Proteinase K Solution, RNA grade (Thermo Scientific, 25530049). Antarctic Phosphatase (New England Biolabs, M0289L). DAPI (Molecular Probes, D1306). 10% Triton X-100 (Sigma-Aldrich, 93443). Concanavalin A, Alexa Fluor 594 Conjugate (Thermo Scientific, C11253). Flamingo Fluorescent Protein Gel Stain (Bio-Rad, 1610490). DL-Dithiothreitol (Sigma-Aldrich, D9779). 0.5ml 30-kDa Amicon Ultra Filters (Millipore, UFC503024). Ribosomal protein L4 Polyclonal antibody (Thermo Scientific, 11302-1-AP). RPS3 Monoclonal Antibody (Thermo Scientific, 66046-1-IG). Micro Bio-Spin™ P-6 Gel Columns, Tris Buffer (Bio-Rad, 7326221). Pierce™ SM(PEG)$_2$, No-Weigh™ Format (Thermo Scientific, A35397). Zeba™ Spin Desalting Columns, 7K MWCO, 0.5 mL (Thermo Scientific, 89882).

*RIBOmap and STARmap probe design*

RIBOmap was performed using the tri-probes strategy that contained splint probes and SNAIL probes. The splint probe consisted of three parts: a 25 nt sequence at the 5' end that is hybridized to 18S ribosomal RNA (rRNA), a 50 nt deoxyadenosine (dA) sequence in the middle, and a 12 nt splint-padlock annealing sequence at the 3' end. The design of the 25 nt sequence that hybridized to 18S rRNA was applied as previously described (44). The probes are targeted to the regions on 18S rRNA that are relatively unstructured and accessible for chemical modification. Five unique probes were designed in total. The 3' end of the splint probes are blocked by 3' Inverted dT modifications and cannot be used as primer for RCA amplification. The splint probes were synthesized by Integrated DNA Technologies (IDT).

The design of SNAIL probes was applied as previously described (20). In brief, Picky 2.2 was used to design the hybridization sequence of each SNAIL probe pair with the length set at 40-46 nt, then the resulting sequence was split into halves of 20-25 nt and separated by 0-2 nt, with the best matching of melting temperature (Tm) between the two halves. For the HeLa cell 981-gene experiments, 5 or 6 pairs of SNAIL probes were designed for each gene. For the mouse brain 5,433-gene experiments, 4 pairs of SNAIL probes were designed for each gene. The SNAIL probes were synthesized and pooled by IDT.

*Sample preparation of RIBOmap and STARmap for cell culture*

The HeLa FUCCI cells were cultured in 24-well plates, washed with PBS before sample collection, and fixed with 300 $\mu$l 1.6% PFA in PBS buffer at room temperature for 15 minutes. After fixation, the cells were permeabilized with 450 $\mu$l of cold methanol and incubated at -20°C for an hour. The HeLa FUCCI cell samples were then taken from -20°C to room temperature for 5 min, then quenched by 200 $\mu$l quenching solution (1 mg/ml Yeast tRNA, 0.1 U/$\mu$l SUPERase·In RNase Inhibitor, 100 mM Glycine, 0.1% Tween-20 in PBS) at room temperature for 10 min. After quenching, the samples were incubated with 200 $\mu$l of 1X hybridization buffer (2xSSC, 10% formamide, 20 mM Ribonucleoside vanadyl complex, 0.1 mg/ml yeast tRNA, 0.5% SUPERaseIn, 0.1% Tween-20, pooled SNAIL probes at 1 nM per oligo and splint probe for RIBOmap sample at 1$\mu$M per probe) at 40 °C in a humidified oven with parafilm wrapping and shaking for 12 h. The samples were washed by 300 $\mu$l PBSTR (0.1 U/$\mu$l SUPERase·In RNase Inhibitor, 0.1% Tween-20 in PBS ) twice at and 300 $\mu$l high-salt washing buffer (4X SSC dissolved in PBSTR) once at 37 °C, 20 min for each wash. Finally, the samples were rinsed once with 300 $\mu$l PBSTR at room temperature.

The samples were then incubated with a 200 $\mu$l ligation mixture (0.25 U/$\mu$l T4 DNA ligase, 0.5 mg/ml BSA, and 0.4 U/$\mu$l of SUPERase·In RNase inhibitor in 1X T4 DNA ligase buffer) at room temperature for two hours with gentle shaking. After the ligation reaction, the samples were washed twice with 300 $\mu$l PBSTR and then incubated with 200 $\mu$l rolling circle amplification (RCA) mixture (0.5 U/$\mu$l Phi29 DNA polymerase, 250 $\mu$M dNTP, 20 $\mu$M 5-(3-aminoallyl)-dUTP, 0.5 mg/ml BSA and 0.4U/$\mu$l of SUPERase·In RNase inhibitor in 1X Phi29 buffer) at 4 °C for 30 min and 30 °C for two hours with gentle shaking. After that, the samples were washed twice with PBST (0.1% Tween-20 in PBS). Then imaging was performed to record the FUCCI fluorescence signal for the RIBOmap sample and STARmap sample simultaneously. Subsequently, the samples were treated with 200 $\mu$l modification mixture (25 mM Methylacrylic acid NHS ester in 100 mM Sodium bicarbonate buffer) at room temperature for one hour and then rinsed once by PBST. The samples were incubated with 150 $\mu$l monomer buffer (4% acrylamide, 0.2% bis-acrylamide in 2X SSC) at room temperature for 15 min. Then the buffer was aspirated, and 25 $\mu$L polymerization mixture (0.2% ammonium persulfate, 0.2% tetramethylethylenediamine dissolved in monomer buffer) was added to the center of the sample and immediately covered by Gel Slick-coated coverslip. The polymerization reaction is performed for one hour at room temperature in a $N_2$ box, then washed by PBST twice for 5 min each.

The tissue-gel hybrids were then digested with 200 $\mu$l Proteinase K mixture (0.2 mg/ml Proteinase K, 1% SDS, 100mM NaCl, and 50 mM Tris) at 37°C for one hour, then washed by PBST three times for 5 min each. Subsequently, the samples were treated with 200 $\mu$l dephosphorylation mixture (0.25 U/$\mu$L Antarctic Phosphatase, 0.5 mg/mL BSA in 1X Antarctic Phosphatase buffer) at 37 °C for 1 hour and washed by PBST three times for 5 min each.

For SEDAL sequencing, each sequencing cycle began with treating the sample with 200$\mu$l stripping buffer (60% formamide, 0.1% Triton X-100 in $H_2O$) at room temperature twice for 10 min each, followed by washing with PBST three times for 5 min each. Then the samples were incubated with a 200 $\mu$l sequencing mixture (0.1875 U/$\mu$l T4 DNA ligase, 0.5 mg/ml BSA, 10 $\mu$M reading probe, and 5 $\mu$M fluorescent oligos in 1X T4 DNA ligase buffer) at room temperature for at least 3 hours. The samples were washed by 300 $\mu$l washing and imaging buffer (10% formamide in 2X SSC buffer) three times for 10 min each, then immersed in washing and imaging buffer for imaging. Images were acquired using Leica TCS SP8 confocal microscopy with 40X oil immersion objective (NA 1.3) and voxel size of 94.64 nm X 94.64 nm X 350 nm. The DAPI signal was imaged at the first round of sequencing. Six cycles of imaging were performed to detect 981 genes.

After SEDAL sequencing, the samples were treated with 200 $\mu$l stripping buffer three times at room temperature for 10 min each. The sample was then washed with PBST three times for 5 min each and incubated with 200 $\mu$l ER staining mixture (0.05 mg/ml concanavalin A diluted in 100 mM sodium bicarbonate buffer), then washed by PBST twice for 5 min each. Subsequently, the sample was incubated with 200 $\mu$l Flamingo staining mixture (1x Flamingo Fluorescent Gel Stain in washing and imaging buffer) at room temperature overnight, then washed by 300 $\mu$l PBST three times.

*Sample preparation of RIBOmap for mouse tissue*

The mice used in this study were anesthetized with isoflurane and then rapidly decapitated. The mouse brain tissue was collected and placed in Tissue-Tek O.C.T. Compound. Then the brain tissue in O.C.T. was frozen in liquid nitrogen and kept at -80 °C. For mouse brain tissue sectioning, the brain tissue was transferred to cryostat (Leica CM1950) and cut into 20 μm coronal sections at -20 °C. The slices were transferred and attached to glass-bottom 12-well plates pretreated by 3-(Trimethoxysilyl)propyl methacrylate and poly-D-lysine. The brain slices were fixed with 4% PFA in PBS at room temperature for 15 min, then permeabilized with cold methanol and placed at -80 °C for an hour. The experimental procedures for mouse brain tissue are almost the same as for HeLa cells, except that all the reaction volumes were doubled as the brain tissues were prepared in 12-well plates. No imaging procedures were performed for fluorescent protein, and no organelle staining procedures were involved. Images were acquired using Leica TCS SP8 confocal microscopy with 63X oil immersion objective (NA 1.3) and voxel size of 90.14 nm X 90.14 nm X 300 nm. The DAPI signal was imaged at the first round of sequencing, and nine cycles of imaging were performed to detect the 5,413 genes.

*Antibody-based RIBOmap strategy for cell culture*

The splint probe-conjugated protein A/G was prepared first. In brief, 5'-thiol modified splint probe (IDT) was activated by DL-Dithiothreitol (DTT) for 2h at room temperature and purified by Micro Bio-Spin™ P-6 Gel Columns to remove excess DTT. Protein A/G was reacted with PEGylated SMCC crosslinker [SM(PEG)$_2$] for 2h at 4 °C, and then purified by Zeba desalting columns to remove excess cross-linkers. Activated splint probe was incubated with protein A/G (molar ratio of protein A/G to splint probe of ~1:11) overnight at 4 °C. The final conjugated protein A/G was washed by using 0.5ml 30-kDa Amicon Ultra Filters five times to remove non-reacted DNA oligonucleotides.

The fixation step is the same as for the rRNA probe-based RIBOmap strategy. For the hybridization step, only the SNAIL probes but not the splint probes were added. The other reagents for this step are the same as for the rRNA probe-based RIBOmap strategy. After hybridization, ribosomal protein (RPS3 or RPL4) antibody incubation was performed. Cell samples were blocked with blocking solution (5 mg/ml BSA in PBSTR) for 30 minutes at room temperature, and then stained with 1:100 diluted primary antibody (RPS3 or RPL4, IgG for the control sample) in the blocking solution for 1h at 4 °C. The samples were washed with PBSTR three times at room temperature for 5 min each. The samples were then incubated with splint probe-conjugated protein A/G for 1 h at room temperature and washed with PBSTR three times at room temperature for 5 min each. The ligation step and RCA step are the same as that for the rRNA probe-based RIBOmap strategy. The samples were then incubated with 50 nM fluorescent oligo complementary to DNA amplicon and DAPI in PBST. The DNA amplicon signal was detected by imaging.

*RIBOmap and STARmap Imaging Preprocessing.*

Image deconvolution was achieved with Huygens Essential version 21.04 (Scientific Volume Imaging, The Netherlands, svi.nl), using the CMLE algorithm, with SNR:10 and 10 iterations. According to previous reports, similar Image registration, spot calling, and barcode filtering with minor adjustments were applied (20).

*Data processing for RIBOmap/STARmap cell culture samples*

*3D Cell Segmentation:* Image segmentation was achieved with a strategy combining 2D reference segmentations generated by CellProfiler v4.1.3, and 3D staining images were processed with customized MATLAB scripts. First, a composite image combining amplicon channels and a flamingo fluorescent gel staining image was created to represent the cell boundary. The maximum projections of both the 3D DAPI staining image and composite image were fed into a customized pipeline created in CellProfiler v4.1.3. A median filter was used to remove the high-frequency noise on the images and the *IdentifyPrimayObjects* function was used to detect the cell nucleus. Then, based on the identified nucleus, cell boundaries were outlined by applying the *IdentifySecondaryObjects* function to the filtered 2D composite image. Both the nucleus and cell segmentation masks in the output were used as reference segmentations in the following procedures to create 3D segmentation.

For 3D segmentation, the images targeting different cellular compartments (DAPI staining for nucleus, composite image for cell boundary, and Concanavalin A staining for ER) were first processed by a median filter and binarized with the manually curated threshold. All connected components (objects) that had fewer than 200 voxels were removed from the binary image. Then, images were dilated with a disk structure element with radius equal to 10. A 3D segmentation mask targeting each cellular region was then generated by an element-wise multiplication process between the binary image and the 2D reference cell segmentation from the previous step. The nucleus regions were removed from the 3D cell segmentation masks to create the cytoplasm segmentation. Lastly, a per-cell gene expression matrix in each cellular compartment was created by quantifying filtered amplicons overlapping each labeled region in 3D.

*Protein signal quantification of FUCCI cell line:* Both mAG and mKO2 fluorescence images were aligned with sequencing images through image registration according to previous reports. The signal level of each protein was quantified as the integrated intensity of the voxels overlapping with the 3D nucleus segmentation mask.

*Quality control of single cells:* Single cell profiles measured by both RIBOmap and STARmap methods were filtered based on the following metrics and thresholds respectively to ensure a high-quality library. First, cells with physical volume defined by 3D segmentation outside the desired range (from $0.5 \times 10^6$ to $2 \times 10^6$ voxels) were excluded. Then, a sample specific threshold for the number of transcripts per cell was used to remove outliers in each dataset (RIBOmap lower boundary (LB): 300, upper boundary (UB): 3500, STARmap LB: 500, UB: 6000). Additionally, the transcript density was defined as the ratio between the number of transcripts and the physical volume of each cell, which was used as a threshold metric (LB: 0.00055) to further exclude cells. The genes were filtered based on the percentage of cells that expressed them (LB: 10%) and their maximum count (RIBOmap LB: 2; STARmap LB: 4) in a cell. The difference in filtering criteria for RIBOmap and STARmap came from different expectations for each technique. After filtering, 1,813 cells for the RIBOmap dataset and 1,757 cells for STARmap with 897 genes entered subsequent analyses.

*Covariation analysis:* To identify covarying gene modules, the expression matrix was first normalized to the same total number of transcripts per cell, then the Pearson correlation coefficient across each gene pair was calculated to measure the similarity in cell-to-cell variation. A hierarchical clustering with *ward linkage* was performed on RIBOmap with *n_cluster* set to 10.

*Colocalization analysis:* Permutation analysis was performed to uncover the co-localized reads clusters. Given one read inside a cell, the gene identities of neighbor reads (within 3 $\mu$m radius) were recorded. By screening all the cells, the counts of each neighbor gene pair, which were identified as the observed count, were obtained. Next, a 1000-times permutation test was performed to randomly shuffle the gene labels of the RNA reads without disturbing their subcellular locations. For each permutation round, the counts of each neighbor gene pair were collected for generating the permutation distribution. Afterwards, the one-tail p-value of each gene pair was inferred by comparing the observed count with the permutation distribution. The pairwise p-values were further used to construct the heatmaps (FIG. 10E). The rows and columns in the heatmap of the RIBOmap sample were ordered by hierarchical clustering. The heatmap for the STARmap sample used the same order as RIBOmap to detect the gene modules with spatial distribution differences. To quantitatively compare the endoplasmic reticulum (ER)-localization level among gene modules, the percentage of reads localized in ER for genes in target gene modules at the single cell level was calculated. The same percentage for all genes was used as a comparison baseline. The Wilcoxon signed-rank test was performed for testing statistical significance.

*Cell cycle and pseudotime:* To construct a pseudotime trajectory representing cell cycle progression, cell cycle phases (G1, G1S, G2M) were first identified using the expression profiles of cells restricted to cycle marker genes (*score_genes_cell_cycle* function in Scanpy). Next, STARmap cells were embedded in a diffusion map constructed by the expression of the same set of genes. After converting this diffusion map to a polar coordinate system, a 'root' cell representing the start of a cell cycle was identified, and a pseudotime value was assigned to each cell according to their angle to the 'root' cell on the polar coordinate. To assign pseudotime values for RIBOmap cells on the same trajectory, cells from two datasets were registered on the same embedding defined by the mAG and mKO2 fluorescence intensity. The pseudotime value of each RIBOmap cell was defined as the averaged values of its nearest neighbors (k = 3) in STARmap.

*DR calculation:* To quantitatively analyze the localization of RNA reads inside the cytoplasm, a distance-ratio (DR) was calculated for each cytoplasmic read. The DR value for a read was defined as the shortest distance of the read to the nucleus boundary ($d_1$), determined by nucleus segmentation, normalized by a sum of this distance and the shortest distance of the read to the cell membrane ($d_2$), determined by cell segmentation. The Euclidean distance transform function (*distance_transform_edit*) in Scipy was used to calculate the shortest distance between each read and target surfaces defined by 3D segmentation.

*Data processing for RIBOmap brain tissue samples*

*Cell Segmentation with ClusterMap:* An RNA amplicon-based segmentation method (ClusterMap (43)) was used to achieve single-cell signal quantification. A basic processing pipeline involving background signal rejection, DAPI signal sampling, and density-based segmentation was applied to each field of view (FOV). Specifically, 10% of the RNA signals were identified as locally low-density signals and were excluded in single-cell level quantification. Additional signal spots were sampled from coupled DAPI staining images with setting parameter *dapi_grid_interval* equal to 5 to further improve the segmentation accuracy. During segmentation in 3D, parameter *cell_num_threshold* was set to 0.02 based on qualitative testing compared with DAPI staining. Algorithm-identified cells with less than 5 transcripts or not overlapping with coupled DAPI staining were excluded. ClusterMap signal assignment of all FOVs were merged to create a cell-by-gene matrix.

*Quality control and preprocessing:* After signal quantification at the single-cell level, the number of transcripts and genes per cell were used to exclude low-quality cells. In detail, the median absolute deviation (MAD) was used to estimate the threshold for reads per cell filtering as shown by the following equations:

$$Lower\ boundary\ =\ median(reads\ per\ cell)\ -\ 4 * MAD$$

**EP 4 696 786 A2**

$$Upper\ boundary\ =\ median(reads\ per\ cell)\ +\ 4*MAD$$

Other standard filtering strategies were also applied such as:

1. A minimum of 10 genes expressed is required for a cell to pass filtering; and
2. A minimum of 10 cells expressed is required for a gene to pass filtering.

After filtering, a transcriptome profile with 62,753 cells and 5,413 genes was obtained. To ensure a high-quality gene library, the genes with a maximum count of less than 3 were further excluded across cells, resulting in 3,995 genes used in the downstream analysis such as cell type classification. The transcriptome profile was then normalized according to the median of the number of transcripts per cell by the *pp.normalize_total* function (Scanpy v1.8.2). Then the logarithmic transformation was performed on the data with the *pp.log1p* function. Finally, the data matrix was scaled to unit variance by *pp.scale* function and was used in downstream analysis such as dimensionality reduction, and unsupervised clustering.

*Cell type classification:* A hierarchical clustering strategy was applied to generate a three-level cell-type annotation for the RIBOmap mouse brain dataset. First, the scaled matrix restricted to the 3,995 highly abundant genes was used to perform a principal component analysis (PCA). The function *tl.pca* was used to calculate the top 30 principal components, and all of them were used to construct a k-nearest neighbor (k-NN) graph where cells were connected with their neighbors in high dimensional space based on their transcriptomic profiles' similarity. The Leiden community detection algorithm was then applied over the kNN graph to detect cell clusters. Based on the expression profile of the canonical markers for common cell types in the brain (*i.e., Slc17a7, Gad1, Gad2, Plp1, Slc1a3,* etc.), each cluster was classified into either neuron or glial cells as their first level annotation.

To assign more detailed annotations, the same analyses described previously were applied to each of the populations (Neuron, Glia) under first-level annotation. The elbow method was used to identify significant principal components after PCA. By plotting the variance ratio of each principal component (*pl.pca_variance_ratio* function), the top 10 and 15 principal components with the highest values were chosen to conduct the following analysis for neuron and glial cells, respectively. For neurons, they were further divided into excitatory and inhibitory neurons based on their expression profiles of *Slc17a7, Gad1,* and *Gad2.* For glial cells, multiple subtypes were identified for some of the major glial cells (*i.e.,* Astro 1, Astro2, and Astro3 for astrocytes). In this case, two levels of annotation were given to each cluster; a second level annotation indicated its major glial cell types (*i.e.,* astrocyte, oligodendrocyte, vascular cells, etc.), and a third level annotation (a unique identifier) represented the subtype within a major population.

To identify neuronal cell subtypes, the same unsupervised clustering was applied to both excitatory and inhibitory neuron populations individually. Specifically, 15 and 10 principal components were used to construct the kNN graph for excitatory and inhibitory neurons, respectively. The majority of neuronal cell clusters were annotated based on their spatial representation in the brain section (*i.e.*, anatomy regions such as L2/3, L4, CA, DG, TH, etc.), whereas some of the inhibitory neuron clusters were annotated based on their gene markers encoding different neuronal peptides (*i.e., Sst* and *Npy*).

*Region-enriched-translation genes identification:* RNA amplicons excluded by ClusterMap in single-cell quantification were treated as RNA outside the somata area of cells. The RNA counts for each gene in both somata and processes regions were calculated and normalized to percentages based on the overall RNA number of each gene. The top 10% genes with highest RNA percentage in each region were annotated as region-enriched-translation genes, and their RNA spatial distributions were visualized on the brain section.

*Gene ontology (GO) enrichment analysis*

DAVID Database (david.ncifcrf.gov) (45, 46) was used for GO enrichment analysis. The Benjamini and Hochberg false discovery rate (FDR) was performed for adjusting the p-values in multiple hypergeometric tests. The results from biological processes (BP) and cellular component (CC) (FDR < 0.05) were selected as the enriched GO terms. The top 3 most significantly enriched GO terms were used for covariation analysis and gene colocalization interrogation, while the top 10 enriched terms were extracted in the somata-enriched-translation genes and processes-enriched-translation genes.

**REFERENCES**

1. J. A. Alfaro, P. Bohländer, M. Dai, M. Filius, C. J. Howard, X. F. van Kooten, S. Ohayon, A. Pomorski, S. Schmid, A. Aksimentiev, E. V. Anslyn, G. Bedran, C. Cao, M. Chinappi, E. Coyaud, C. Dekker, G. Dittmar, N. Drachman, R.

34

Eelkema, D. Goodlett, S. Hentz, U. Kalathiya, N. L. Kelleher, R. T. Kelly, Z. Kelman, S. H. Kim, B. Kuster, D. Rodriguez-Larrea, S. Lindsay, G. Maglia, E. M. Marcotte, J. P. Marino, C. Masselon, M. Mayer, P. Samaras, K. Sarthak, L. Sepiashvili, D. Stein, M. Wanunu, M. Wilhelm, P. Yin, A. Meller, C. Joo, The emerging landscape of single-molecule protein sequencing technologies. Nat. Methods. 18, 604-617 (2021).

2. T. Maier, M. Güell, L. Serrano, Correlation of mRNA and protein in complex biological samples. FEBS Lett. 583, 3966-3973 (2009).

3. R. de Sousa Abreu, L. O. Penalva, E. M. Marcotte, C. Vogel, Global signatures of protein and mRNA expression levels. Mol. Biosyst. 5, 1512-1526 (2009).

4. C. Vogel, E. M. Marcotte, Insights into the regulation of protein abundance from proteomic and transcriptomic analyses. Nat. Rev. Genet. 13, 227-232 (2012).

5. B. Schwanhäusser, D. Busse, N. Li, G. Dittmar, J. Schuchhardt, J. Wolf, W. Chen, M. Selbach, Global quantification of mammalian gene expression control. Nature. 473, 337-342 (2011).

6. S. Das, M. Vera, V. Gandin, R. H. Singer, E. Tutucci, Intracellular mRNA transport and localized translation. Nat. Rev. Mol. Cell Biol. 22, 483-504 (2021).

7. N. T. Ingolia, S. Ghaemmaghami, J. R. S. Newman, J. S. Weissman, Genome-wide analysis in vivo of translation with nucleotide resolution using ribosome profiling. Science. 324, 218-223 (2009).

8. C. H. Jan, C. C. Williams, J. S. Weissman, Principles of ER cotranslational translocation revealed by proximity-specific ribosome profiling. Science (2014), doi:10.1126/science.1257521.

9. C. C. Williams, C. H. Jan, J. S. Weissman, Targeting and plasticity of mitochondrial proteins revealed by proximity-specific ribosome profiling. Science. 346, 748-751 (2014).

10. M. VanInsberghe, J. van den Berg, A. Andersson-Rolf, H. Clevers, A. van Oudenaarden, Single-cell Ribo-seq reveals cell cycle-dependent translational pausing. Nature. 597, 561-565 (2021).

11. N. T. Ingolia, Ribosome Footprint Profiling of Translation throughout the Genome. Cell. 165, 22-33 (2016).

12. Y. Liu, A. Beyer, R. Aebersold, On the Dependency of Cellular Protein Levels on mRNA Abundance. Cell. 165, 535-550 (2016).

13. J. M. Halstead, T. Lionnet, J. H. Wilbertz, F. Wippich, A. Ephrussi, R. H. Singer, J. A. Chao, Translation. An RNA biosensor for imaging the first round of translation from single cells to living animals. Science. 347, 1367-1671 (2015).

14. B. Wu, C. Eliscovich, Y. J. Yoon, R. H. Singer, Translation dynamics of single mRNAs in live cells and neurons. Science. 352, 1430-1435 (2016).

15. T. Morisaki, K. Lyon, K. F. DeLuca, J. G. DeLuca, B. P. English, Z. Zhang, L. D. Lavis, J. B. Grimm, S. Viswanathan, L. L. Looger, T. Lionnet, T. J. Stasevich, Real-time quantification of single RNA translation dynamics in living cells. Science. 352, 1425-1429 (2016).

16. X. Yan, T. A. Hoek, R. D. Vale, M. E. Tanenbaum, Dynamics of Translation of Single mRNA Molecules In Vivo. Cell. 165, 976-989 (2016).

17. S. Boersma, D. Khuperkar, B. M. P. Verhagen, S. Sonneveld, J. B. Grimm, L. D. Lavis, M. E. Tanenbaum, Multi-Color Single-Molecule Imaging Uncovers Extensive Heterogeneity in mRNA Decoding. Cell. 178, 458-472.e19 (2019).

18. Z. B. Katz, B. P. English, T. Lionnet, Y. J. Yoon, N. Monnier, B. Ovryn, M. Bathe, R. H. Singer, Mapping translation "hot-spots" in live cells by tracking single molecules of mRNA and ribosomes. Elife. 5, e10415 (2016).

19. S. T. Dieck, L. Kochen, C. Hanus, M. Heumüller, I. Bartnik, B. Nassim-Assir, K. Merk, T. Mosler, S. Garg, S. Bunse, D. A. Tirrell, E. M. Schuman, Direct visualization of newly synthesized target proteins in situ. Nature Methods. 12 (2015), pp. 411-414.

20. X. Wang, W. E. Allen, M. A. Wright, E. L. Sylwestrak, N. Samusik, S. Vesuna, K. Evans, C. Liu, C. Ramakrishnan, J. Liu, G. P. Nolan, F.-A. Bava, K. Deisseroth, Three-dimensional intact-tissue sequencing of single-cell transcriptional states. Science. 361 (2018), doi:10.1126/science.aat5691.

21. I. Tirosh, B. Izar, S. M. Prakadan, M. H. Wadsworth 2nd, D. Treacy, J. J. Trombetta, A. Rotem, C. Rodman, C. Lian, G. Murphy, M. Fallahi-Sichani, K. Dutton-Regester, J.-R. Lin, O. Cohen, P. Shah, D. Lu, A. S. Genshaft, T. K. Hughes, C. G. K. Ziegler, S. W. Kazer, A. Gaillard, K. E. Kolb, A.-C. Villani, C. M. Johannessen, A. Y. Andreev, E. M. Van Allen, M. Bertagnolli, P. K. Sorger, R. J. Sullivan, K. T. Flaherty, D. T. Frederick, J. Jané-Valbuena, C. H. Yoon, O. Rozenblatt-Rosen, A. K. Shalek, A. Regev, L. A. Garraway, Dissecting the multicellular ecosystem of metastatic melanoma by single-cell RNA-seq. Science. 352, 189-196 (2016).

22. F. M. Fazal, S. Han, K. R. Parker, P. Kaewsapsak, J. Xu, A. N. Boettiger, H. Y. Chang, A. Y. Ting, Atlas of Subcellular RNA Localization Revealed by APEX-Seq. Cell. 178, 473-490.e26 (2019).

23. X. Wang, Z. Lu, A. Gomez, G. C. Hon, Y. Yue, D. Han, Y. Fu, M. Parisien, Q. Dai, G. Jia, B. Ren, T. Pan, C. He, N6-methyladenosine-dependent regulation of messenger RNA stability. Nature. 505, 117-120 (2014).

24. H. Shi, X. Wang, Z. Lu, B. S. Zhao, H. Ma, P. J. Hsu, C. Liu, C. He, YTHDF3 facilitates translation and decay of N6-methyladenosine-modified RNA. Cell Res. 27, 315-328 (2017).

25. A. Sakaue-Sawano, H. Kurokawa, T. Morimura, A. Hanyu, H. Hama, H. Osawa, S. Kashiwagi, K. Fukami, T.

Miyata, H. Miyoshi, T. Imamura, M. Ogawa, H. Masai, A. Miyawaki, Visualizing spatiotemporal dynamics of multicellular cell-cycle progression. Cell. 132, 487-498 (2008).

26. S.-B. Koh, P. Mascalchi, E. Rodriguez, Y. Lin, D. I. Jodrell, F. M. Richards, S. K. Lyons, Quantitative FastFUCCI assay defines cell cycle dynamics at single-cell level. Journal of Cell Science. 130(2), 512-520 (2016) doi:10.1242/jcs.195164.

27. D. Mahdessian, A. J. Cesnik, C. Gnann, F. Danielsson, L. Stenström, M. Arif, C. Zhang, T. Le, F. Johansson, R. Shutten, A. Bäckström, U. Axelsson, P. Thul, N. H. Cho, O. Carja, M. Uhlén, A. Mardinoglu, C. Stadler, C. Lindskog, B. Ayoglu, M. D. Leonetti, F. Pontén, D. P. Sullivan, E. Lundberg, Spatiotemporal dissection of the cell cycle with single-cell proteogenomics. Nature. 590, 649-654 (2021).

28. B. S. Zhao, I. A. Roundtree, C. He, Post-transcriptional gene regulation by mRNA modifications. Nat. Rev. Mol. Cell Biol. 18, 31-42 (2017).

29. A. Saunders, E. Z. Macosko, A. Wysoker, M. Goldman, F. M. Krienen, H. de Rivera, E. Bien, M. Baum, L. Bortolin, S. Wang, A. Goeva, J. Nemesh, N. Kamitaki, S. Brumbaugh, D. Kulp, S. A. McCarroll, Molecular Diversity and Specializations among the Cells of the Adult Mouse Brain. Cell. 174, 1015-1030.e16 (2018).

30. Z. Yao, C. T. J. van Velthoven, T. N. Nguyen, J. Goldy, A. E. Sedeno-Cortes, F. Baftizadeh, D. Bertagnolli, T. Casper, M. Chiang, K. Crichton, S.-L. Ding, O. Fong, E. Garren, A. Glandon, N. W. Gouwens, J. Gray, L. T. Graybuck, M. J. Hawrylycz, D. Hirschstein, M. Kroll, K. Lathia, C. Lee, B. Levi, D. McMillen, S. Mok, T. Pham, Q. Ren, C. Rimorin, N. Shapovalova, J. Sulc, S. M. Sunkin, M. Tieu, A. Torkelson, H. Tung, K. Ward, N. Dee, K. A. Smith, B. Tasic, H. Zeng, A taxonomy of transcriptomic cell types across the isocortex and hippocampal formation. Cell. 184(12), 3222-3241 (2021), doi:10.1016/j.cell.2021.04.021.

31. A. Zeisel, H. Hochgerner, P. Lönnerberg, A. Johnsson, F. Memic, J. van der Zwan, M. Häring, E. Braun, L. E. Borm, G. La Manno, S. Codeluppi, A. Furlan, K. Lee, N. Skene, K. D. Harris, J. Hjerling-Leffler, E. Arenas, P. Ernfors, U. Marklund, S. Linnarsson, Molecular Architecture of the Mouse Nervous System. Cell. 174, 999-1014.e22 (2018).

32. Tabula Muris Consortium, Overall coordination, Logistical coordination, Organ collection and processing, Library preparation and sequencing, Computational data analysis, Cell type annotation, Writing group, Supplemental text writing group, Principal investigators, Single-cell transcriptomics of 20 mouse organs creates a Tabula Muris. Nature. 562, 367-372 (2018).

33. Tabula Muris Consortium, A single-cell transcriptomic atlas characterizes ageing tissues in the mouse. Nature. 583, 590-595 (2020).

34. X. Han, R. Wang, Y. Zhou, L. Fei, H. Sun, S. Lai, A. Saadatpour, Z. Zhou, H. Chen, F. Ye, D. Huang, Y. Xu, W. Huang, M. Jiang, X. Jiang, J. Mao, Y. Chen, C. Lu, J. Xie, Q. Fang, Y. Wang, R. Yue, T. Li, H. Huang, S. H. Orkin, G.-C. Yuan, M. Chen, G. Guo, Mapping the Mouse Cell Atlas by Microwell-Seq. Cell. 173, 1307 (2018).

35. J. Cao, M. Spielmann, X. Qiu, X. Huang, D. M. Ibrahim, A. J. Hill, F. Zhang, S. Mundlos, L. Christiansen, F. J. Steemers, C. Trapnell, J. Shendure, The single-cell transcriptional landscape of mammalian organogenesis. Nature. 566, 496-502 (2019).

36. E. S. Lein, M. J. Hawrylycz, N. Ao, M. Ayres, A. Bensinger, A. Bernard, A. F. Boe, M. S. Boguski, K. S. Brockway, E. J. Byrnes, L. Chen, L. Chen, T.-M. Chen, M. C. Chin, J. Chong, B. E. Crook, A. Czaplinska, C. N. Dang, S. Datta, N. R. Dee, A. L. Desaki, T. Desta, E. Diep, T. A. Dolbeare, M. J. Donelan, H.-W. Dong, J. G. Dougherty, B. J. Duncan, A. J. Ebbert, G. Eichele, L. K. Estin, C. Faber, B. A. Facer, R. Fields, S. R. Fischer, T. P. Fliss, C. Frensley, S. N. Gates, K. J. Glattfelder, K. R. Halverson, M. R. Hart, J. G. Hohmann, M. P. Howell, D. P. Jeung, R. A. Johnson, P. T. Karr, R. Kawal, J. M. Kidney, R. H. Knapik, C. L. Kuan, J. H. Lake, A. R. Laramee, K. D. Larsen, C. Lau, T. A. Lemon, A. J. Liang, Y. Liu, L. T. Luong, J. Michaels, J. J. Morgan, R. J. Morgan, M. T. Mortrud, N. F. Mosqueda, L. L. Ng, R. Ng, G. J. Orta, C. C. Overly, T. H. Pak, S. E. Parry, S. D. Pathak, O. C. Pearson, R. B. Puchalski, Z. L. Riley, H. R. Rockett, S. A. Rowland, J. J. Royall, M. J. Ruiz, N. R. Sarno, K. Schaffnit, N. V. Shapovalova, T. Sivisay, C. R. Slaughterbeck, S. C. Smith, K. A. Smith, B. I. Smith, A. J. Sodt, N. N. Stewart, K.-R. Stumpf, S. M. Sunkin, M. Sutram, A. Tam, C. D. Teemer, C. Thaller, C. L. Thompson, L. R. Varnam, A. Visel, R. M. Whitlock, P. E. Wohnoutka, C. K. Wolkey, V. Y. Wong, M. Wood, M. B. Yaylaoglu, R. C. Young, B. L. Youngstrom, X. F. Yuan, B. Zhang, T. A. Zwingman, A. R. Jones, Genome-wide atlas of gene expression in the adult mouse brain. Nature. 445, 168-176 (2007).

37. M. S. Fernandopulle, J. Lippincott-Schwartz, M. E. Ward, RNA transport and local translation in neurodevelopmental and neurodegenerative disease. Nat. Neurosci. 24, 622-632 (2021).

38. A. Biever, C. Glock, G. Tushev, E. Ciirdaeva, T. Dalmay, J. D. Langer, E. M. Schuman, Monosomes actively translate synaptic mRNAs in neuronal processes. Science. 367 (2020), doi:10.1126/science.aay4991.

39. C. Glock, A. Biever, G. Tushev, B. Nassim-Assir, A. Kao, I. Bartnik, S. Tom Dieck, E. M. Schuman, The translatome of neuronal cell bodies, dendrites, and axons. Proc. Natl. Acad. Sci. U. S. A. 118 (2021), doi:10.1073/pnas.2113929118.

40. L. M. Meservey, V. V. Topkar, M.-M. Fu, mRNA Transport and Local Translation in Glia. Trends Cell Biol. 31, 419-423 (2021).

41. K. Sakers, A. M. Lake, R. Khazanchi, R. Ouwenga, M. J. Vasek, A. Dani, J. D. Dougherty, Astrocytes locally translate transcripts in their peripheral processes. Proc. Natl. Acad. Sci. U. S. A. 114, E3830-E3838 (2017).

42. C. Müller, N. M. Bauer, I. Schäfer, R. White, Making myelin basic protein - from mRNA transport to localized translation. Frontiers in Cellular Neuroscience. 7 (2013) doi:10.3389/fncel.2013.00169.

43. Y. He, X. Tang, J. Huang, J. Ren, H. Zhou, K. Chen, A. Liu, H. Shi, Z. Lin, Q. Li, A. Aditham, J. Ounadjela, E. I. Grody, J. Shu, J. Liu, X. Wang, ClusterMap for multi-scale clustering analysis of spatial gene expression. Nat. Commun. 12, 5909 (2021).

44. K. S. Burke, K. A. Antilla, D. A. Tirrell, A Fluorescence in Situ Hybridization Method To Quantify mRNA Translation by Visualizing Ribosome-mRNA Interactions in Single Cells. ACS Cent. Sci. 3, 425-433 (2017).

45. D. W. Huang, B. T. Sherman, R. A. Lempicki, Systematic and integrative analysis of large gene lists using DAVID bioinformatics resources. Nat. Protoc. 4, 44-57 (2009).

46. D. W. Huang, B. T. Sherman, R. A. Lempicki, Bioinformatics enrichment tools: paths toward the comprehensive functional analysis of large gene lists. Nucleic Acids Res. 37, 1-13 (2008).

## INCORPORATION BY REFERENCE

The present application refers to various issued patent, published patent applications, scientific journal articles, and other publications, all of which are incorporated herein by reference. The details of one or more embodiments of the invention are set forth herein. Other features, objects, and advantages of the invention will be apparent from the Detailed Description, the Figures, the Examples, and the Claims.

## EQUIVALENTS AND SCOPE

In the articles such as "a," "an," and "the" may mean one or more than one unless indicated to the contrary or otherwise evident from the context. Embodiments or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the disclosure encompasses all variations, combinations, and permutations in which one or more limitations, elements, clauses, and descriptive terms from one or more of the listed claims is introduced into another claim. For example, any claim that is dependent on another claim can be modified to include one or more limitations found in any other claims that is dependent on the same base claim. Where elements are presented as lists, *e.g.,* in Markush group format, each subgroup of the elements is also disclosed, and any element(s) can be removed from the group. It should it be understood that, in general, where the invention, or aspects of the invention, is/are referred to as comprising particular elements and/or features, certain embodiments of the disclosure or aspects of the disclosure consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise.

This application refers to various issued patents, published patent applications, journal articles, and other publications, all of which are incorporated herein by reference. If there is a conflict between any of the incorporated references and the instant specification, the specification shall control. In addition, any particular embodiment of the present invention that falls within the prior art may be explicitly excluded from any one or more of the embodiments. Because such embodiments are deemed to be known to one of ordinary skill in the art, they may be excluded even if the exclusion is not set forth explicitly herein. Any particular embodiment of the invention can be excluded from any embodiment, for any reason, whether or not related to the existence of prior art.

Those skilled in the art will recognize or be able to ascertain using no more than routine experimentation many equivalents to the specific embodiments described herein. The scope of the present embodiments described herein is not intended to be limited to the above Description, but rather is as set forth in the appended embodiments. Those of ordinary skill in the art will appreciate that various changes and modifications to this description may be made without departing from the spirit or scope of the present invention, as defined in the following claims.

## CLAUSES

1. A method for profiling RNAs being translated in a cell, the method comprising:

> a) contacting the cell with one or more sets of probes, wherein each set of probes comprises a first probe, a second probe, and a third probe, wherein
>
>> i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;
>> ii) the second probe comprises a portion that recognizes the ribosome and an oligonucleotide portion that is complementary to a portion of the first probe; and
>> iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe;
>
> b) ligating the 5' end and the 3' end of the first probe together to produce a circular oligonucleotide;
> c) performing rolling circle amplification to amplify the circular oligonucleotide using the third probe as a primer to produce one or more concatenated amplicons;
> d) embedding the one or more concatenated amplicons in a polymeric matrix; and
> e) sequencing the concatenated amplicons embedded in the polymeric matrix to determine the identity and location of each RNA of interest in the cell.

2. The method of clause 1, wherein the portion of the second probe that recognizes the ribosome comprises an agent that binds an antibody, or an antibody variant or fragment.

3. The method of clause 1 or 2, wherein the portion of the second probe that recognizes the ribosome comprises an antibody, or an antibody variant or fragment.

4. The method of clause 3, wherein the antibody is a secondary antibody.

5. The method of clause 4 further comprising contacting the cell with a primary antibody that recognizes the ribosome and can be bound by the secondary antibody of the second probe.

6. The method of clause 5, wherein the primary antibody is an anti-40S ribosomal protein S3 (RPS3) antibody or an anti-60S ribosomal protein L4 (RPL4) antibody.

7. The method of clause 1, wherein the portion of the second probe that recognizes the ribosome comprises an oligonucleotide that is complementary to a portion of rRNA within the ribosome.

8. The method of clause 7, wherein the portion of the second probe that recognizes the ribosome comprises an oligonucleotide that is complementary to a portion of the 18S rRNA.

9. The method of clause 7 or 8, wherein the oligonucleotide that is complementary to a portion of the rRNA is 5, 6, 7, 8, 9, 10, 11, 12, 13, 14, 15, 16, 17, 18, 19, 20, 21, 22, 23, 24, 25, 26, 27, 28, 29, 30, or more than 30 nucleotides in length.

10. The method of any one of clauses 7-9, wherein the oligonucleotide that is complementary to a portion of the rRNA is about 25 nucleotides in length.

11. The method of any one of clauses 1-10, wherein the second probe further comprises a polymerization blocker.

12. The method of clause 11, wherein the polymerization blocker is located at the 3' end of second probe.

13. The method of clause 11 or 12, wherein the polymerization blocker comprises an inverted nucleic acid residue.

14. The method of clause 13, wherein the inverted nucleic acid residue is an inverted thymine residue.

15. The method of any one of clauses 1-14, wherein the oligonucleotide barcode sequences are 5-15, 6-14, 7-13, 8-12, or 9-11 nucleotides in length.

16. The method of any one of clauses 1-15, wherein the oligonucleotide barcode sequences are 5, 6, 7, 8, 9, 10, 11, 12, 13, 14, or 15 nucleotides in length.

17. The method of any one of clauses 1-16, wherein the first and third oligonucleotide probes are complementary to different portions of the RNA of interest.

18. The method of any one of clauses 1-17, wherein the first probe comprises the structure:
5'-[portion complementary to second probe]-[first barcode sequence]-[portion complementary to portion of third probe]-[portion complementary to RNA of interest]-[second barcode sequence]-3'.

19. The method any one of clauses 1-18, wherein the portion of the first probe that is complementary to a portion of the second probe is 4-20, 5-19, 6-18, 7-17, 8-16, 9-15, 10-14, or 11-13 nucleotides in length.

20. The method of any one of clauses 1-19, wherein the portion of the first probe that is complementary to a portion of the second probe is 4, 5, 6, 7, 8, 9, 10, 11, 12, 13, 14, 15, 16, 17, 18, 19, or 20 nucleotides in length.

21. The method of any one of clauses 1-20, wherein the portion of the first probe that is complementary to a portion of the second probe is split between the 5' end and the 3' end of the first probe.

22. The method any one of clauses 1-21, wherein the portion of the first probe that is complementary to a portion of the third probe is 5-15, 6-14, 7-13, 8-12, or 9-11 nucleotides in length.

23. The method of any one of clauses 1-22, wherein the portion of the first probe that is complementary to a portion of the third probe is 5, 6, 7, 8, 9, 10, 11, 12, 13, 14, or 15 nucleotides in length.

24. The method any one of clauses 1-23, wherein the portion of the first probe that is complementary to the RNA of interest is 10-30, 11-29, 12-28, 13-27, 14-26, 15-25, 16-24, 17-23, 18-22, or 19-21 nucleotides in length.

25. The method any one of clauses 1-24, wherein the portion of the first probe that is complementary to the RNA of interest is 10, 11, 12, 13, 14, 15, 16, 17, 18, 19, 20, 21, 22, 23, 24, 25, 26, 27, 28, 29, or 30 nucleotides in length.

26. The method of any one of clauses 1-25, wherein the second probe comprises the structure:
5'-[portion recognizing ribosome]-[portion complementary to first probe]-3'.

27. The method of any one of clauses 1-26, wherein the portion of the second probe that is complementary to a portion of the first probe is 4-20, 5-19, 6-18, 7-17, 8-16, 9-15, 10-14, or 11-13 nucleotides in length.

28. The method of any one of clauses 1-27, wherein the portion of the second probe that is complementary to a portion of the first probe is 4, 5, 6, 7, 8, 9, 10, 11, 12, 13, 14, 15, 16, 17, 18, 19, or 20 nucleotides in length.

29. The method of any one of clauses 1-28, wherein the portion of the second probe that recognizes the ribosome and the portion of the second probe that is complementary to a portion of the first probe are joined by a poly-A nucleotide linker.

30. The method of clause 29, wherein the poly-A nucleotide linker is 20-80, 30-70, or 40-60 nucleotides in length.

31. The method of clause 29 or 30, wherein the poly-A nucleotide linker is about 50 nucleotides in length.

32. The method of any one of clauses 1-31, wherein the third probe comprises the structure:
5'-[portion complementary to RNA of interest]-[portion complementary to first probe]-[barcode sequence]-3'.

33. The method of any one of clauses 1-32, wherein the portion of the third probe that is complementary to the RNA of interest is 10-30, 11-29, 12-28, 13-27, 14-26, 15-25, 16-24, 17-23, 18-22, or 19-21 nucleotides in length.

34. The method of any one of clauses 1-33, wherein the portion of the third probe that is complementary to the RNA of interest is 10, 11, 12, 13, 14, 15, 16, 17, 18, 19, 20, 21, 22, 23, 24, 25, 26, 27, 28, 29, or 30 nucleotides in length.

35. The method of any one of clauses 1-34, wherein the portion of the third probe that is complementary to a portion of the first probe is 5-15, 6-14, 7-13, 8-12, or 9-11 nucleotides in length.

36. The method of any one of clauses 1-35, wherein the portion of the third probe that is complementary to a portion of the first probe is 5, 6, 7, 8, 9, 10, 11, 12, 13, 14, or 15 nucleotides in length.

37. The method of any one of clauses 1-36, wherein RNAs being translated are profiled in multiple cells simultaneously.

38. The method of clause 37, wherein RNAs being translated are profiled in more than 100 cells, more than 200 cells, more than 300 cells, more than 400 cells, more than 500 cells, more than 1000 cells, more than 10,000 cells, more than 20,000 cells, more than 30,000 cells, more than 40,000 cells, or more than 50,000 cells simultaneously.

39. The method of clause 37 or 38, wherein the cells comprise a plurality of cell types.

40. The method of clause 39, wherein the cell types are selected from the group consisting of stem cells, progenitor cells, neuronal cells, astrocytes, dendritic cells, endothelial cells, microglia, oligodendrocytes, muscle cells, myocardial cells, mesenchymal cells, epithelial cells, immune cells, hepatic cells, smooth and skeletal muscle cells, hematopoietic cells, lymphocytes, monocytes, neutrophils, macrophages, natural killer cells, mast cells, adipocytes, and neurons.

41. The method of any one of clauses 1-40, wherein the cell is present within an intact tissue.

42. The method of clause 41, wherein the intact tissue is a fixed tissue sample.

43. The method of clause 41 or 42, wherein the tissue is brain tissue.

44. The method of any one of clauses 1-43, wherein translation of more than 1, more than 2, more than 3, more than 4, more than 5, more than 10, more than 20, more than 30, more than 40, more than 50, more than 100, more than 200, more than 500, more than 1000, more than 2000, 3000 RNAs, more than 4000 RNAs, or more than 5000 RNAs are profiled simultaneously.

45. The method of any one of clauses 1-44, wherein the second oligonucleotide barcode sequence of the first probe is a gene specific sequence used to identify an RNA of interest.

46. The method of any one of clauses 1-45, wherein the step of sequencing comprises performing sequencing with error-reduction by dynamic annealing and ligation (SEDAL).

47. The method of clause 46, wherein SEDAL is performed, two, three, four, five, or more than five times.

48. The method of any one of clauses 1-47, wherein the polymeric matrix is a hydrogel.

49. The method of clause 48, wherein the hydrogel is a polyvinyl alcohol hydrogel, a polyethylene glycol hydrogel, a sodium polyacrylate hydrogel, an acrylate hydrogel, or a polyacrylamide hydrogel.

50. The method of any one of clauses 1-49, wherein the step of performing rolling circle amplification further comprises providing amine-modified nucleotides, wherein the amine-modified nucleotides are incorporated into the one or more concatenated amplicons.

51. The method of clause 50, wherein the step of embedding the one or more concatenated amplicons in the polymeric matrix comprises reacting the amine-modified nucleotides of the one or more amplicons with acrylic acid N-hydroxysuccinimide ester and co-polymerizing the one or more concatenated amplicons and the polymeric matrix.

52. The method of any one of clauses 1-51 further comprising profiling additional molecules within the cell.

53. The method of clause 52, wherein the additional molecules are non-translating RNAs, RNAs in other subcellular locations, proteins, lipids, or small molecules.

54. The method of any one of clauses 1-53 further comprising determining the cell type of the profiled cell, or the cell types of multiple profiled cells, by comparing the RNA translation profile of the cell or cells to reference data comprising RNA translation profiles of cells of various cell types.

55. The method of any one of clauses 1-54 further comprising determining the cell state of the profiled cell, or the cell states of multiple profiled cells, by comparing the RNA translation profile of the cell or cells to reference data comprising RNA translation profiles of cells of various cell states.

56. The method of any one of clauses 1-55 further comprising overexpressing or knocking out one or more genes in the cell to determine whether the one or more genes are involved in regulating the translation of the RNA of interest.

57. The method of any one of clauses 1-56, wherein the method does not disrupt spatial information of subcellular structure, cell morphology, and/or tissue organization.

58. A method for profiling RNAs being translated in a cell, the method comprising:

a) contacting the cell with one or more pairs of probes, wherein each pair of probes comprises a first probe and a second probe, wherein

i) the first probe comprises an oligonucleotide portion that is complementary to the second probe, an oligonucleotide portion that is complementary to an RNA of interest, and an oligonucleotide barcode sequence; and
ii) the second probe comprises a portion that recognizes the ribosome and an oligonucleotide portion that is complementary to a portion of the first probe;

b) ligating the 5' end and the 3' end of the first probe together to produce a circular oligonucleotide;
c) performing rolling circle amplification to amplify the circular oligonucleotide using the second probe as a primer to produce one or more concatenated amplicons;
d) embedding the one or more concatenated amplicons in a polymeric matrix; and
e) sequencing the concatenated amplicons embedded in the polymeric matrix to determine the identity and location of each RNA of interest in the cell.

59. The method of clause 58, wherein the portion of the second probe that recognizes the ribosome comprises an agent that binds an antibody, or an antibody variant or fragment.

60. The method of clause 58 or 59, wherein the portion of the second probe that recognizes the ribosome comprises an antibody, or an antibody variant or fragment.

61. The method of clause 60, wherein the antibody is a secondary antibody.

62. The method of clause 61 further comprising contacting the cell with a primary antibody that recognizes a ribosome and is recognized by the secondary antibody of the second probe.

63. The method of clause 62, wherein the primary antibody is an anti-40S ribosomal protein S3 (RPS3) antibody or an anti-60S ribosomal protein L4 (RPL4) antibody.

64. The method of clause 58, wherein the portion of the second probe that recognizes the ribosome comprises an oligonucleotide that is complementary to a portion of ribosomal RNA (rRNA) within the ribosome.

65. The method of clause 64, wherein the portion of the second probe that recognizes the ribosome comprises an oligonucleotide that is complementary to a portion of the 18S rRNA.

66. The method of clause 64 or 65, wherein the oligonucleotide that is complementary to a portion of the rRNA is 5, 6, 7, 8, 9, 10, 11, 12, 13, 14, 15, 16, 17, 18, 19, 20, 21, 22, 23, 24, 25, 26, 27, 28, 29, 30, or more than 30 nucleotides in length.

67. The method of any one of clauses 64-66, wherein the oligonucleotide that is complementary to a portion of the rRNA is about 25 nucleotides in length.

68. The method of any one of clauses 58-67, wherein the oligonucleotide barcode sequence of the first probe is 5-15, 6-14, 7-13, 8-12, or 9-11 nucleotides in length.

69. The method of any one of clauses 58-68, wherein the oligonucleotide barcode sequence of the first probe is 5, 6, 7, 8, 9, 10, 11, 12, 13, 14, or 15 nucleotides in length.

70. The method of any one of clauses 58-69, wherein the first probe comprises the structure:
5'-[portion complementary to second probe]-[portion complementary to RNA of interest]-[barcode sequence]-3'.

71. The method of any one of clauses 58-70, wherein the second probe comprises the structure:
5'-[portion recognizing ribosome]-[portion complementary to first probe]-3'.

72. The method of any one of clauses 58-71, wherein the oligonucleotide barcode sequence of the first probe is a gene specific sequence used to identify an RNA of interest.

73. A method for diagnosing a disease or disorder in a subject, the method comprising:

a) contacting a cell taken from the subject with one or more pairs of probes, wherein each pair of probes comprises a first probe and a second probe, wherein

i) the first probe comprises an oligonucleotide portion that is complementary to the second probe, an oligonucleotide portion that is complementary to an RNA of interest, and an oligonucleotide barcode sequence; and
ii) the second probe comprises a portion that recognizes the ribosome and an oligonucleotide portion that is complementary to a portion of the first probe;

b) ligating the 5' end and the 3' end of the first probe together to produce a circular oligonucleotide;
c) performing rolling circle amplification to amplify the circular oligonucleotide using the second probe as a primer to produce one or more concatenated amplicons;
d) embedding the one or more concatenated amplicons in a polymeric matrix; and
e) sequencing the concatenated amplicons embedded in the polymeric matrix to determine the identity and location of each RNA of interest to profile RNAs being translated in the cell,

wherein a difference in the RNA translation profile of the cell relative to one or more non-diseased cells indicates that the subject has the disease or disorder.

74. A method for diagnosing a disease or disorder in a subject, the method comprising:

a) contacting a cell taken from the subject with one or more sets of probes, wherein each set of probes comprises a first probe, a second probe, and a third probe, wherein

i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;
ii) the second probe comprises a portion that recognizes the ribosome and an oligonucleotide portion that is complementary to a portion of the first probe; and
iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe;

b) ligating the 5' end and the 3' end of the first probe together to produce a circular oligonucleotide;
c) performing rolling circle amplification to amplify the circular oligonucleotide using the third probe as a primer to produce one or more concatenated amplicons;
d) embedding the one or more concatenated amplicons in a polymeric matrix; and
e) sequencing the concatenated amplicons embedded in the polymeric matrix to determine the identity and location of each RNA of interest in the cell,

wherein a difference in the RNA translation profile of the cell relative to one or more non-diseased cells indicates that the subject has the disease or disorder.

75. The method of clause 73 or 74, wherein RNAs being translated in one or more non-diseased cells are profiled as a control experiment alongside the cell taken from the subject.

76. The method of clause 73 or 74, wherein the profile of RNAs being translated in one or more non-diseased cells comprises reference data.

77. The method of any one of clauses 73-76, wherein the disease or disorder is a genetic disease, a proliferative disease, an inflammatory disease, an autoimmune disease, a liver disease, a spleen disease, a lung disease, a hematological disease, a neurological disease, a psychiatric disease, a gastrointestinal (GI) tract disease, a genitourinary disease, an infectious disease, a musculoskeletal disease, an endocrine disease, a metabolic disorder, an immune disorder, a central nervous system (CNS) disorder, or a cardiovascular disease.

78. The method of any one of clauses 73-77, wherein the cell is present in a tissue.

79. The method of clause 78, wherein the tissue is epithelial tissue, connective tissue, muscular tissue, or nervous tissue.

80. The method of clause 78 or 79, wherein the tissue is brain tissue.

81. The method of any one of clauses 78-80, wherein the tissue is a tissue sample taken from a subject.

82. The method of clause 81, wherein the subject is a non-human experimental animal.

83. The method of clause 82, wherein the non-human experimental animal is a mouse, a rat, a dog, a pig, or a non-human primate.

84. The method of clause 81, wherein the subject is a human.

85. A method for screening for an agent capable of modulating translation of one or more RNAs, the method comprising:

a) contacting a cell that is being treated with or has been treated with a candidate agent with one or more pairs of probes, wherein each pair of probes comprises a first probe and a second probe, wherein

i) the first probe comprises an oligonucleotide portion that is complementary to the second probe, an oligonucleotide portion that is complementary to an RNA of interest, and an oligonucleotide barcode sequence; and
ii) the second probe comprises a portion that recognizes the ribosome and an oligonucleotide portion that is complementary to a portion of the first probe;

b) ligating the 5' end and the 3' end of the first probe together to produce a circular oligonucleotide;
c) performing rolling circle amplification to amplify the circular oligonucleotide using the second probe as a primer to produce one or more concatenated amplicons;
d) embedding the one or more concatenated amplicons in a polymeric matrix; and
e) sequencing the concatenated amplicons embedded in the polymeric matrix to determine the identity and location of each RNA of interest to profile RNAs being translated in the cell,

wherein a difference in the profile of RNAs being translated in the presence of the candidate agent relative to in the absence of the candidate agent indicates that the candidate agent modulates translation of one or more RNAs.

86. A method for screening for an agent capable of modulating translation of one or more RNAs, the method comprising:

a) contacting a cell that is being treated with or has been treated with a candidate agent with one or more sets of probes, wherein each set of probes comprises a first probe, a second probe, and a third probe, wherein

i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;

ii) the second probe comprises a portion that recognizes the ribosome and an oligonucleotide portion that is complementary to a portion of the first probe; and

iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe;

b) ligating the 5' end and the 3' end of the first probe together to produce a circular oligonucleotide;

c) performing rolling circle amplification to amplify the circular oligonucleotide using the third probe as a primer to produce one or more concatenated amplicons;

d) embedding the one or more concatenated amplicons in a polymeric matrix; and

e) sequencing the concatenated amplicons embedded in the polymeric matrix to determine the identity and location of each RNA of interest bound to a ribosome in the cell,

wherein a difference in the profile of RNAs being translated in the presence of the candidate agent relative to in the absence of the candidate agent indicates that the candidate agent modulates translation of one or more RNAs.

87. The method of clause 85 or 86, wherein the candidate agent is a small molecule, a protein, a peptide, a nucleic acid, a lipid, or a carbohydrate.

88. The method of any one of clauses 85-87, where the candidate agent is a known drug or an FDA-approved drug.

89. The method of clause 87 or 88, wherein the protein is an antibody, or an antibody variant or fragment.

90. The method of clause 87 or 88, wherein the nucleic acid is an mRNA, an antisense RNA, an miRNA, an siRNA, an RNA aptamer, a double stranded RNA (dsRNA), a short hairpin RNA (shRNA), or an antisense oligonucleotide (ASO).

91. The method of any one of clauses 85-90, wherein modulating RNA translation is associated with reducing, relieving, or eliminating the symptoms of a disease or disorder.

92. The method of clause 91, wherein the disease or disorder is a genetic disease, a proliferative disease, an inflammatory disease, an autoimmune disease, a liver disease, a spleen disease, a lung disease, a hematological disease, a neurological disease, a psychiatric disease, a gastrointestinal (GI) tract disease, a genitourinary disease, an infectious disease, a musculoskeletal disease, an endocrine disease, a metabolic disorder, an immune disorder, a central nervous system (CNS) disorder, or a cardiovascular disease.

93. A method for treating a disease or disorder in a subject, the method comprising:

a) contacting a cell taken from the subject with one or more pairs of probes, wherein each pair of probes comprises a first probe and a second probe, wherein

i) the first probe comprises an oligonucleotide portion that is complementary to the second probe, an oligonucleotide portion that is complementary to an RNA of interest, and an oligonucleotide barcode sequence; and

ii) the second probe comprises a portion that recognizes the ribosome and an oligonucleotide portion that is complementary to a portion of the first probe;

b) ligating the 5' end and the 3' end of the first probe together to produce a circular oligonucleotide;

c) performing rolling circle amplification to amplify the circular oligonucleotide using the second probe as a primer to produce one or more concatenated amplicons;

d) embedding the one or more concatenated amplicons in a polymeric matrix;

e) sequencing the concatenated amplicons embedded in the polymeric matrix to determine the identity and location of each RNA of interest bound to a ribosome to profile RNAs being translated in the cell; and

f) administering a treatment for the disease or disorder to the subject if a difference in the profile of RNAs being translated in the cell relative to one or more non-diseased cells is observed.

94. A method for treating a disease or disorder in a subject, the method comprising:

a) contacting a cell taken from the subject with one or more sets of probes, wherein each set of probes comprises a first probe, a second probe, and a third probe, wherein

i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;
ii) the second probe comprises a portion that recognizes the ribosome and an oligonucleotide portion that is complementary to a portion of the first probe; and
iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe;

b) ligating the 5' end and the 3' end of the first probe together to produce a circular oligonucleotide;
c) performing rolling circle amplification to amplify the circular oligonucleotide using the third probe as a primer to produce one or more concatenated amplicons;
d) embedding the one or more concatenated amplicons in a polymeric matrix;
e) sequencing the concatenated amplicons embedded in the polymeric matrix to determine the identity and location of each RNA of interest bound to a ribosome in the cell; and
f) administering a treatment for the disease or disorder to the subject if a difference in the profile of RNAs being translated in the cell relative to one or more non-diseased cells is observed.

95. The method of clause 93 or 94, wherein translation of one or more RNAs in one or more non-diseased cells is profiled simultaneously as a control experiment.

96. The method of clause 93 or 94, wherein the profile of translation of one or more RNAs in one or more non-diseased cells comprises reference data.

97. The method of any one of clauses 93-96, wherein the treatment comprises administering a therapeutic agent, surgery, or radiation therapy.

98. The method of any one of clauses 93-97, wherein the therapeutic agent is a small molecule, a protein, a peptide, a nucleic acid, a lipid, or a carbohydrate.

99. The method of any one of clauses 93-98, where the therapeutic agent is a known drug or an FDA-approved drug.

100. The method of clause 98 or 99, wherein the protein is an antibody, or an antibody variant or fragment.

101. The method of clause 98 or 99, wherein the nucleic acid is an mRNA, an antisense RNA, an miRNA, an siRNA, an RNA aptamer, a double stranded RNA (dsRNA), a short hairpin RNA (shRNA), or an antisense oligonucleotide (ASO).

102. The method of any one of clauses 93-101, wherein the disease or disorder is a genetic disease, a proliferative disease, an inflammatory disease, an autoimmune disease, a liver disease, a spleen disease, a lung disease, a hematological disease, a neurological disease, a psychiatric disease, a gastrointestinal (GI) tract disease, a genitourinary disease, an infectious disease, a musculoskeletal disease, an endocrine disease, a metabolic disorder, an immune disorder, a central nervous system (CNS) disorder, or a cardiovascular disease.

103. A pair of probes comprising a first probe and a second probe, wherein:

i) the first probe comprises an oligonucleotide portion that is complementary to the second probe, an oligonucleotide portion that is complementary to an RNA of interest, and an oligonucleotide barcode sequence; and
ii) the second probe comprises a portion that recognizes the ribosome and an oligonucleotide portion that is complementary to a portion of the first probe.

104. The pair of probes of clause 103, wherein the portion of the second probe that recognizes the ribosome comprises an agent that binds an antibody, or an antibody variant or fragment.

105. The pair of probes of clause 103 or 104, wherein the portion of the second probe that recognizes the ribosome comprises an antibody, or an antibody variant or fragment.

106. The pair of probes of clause 105, wherein the antibody is a secondary antibody.

107. The pair of probes of clause 106, wherein the secondary antibody binds to a primary antibody that recognizes and binds to the ribosome.

108. The pair of probes of clause 107, wherein the primary antibody is an anti-40S ribosomal protein S3 (RPS3) antibody or an anti-60S ribosomal protein L4 (RPL4) antibody.

109. The pair of probes of clause 103, wherein the portion of the second probe that recognizes the ribosome comprises an oligonucleotide that is complementary to a portion of rRNA within the ribosome.

110. The pair of probes of clause 109, wherein the portion of the second probe that recognizes the ribosome comprises an oligonucleotide that is complementary to a portion of the 18S rRNA.

111. The pair of probes of clause 109 or 110, wherein the oligonucleotide that is complementary to a portion of the rRNA is 5, 6, 7, 8, 9, 10, 11, 12, 13, 14, 15, 16, 17, 18, 19, 20, 21, 22, 23, 24, 25, 26, 27, 28, 29, 30, or more than 30 nucleotides in length.

112. The pair of probes of any one of clauses 109-111, wherein the oligonucleotide that is complementary to a portion of the rRNA is about 25 nucleotides in length.

113. The pair of probes of any one of clauses 103-112, wherein the oligonucleotide barcode sequence of the first probe is 5-15, 6-14, 7-13, 8-12, or 9-11 nucleotides in length.

114. The pair of probes of any one of clauses 103-113, wherein the oligonucleotide barcode sequence of the first probe is 5, 6, 7, 8, 9, 10, 11, 12, 13, 14, or 15 nucleotides in length.

115. The pair of probes of any one of clauses 103-114, wherein the first probe comprises the structure:
5'-[portion complementary to second probe]-[portion complementary to RNA of interest]-[barcode sequence]-3'.

116. The pair of probes of any one of clauses 103-115, wherein the second probe comprises the structure:
5'-[portion recognizing ribosome]-[portion complementary to first probe]-3'.

117. A set of probes comprising a first probe, a second probe, and a third probe, wherein:

i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;
ii) the second probe comprises a portion that recognizes the ribosome and an oligonucleotide portion that is complementary to a portion of the first probe; and
iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe.

118. The set of probes of clause 117, wherein the portion of the second probe that recognizes the ribosome comprises an agent that binds an antibody, or an antibody variant or fragment.

119. The set of probes of clause 117 or 118, wherein the portion of the second probe that recognizes the ribosome comprises an antibody, or an antibody variant or fragment.

120. The set of probes of clause 119, wherein the antibody is a secondary antibody.

121. The set of probes of clause 120, wherein the secondary antibody binds to a primary antibody that recognizes and binds to the ribosome.

122. The set of probes of clause 121, wherein the primary antibody is an anti-40S ribosomal protein S3 (RPS3) antibody or an anti-60S ribosomal protein L4 (RPL4) antibody.

123. The set of probes of clause 117, wherein the portion of the second probe that recognizes the ribosome comprises an oligonucleotide that is complementary to a portion of the rRNA within the ribosome.

124. The set of probes of clause 123, wherein the portion of the second probe that recognizes the ribosome comprises an oligonucleotide that is complementary to a portion of the 18S rRNA.

125. The set of probes of clause 123 or 124, wherein the oligonucleotide that is complementary to a portion of the rRNA is 5, 6, 7, 8, 9, 10, 11, 12, 13, 14, 15, 16, 17, 18, 19, 20, 21, 22, 23, 24, 25, 26, 27, 28, 29, 30, or more than 30 nucleotides in length.

126. The set of probes of any one of clauses 123-125, wherein the oligonucleotide that is complementary to a portion of the rRNA is about 25 nucleotides in length.

127. The set of probes of any one of clauses 117-126, wherein the second probe further comprises a polymerization blocker.

128. The set of probes of clause 127, wherein the polymerization blocker is located at the 3' end of the second probe.

129. The set of probes of clauses 127 or 128, wherein the polymerization blocker comprises an inverted nucleic acid residue.

130. The set of probes of clause 129, wherein the inverted nucleic acid residue is an inverted thymine residue.

131. The set of probes of any one of clauses 117-130, wherein the oligonucleotide barcode sequences are 5-15, 6-14, 7-13, 8-12, or 9-11 nucleotides in length.

132. The set of probes of any one of clauses 117-131, wherein the barcode sequences are 5, 6, 7, 8, 9, 10, 11, 12, 13, 14, or 15 nucleotides in length.

133. The set of probes of any one of clause 117-132, wherein the first and third oligonucleotide probes are complementary to different portions of the RNA of interest.

134. The set of probes of any one of clause 117-133, wherein the first probe comprises the structure:
5'-[portion complementary to second probe]-[first barcode sequence]-[portion complementary to portion of third probe]-[portion complementary to RNA of interest]-[second barcode sequence]-3'.

135. The set of probes any one of clause 117-134, wherein the portion of the first probe that is complementary to a portion of the second probe is 4-20, 5-19, 6-18, 7-17, 8-16, 9-15, 10-14, or 11-13 nucleotides in length.

136. The set of probes of any one of clause 117-135, wherein the portion of the first probe that is complementary to a portion of the second probe is 4, 5, 6, 7, 8, 9, 10, 11, 12, 13, 14, 15, 16, 17, 18, 19, or 20 nucleotides in length.

137. The set of probes of any one of clause 117-136, wherein the portion of the first probe that is complementary to a portion of the second probe is split between the 5' end and the 3' end of the first probe.

138. The set of probes any one of clause 117-137, wherein the portion of the first probe that is complementary to a portion of the third probe is 5-15, 6-14, 7-13, 8-12, or 9-11 nucleotides in length.

139. The set of probes of any one of clause 117-138, wherein the portion of the first probe that is complementary to a portion of the third probe is 5, 6, 7, 8, 9, 10, 11, 12, 13, 14, or 15 nucleotides in length.

140. The set of probes any one of clause 117-139, wherein the portion of the first probe that is complementary to the RNA of interest is 10-30, 11-29, 12-28, 13-27, 14-26, 15-25, 16-24, 17-23, 18-22, or 19-21 nucleotides in length.

141. The set of probes any one of clause 117-140, wherein the portion of the first probe that is complementary to the RNA of interest is 10, 11, 12, 13, 14, 15, 16, 17, 18, 19, 20, 21, 22, 23, 24, 25, 26, 27, 28, 29, or 30 nucleotides in length.

142. The set of probes of any one of clauses 117-141, wherein the second probe comprises the structure:
5'-[portion recognizing ribosome]-[portion complementary to first probe]-3'.

143. The set of probes of any one of clauses 117-142, wherein the portion of the second probe that is complementary to a portion of the first probe is 4-20, 5-19, 6-18, 7-17, 8-16, 9-15, 10-14, or 11-13 nucleotides in length.

144. The set of probes of any one of clauses 117-143, wherein the portion of the second probe that is complementary to a portion of the first probe is 4, 5, 6, 7, 8, 9, 10, 11, 12, 13, 14, 15, 16, 17, 18, 19, or 20 nucleotides in length.

145. The set of probes of any one of clauses 117-144, wherein the portion of the second probe that recognizes the ribosome and the portion of the second probe that is complementary to a portion of the first probe are joined by a poly-A nucleotide linker.

146. The set of probes of clause 145, wherein the poly-A nucleotide linker is 20-80, 30-70, or 40-60 nucleotides in length.

147. The set of probes of clause 145 or 146, wherein the poly-A nucleotide linker is about 50 nucleotides in length.

148. The set of probes of any one of clauses 117-147, wherein the third probe comprises the structure:
5'-[portion complementary to RNA of interest]-[portion complementary to first probe]-[barcode sequence]-3'.

149. The set of probes of any one of clauses 117-148, wherein the portion of the third probe that is complementary to the RNA of interest is 10-30, 11-29, 12-28, 13-27, 14-26, 15-25, 16-24, 17-23, 18-22, or 19-21 nucleotides in length.

150. The set of probes of any one of clauses 117-149, wherein the portion of the third probe that is complementary to the RNA of interest is 10, 11, 12, 13, 14, 15, 16, 17, 18, 19, 20, 21, 22, 23, 24, 25, 26, 27, 28, 29, or 30 nucleotides in length.

151. The set of probes of any one of clauses 117-150, wherein the portion of the third probe that is complementary to a portion of the first probe is 5-15, 6-14, 7-13, 8-12, or 9-11 nucleotides in length.

152. The set of probes of any one of clauses 117-151, wherein the portion of the third probe that is complementary to a portion of the first probe is 5, 6, 7, 8, 9, 10, 11, 12, 13, 14, or 15 nucleotides in length.

153. A plurality of probes comprising multiple pairs of probes of any one of clauses 103-116 or multiple sets of probes of any one of clauses 117-152, and wherein each pair of probes or set of probes comprises oligonucleotide portions that are complementary to a different RNA of interest.

154. A kit comprising the pair of probes of any one of clauses 103-116 or the set of probes of any one of clauses 117-152.

155. The kit of clause 154, wherein the kit comprises multiple pairs of probes of any one of clause 103-116 or multiple sets of probes of any one of clauses 117-152, and wherein each pair of probes or set of probes comprises oligonucleotide portions that are complementary to a different RNA of interest.

156. The kit of clause 154 or 155 further comprising cells.

157. The kit of any one of clauses 154-156 further comprising one or more enzymes.

158. The kit of clause 157, wherein the one or more enzymes comprise a ligase.

159. The kit of clause 157 or 158, wherein the one or more enzymes comprise a polymerase.

160. The kit of any one of clauses 154-159 further comprising amine-modified nucleotides.

161. The kit of any one of clauses 154-160 further comprising reagents and monomers for preparing a polymeric matrix.

162. A system for profiling RNAs being translated in a cell comprising

a) a cell;
b) one or more pairs of probes comprising a first probe and a second probe, wherein

i) the first probe comprises an oligonucleotide portion that is complementary to the second probe, an oligonucleotide portion that is complementary to an RNA of interest, and an oligonucleotide barcode sequence; and
ii) the second probe comprises a portion that recognizes the ribosome and an oligonucleotide portion that is complementary to a portion of the first probe;

c) a microscope; and
d) a computer.

163. The system of clause 162, wherein the portion of the second probe that recognizes the ribosome comprises an agent that binds an antibody, or an antibody variant or fragment.

164. The system of clause 162 or 163, wherein the portion of the second probe that recognizes the ribosome comprises an antibody, or an antibody variant or fragment.

165. The system of clause 164, wherein the antibody is a secondary antibody.

166. The system of clause 165, wherein the secondary antibody binds to a primary antibody that recognizes and binds to the ribosome.

167. The system of clause 166, wherein the primary antibody is an anti-40S ribosomal protein S3 (RPS3) antibody or an anti-60S ribosomal protein L4 (RPL4) antibody.

168. The system of clause 162, wherein the portion of the second probe that recognizes the ribosome comprises an oligonucleotide that is complementary to a portion of the rRNA within the ribosome.

169. The system of clause 168, wherein the portion of the second probe that recognizes the ribosome comprises an oligonucleotide that is complementary to a portion of the 18S rRNA.

170. The system of clause 168 or 169, wherein the oligonucleotide that is complementary to a portion of the rRNA is 5, 6, 7, 8, 9, 10, 11, 12, 13, 14, 15, 16, 17, 18, 19, 20, 21, 22, 23, 24, 25, 26, 27, 28, 29, 30, or more than 30 nucleotides in length.

171. The system of any one of clauses 168-170, wherein the oligonucleotide that is complementary to a portion of the rRNA is about 25 nucleotides in length.

172. The system of any one of clauses 162-171, wherein the oligonucleotide barcode sequence of the first probe is 5-15, 6-14, 7-13, 8-12, or 9-11 nucleotides in length.

173. The system of any one of clauses 162-172, wherein the oligonucleotide barcode sequence of the first probe is 5, 6, 7, 8, 9, 10, 11, 12, 13, 14, or 15 nucleotides in length.

174. The system of any one of clauses 162-173, wherein the first probe comprises the structure:
5'-[portion complementary to second probe]-[portion complementary to RNA of interest]-[barcode sequence]-3'.

175. The system of any one of clauses 162-174, wherein the second probe comprises the structure:
5'-[portion recognizing ribosome]-[portion complementary to first probe]-3'.

176. A system for profiling RNAs being translated in a cell comprising

a) a cell;
b) one or more sets of probes comprising a first probe, a second probe, and a third probe, wherein

i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;
ii) the second probe comprises a portion that recognizes the ribosome and an oligonucleotide portion that is complementary to a portion of the first probe; and
iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe;

c) a microscope; and
d) a computer.

177. The system of clause 176, wherein the portion of the second probe that recognizes the ribosome comprises an agent that binds an antibody, or an antibody variant or fragment.

178. The system of clause 176 or 177, wherein the portion of the second probe that recognizes the ribosome comprises an antibody, or an antibody variant or fragment.

179. The system of clause 178, wherein the antibody is a secondary antibody.

180. The system of clause 179, wherein the secondary antibody binds to a primary antibody that recognizes and binds to the ribosome.

181. The system of clause 180, wherein the primary antibody is an anti-40S ribosomal protein S3 (RPS3) antibody or an anti-60S ribosomal protein L4 (RPL4) antibody.

182. The system of clause 176, wherein the portion of the second probe that recognizes the ribosome comprises an oligonucleotide that is complementary to a portion of the rRNA within the ribosome.

183. The system of clause 182, wherein the portion of the second probe that recognizes the ribosome comprises an oligonucleotide that is complementary to a portion of the 18S rRNA.

184. The system of clause 182 or 183, wherein the oligonucleotide that is complementary to a portion of the rRNA is 5, 6, 7, 8, 9, 10, 11, 12, 13, 14, 15, 16, 17, 18, 19, 20, 21, 22, 23, 24, 25, 26, 27, 28, 29, 30, or more than 30 nucleotides in length.

185. The system of any one of clauses 176-184, wherein the oligonucleotide that is complementary to a portion of the rRNA is about 25 nucleotides in length.

186. The system of any one of clauses 176-185, wherein the second probe further comprises a polymerization blocker.

187. The system of clause 186, wherein the polymerization blocker is located at the 3' end of second probe.

188. The system of clause 186 or 187, wherein the polymerization blocker comprises an inverted nucleic acid residue.

189. The system of clause 188, wherein the inverted nucleic acid residue is an inverted thymine residue.

190. The system of any one of clauses 176-189, wherein the oligonucleotide barcode sequences are 5-15, 6-14, 7-13, 8-12, or 9-11 nucleotides in length.

191. The system of any one of clauses 176-190, wherein the barcode sequences are 5, 6, 7, 8, 9, 10, 11, 12, 13, 14, or 15 nucleotides in length.

192. The system of any one of clauses 176-191, wherein the first and third oligonucleotide probes are complementary to different portions of the RNA of interest.

193. The system of any one of clauses 176-192, wherein the first probe comprises the structure:
5'-[portion complementary to second probe]-[first barcode sequence]-[portion complementary to portion of third probe]-[portion complementary to RNA of interest]-[second barcode sequence]-3'.

194. The system any one of clauses 176-193, wherein the portion of the first probe that is complementary to a portion of the second probe is 4-20, 5-19, 6-18, 7-17, 8-16, 9-15, 10-14, or 11-13 nucleotides in length.

195. The system of any one of clauses 176-194, wherein the portion of the first probe that is complementary to a portion of the second probe is 4, 5, 6, 7, 8, 9, 10, 11, 12, 13, 14, 15, 16, 17, 18, 19, or 20 nucleotides in length.

196. The system of any one of clauses 176-195, wherein the portion of the first probe that is complementary to a portion of the second probe is split between the 5' end and the 3' end of the first probe.

197. The system any one of clauses 176-196, wherein the portion of the first probe that is complementary to a portion of the third probe is 5-15, 6-14, 7-13, 8-12, or 9-11 nucleotides in length.

198. The system of any one of clauses 176-197, wherein the portion of the first probe that is complementary to a portion of the third probe is 5, 6, 7, 8, 9, 10, 11, 12, 13, 14, or 15 nucleotides in length.

199. The system any one of clauses 176-198, wherein the portion of the first probe that is complementary to the RNA of interest is 10-30, 11-29, 12-28, 13-27, 14-26, 15-25, 16-24, 17-23, 18-22, or 19-21 nucleotides in length.

200. The system any one of clauses 176-199, wherein the portion of the first probe that is complementary to the RNA of interest is 10, 11, 12, 13, 14, 15, 16, 17, 18, 19, 20, 21, 22, 23, 24, 25, 26, 27, 28, 29, or 30 nucleotides in length.

201. The system of any one of clauses 176-200, wherein the second probe comprises the structure:
5'-[portion recognizing ribosome]-[portion complementary to first probe]-3'.

202. The system of any one of clauses 176-201, wherein the portion of the second probe that is complementary to a portion of the first probe is 4-20, 5-19, 6-18, 7-17, 8-16, 9-15, 10-14, or 11-13 nucleotides in length.

203. The system of any one of clauses 176-202, wherein the portion of the second probe that is complementary to a portion of the first probe is 4, 5, 6, 7, 8, 9, 10, 11, 12, 13, 14, 15, 16, 17, 18, 19, or 20 nucleotides in length.

204. The system of any one of clauses 176-203, wherein the portion of the second probe that recognizes the ribosome and the portion of the second probe that is complementary to a portion of the first probe are joined by a poly-A nucleotide linker.

205. The system of clause 204, wherein the poly-A nucleotide linker is 20-80, 30-70, or 40-60 nucleotides in length.

206. The system of clause 204 or 205, wherein the poly-A nucleotide linker is about 50 nucleotides in length.

207. The system of any one of clauses 176-206, wherein the third probe comprises the structure:
5'-[portion complementary to RNA of interest]-[portion complementary to first probe]-[barcode sequence]-3'.

208. The system of any one of clauses 176-207, wherein the portion of the third probe that is complementary to the RNA of interest is 10-30, 11-29, 12-28, 13-27, 14-26, 15-25, 16-24, 17-23, 18-22, or 19-21 nucleotides in length.

209. The system of any one of clauses 176-208, wherein the portion of the third probe that is complementary to the RNA of interest is 10, 11, 12, 13, 14, 15, 16, 17, 18, 19, 20, 21, 22, 23, 24, 25, 26, 27, 28, 29, or 30 nucleotides in length.

210. The system of any one of clauses 176-209, wherein the portion of the third probe that is complementary to a portion of the first probe is 5-15, 6-14, 7-13, 8-12, or 9-11 nucleotides in length.

211. The system of any one of clauses 176-210, wherein the portion of the third probe that is complementary to a portion of the first probe is 5, 6, 7, 8, 9, 10, 11, 12, 13, 14, or 15 nucleotides in length.

## Claims

1. A system comprising:

   a cell;
   one or more sets of probes, wherein each set of probes comprises a first probe, a second probe, and a third probe, wherein:

   i) the first probe comprises an oligonucleotide portion that is complementary to the second probe, a first oligonucleotide barcode sequence, an oligonucleotide portion that is complementary to a portion of the third probe, an oligonucleotide portion that is complementary to an RNA of interest, and a second oligonucleotide barcode sequence;
   ii) the second probe comprises a portion that recognizes the ribosome and an oligonucleotide portion that is complementary to a portion of the first probe; and
   iii) the third probe comprises an oligonucleotide portion that is complementary to the RNA of interest, an oligonucleotide portion that is complementary to a portion of the first probe, and an oligonucleotide barcode sequence, wherein the oligonucleotide barcode sequence of the third probe is complementary to the first oligonucleotide barcode sequence of the first probe; and a computer;

   wherein the system is configured to perform the following method steps:

   a) contacting the cell with one or more pairs of oligonucleotide probes;
   b) ligating the 5' end and the 3' end of the first probe together to produce a circular oligonucleotide;
   c) performing rolling circle amplification to amplify the circular oligonucleotide using the third probe as a primer to produce one or more concatenated amplicons;
   d) embedding the one or more concatenated amplicons in a polymeric matrix; and
   e) sequencing the concatenated amplicons embedded in the polymeric matrix to determine the identity and location of each RNA of interest in the cell.

2. The system of claim 1, wherein the portion of the second probe that recognizes the ribosome comprises an antibody, or an antibody variant or fragment.

3. The system of claim 1 or 2, wherein the antibody is an anti-40S ribosomal protein S3 antibody or an anti-60S ribosomal protein L4 antibody.

4. The system of claim 1, wherein the portion of the second probe that recognizes the ribosome comprises an oligonucleotide that is complementary to a portion of the 18S ribosomal RNA.

5. The system of any one of claims 1-4, wherein the second probe further comprises a polymerization blocker.

6. The system of any one of claims 1-5, wherein the cell is present within an intact tissue.

7. The system of any one of claims 1-6, wherein the second oligonucleotide barcode sequence of the first probe is used to identify an RNA of interest.

8. The system of any one of claims 1-7, wherein step (c) comprises providing amine-modified nucleotides, wherein the amine-modified nucleotides are incorporated into the one or more concatenated amplicons.

9. The system of claim 8, wherein step (d) comprises reacting the amine-modified nucleotides.

10. The system of any one of claims 1-9, wherein the polymeric matrix is a hydrogel.

11. The system of any one of claims 1-10, wherein the hydrogel is a polyacrylamide hydrogel.

12. The system of any one of claims 1-11, wherein step (b) comprises use of a ligase, optionally wherein the ligase is a T4 ligase.

13. The system of any one of claims 1-12, wherein step (c) comprises use of a polymerase.

14. The system of claim 13, wherein the polymerase is a DNA polymerase.

15. The system of any one of claims 1-14, wherein step (e) comprises use of a microscope to detect a fluorescence signal associated with the RNA of interest.

FIG. 1

**FIG. 2A**

**FIG. 2B**

**FIG. 2C**

**FIG. 2D**

FIG. 3

**FIG. 4A**

**FIG. 4B**

**FIG. 4C**

**FIG. 5A**

**FIG. 5B**

**FIG. 5C**

SNAIL probe

Translating mRNA    Silencing mRNA

Hybridization

rRNA-hybridized probe

Ligation

DNA amplicon

Agtification

Intact padlock probe

Hydrogel-tissue chemistry
and SEDAL sequencing

Ribosome profiling signal readout

Decode

TTGCACT    Translating gene 1
ACGTTAC    Translating gene 2
....
....
GTACTTA    Translating gene n

Oganelles staining

Annotate

Endoplasmic reticulum region 1       Mitocondria region 1
Endoplasmic reticulum region 2       Mitocondria region 3
....
....
Endoplasmic reticulum region  m      Mitocondria region i

in situ
single-cell
translational
states with
subcellular
localization
information

**FIG. 6**

**FIG. 7A**

**FIG. 7B**

**FIG. 7C**

FIG. 8

FIG. 9A

**FIG. 9B**

**FIG. 9C**

FIG. 9D

FIG. 9E

**FIG. 9F**

**FIG. 9G**

**FIG. 9H**

**FIG. 9I**

**FIG. 9J**

RIBOmap of 981 genes

Ribosome-bound
RNA mapping
Organelle staining

1,813 HeLa/FUCCI cells

localized translation

STARmap of 981 genes

RNA mapping
Organelle staining

1,757 HeLa/FUCCI cells

RNA localization

**FIG. 10A**

FIG. 10B

**FIG. 10C**

**FIG. 10D**

**FIG. 10E**

**FIG. 10F**

**FIG. 10G**

**FIG. 10H**

**FIG. 10I**

Distance ratio (DR)
= d1/(d1+d2)

**FIG. 10J**

**FIG. 10K**

**FIG. 11A**

FIG. 11B

**FIG. 11C**

**FIG. 11D**

Isocortex
Striatum
Hippocampus
Thalamus

Olfactory
areas

Cortical
subplate

Hypothalamus

500 μm

200 μm

50 μm

FIG. 11E

EP 4 696 786 A2

**FIG. 11F**

Somata reads / Neuronal and glial processes reads

50 µm

XY veiw

**FIG. 11G**

**FIG. 11H**

**FIG. 11I**

FIG. 11J

Shank1 (41.1%)

Eef2 (33.3%)

Kif5a (33.6%)

Calm1 (30.5%)

**FIG. 11K**

App (13.9%)

Rtn4 (14.8%)

**FIG. 11L**

Gfap (35.8%, Astro)

200 μm

Mbp (39.2%, Oligo)

200 μm

**FIG. 11M**

Mal (8.2%, Oligo)

200 μm

**FIG. 11N**

**FIG. 12A**

**FIG. 12B**

**FIG. 12C**

**FIG. 12D**

**FIG. 12E**

**FIG. 12F**

FIG. 13A

**FIG. 13B**

**FIG. 14A**

**FIG. 14B**

**FIG. 14C**

**FIG. 14D**

FIG. 14E

FIG. 14F

**FIG. 15A**

**FIG. 15B**

**FIG. 15C**

**FIG. 15D**

**FIG. 15E**

**FIG. 15F**

FIG. 16A

Sst

RIBOmap       Allen Brain ISH       HPA Proteome

**FIG. 16B**

**Nefl**

RIBOmap

Allen Brain ISH

HPA Proteome

**FIG. 16C**

**FIG. 17A**

FIG. 17B

**FIG. 18A**

Neuronal cell    Glia

**FIG. 18B**

Excitatory neuron    Inhibitory neuron    Astrocyte

Oligodendrocyte    Oligodentrocyte precursor cell    Microglia

Vascular cell    Unknow

**FIG. 18C**

**FIG. 18D**

**FIG. 19A**

**FIG. 19B**

**FIG. 19C**

EP 4 696 786 A2

**FIG. 20A**

FIG. 20B

**FIG. 20C**

**FIG. 21A**

**FIG. 21B**

**FIG. 21C**

FIG. 22A

**FIG. 22B**

FIG. 22C

FIG. 22D

## REFERENCES CITED IN THE DESCRIPTION

**Patent documents cited in the description**

- US 63216315 A

- WO 2019199579 A

**Non-patent literature cited in the description**

- **SINGLETON et al.** Dictionary of Microbiology and Molecular Biology. 1994
- The Cambridge Dictionary of Science and Technology. 1988
- The Glossary of Genetics. Springer Verlag, 1991
- **HALE** ; **MARHAM**. *The Harper Collins Dictionary of Biology*, 1991
- Stedman's Medical Dictionary,. Williams & Wilkins, 1990
- **WANG et al.** *Science*, 2018, vol. 361, 380
- **WANG, X. et al.** Three-dimensional intact-tissue sequencing of single-cell transcriptional states. *Science*, 2018, vol. 361, 380
- **J. A. ALFARO** ; **P. BOHLÄNDER** ; **M. DAI** ; **M. FILIUS** ; **C. J. HOWARD** ; **X. F. VAN KOOTEN** ; **S. OHAYON** ; **A. POMORSKI** ; **S. SCHMID** ; **A. AKSIMENTIEV**. The emerging landscape of single-molecule protein sequencing technologies. *Nat. Methods*, 2021, vol. 18, 604-617
- **T. MAIER** ; **M. GÜELL** ; **L. SERRANO**. Correlation of mRNA and protein in complex biological samples. *FEBS Lett.*, 2009, vol. 583, 3966-3973
- **R. DE SOUSA ABREU** ; **L. O. PENALVA** ; **E. M. MARCOTTE** ; **C. VOGEL**. Global signatures of protein and mRNA expression levels. *Mol. Biosyst.*, 2009, vol. 5, 1512-1526
- **C. VOGEL** ; **E. M. MARCOTTE**. Insights into the regulation of protein abundance from proteomic and transcriptomic analyses. *Nat. Rev. Genet.*, 2012, vol. 13, 227-232
- **B. SCHWANHÄUSSER** ; **D. BUSSE** ; **N. LI** ; **G. DITTMAR** ; **J. SCHUCHHARDT** ; **J. WOLF** ; **W. CHEN** ; **M. SELBACH**. Global quantification of mammalian gene expression control. *Nature.*, 2011, vol. 473, 337-342
- **S. DAS** ; **M. VERA** ; **V. GANDIN** ; **R. H. SINGER** ; **E. TUTUCCI**. Intracellular mRNA transport and localized translation.. *Nat. Rev. Mol. Cell Biol.*, 2021, vol. 22, 483-504

- **N. T. INGOLIA** ; **S. GHAEMMAGHAMI** ; **J. R. S. NEWMAN** ; **J. S. WEISSMAN**. Genome-wide analysis in vivo of translation with nucleotide resolution using ribosome profiling. *Science*, 2009, vol. 324, 218-223
- **C. H. JAN** ; **C. C. WILLIAMS** ; **J. S. WEISSMAN**. Principles of ER cotranslational translocation revealed by proximity-specific ribosome profiling. *Science*, 2014
- **C. C. WILLIAMS** ; **C. H. JAN** ; **J. S. WEISSMAN**. Targeting and plasticity of mitochondrial proteins revealed by proximity-specific ribosome profiling. *Science*, 2014, vol. 346, 748-751
- **M. VANINSBERGHE** ; **J. VAN DEN BERG** ; **A. ANDERSSON-ROLF** ; **H. CLEVERS** ; **A. VAN OUDENAARDEN**. Single-cell Ribo-seq reveals cell cycle-dependent translational pausing.. *Nature.*, 2021, vol. 597, 561-565
- **N. T. INGOLIA**. Ribosome Footprint Profiling of Translation throughout the Genome. *Cell*, 2016, vol. 165, 22-33
- **Y. LIU** ; **A. BEYER** ; **R. AEBERSOLD**. On the Dependency of Cellular Protein Levels on mRNA Abundance. *Cell*, 2016, vol. 165, 535-550
- **J. M. HALSTEAD** ; **T. LIONNET** ; **J. H. WILBERTZ** ; **F. WIPPICH** ; **A. EPHRUSSI** ; **R. H. SINGER** ; **J. A. CHAO**. Translation. An RNA biosensor for imaging the first round of translation from single cells to living animals. *Science*, 2015, vol. 347, 1367-1671
- **B. WU** ; **C. ELISCOVICH** ; **Y. J. YOON** ; **R. H. SINGER**. Translation dynamics of single mRNAs in live cells and neurons. *Science*, 2016, vol. 352, 1430-1435
- **T. MORISAKI** ; **K. LYON** ; **K. F. DELUCA** ; **J. G. DELUCA** ; **B. P. ENGLISH** ; **Z. ZHANG** ; **L. D. LAVIS** ; **J. B. GRIMM** ; **S. VISWANATHAN** ; **L. L. LOOGER**. Real-time quantification of single RNA translation dynamics in living cells. *Science*, 2016, vol. 352, 1425-1429
- **X. YAN** ; **T. A. HOEK** ; **R. D. VALE** ; **M. E. TANENBAUM**. Dynamics of Translation of Single mRNA Molecules. *In Vivo. Cell*, 2016, vol. 165, 976-989

- **S. BOERSMA** ; **D. KHUPERKAR** ; **B. M. P. VERHAGEN** ; **S. SONNEVELD** ; **J. B. GRIMM** ; **L. D. LAVIS** ; **M. E. TANENBAUM**. Multi-Color Single-Molecule Imaging Uncovers Extensive Heterogeneity in mRNA Decoding.. *Cell*, 2019, vol. 178
- **Z. B. KATZ** ; **B. P. ENGLISH** ; **T. LIONNET** ; **Y. J. YOON** ; **N. MONNIER** ; **B. OVRYN** ; **M. BATHE** ; **R. H. SINGER**. Mapping translation "hot-spots" in live cells by tracking single molecules of mRNA and ribosomes. *Elife*, 2016, vol. 5, e10415
- **S. T. DIECK** ; **L. KOCHEN** ; **C. HANUS** ; **M. HEUMÜLLER** ; **I. BARTNIK** ; **B. NASSIM-ASSIR** ; **K. MERK** ; **T. MOSLER** ; **S. GARG** ; **S. BUNSE**. Direct visualization of newly synthesized target proteins in situ.. *Nature Methods*, 2015, vol. 12, 411-414
- **X. WANG** ; **W. E. ALLEN** ; **M. A. WRIGHT** ; **E. L. SYLWESTRAK** ; **N. SAMUSIK** ; **S. VESUNA** ; **K. EVANS** ; **C. LIU** ; **C. RAMAKRISHNAN** ; **J. LIU**. Three-dimensional intact-tissue sequencing of single-cell transcriptional states. *Science*, 2018, vol. 361
- **I. TIROSH** ; **B. IZAR** ; **S. M. PRAKADAN** ; **M. H. WADSWORTH 2ND** ; **D. TREACY** ; **J. J. TROMBETTA** ; **A. ROTEM** ; **C. RODMAN** ; **C. LIAN** ; **G. MURPHY**. Dissecting the multicellular ecosystem of metastatic melanoma by single-cell RNA-seq. *Science*, 2016, vol. 352, 189-196
- **F. M. FAZAL** ; **S. HAN** ; **K. R. PARKER** ; **P. KAEWSAPSAK** ; **J. XU** ; **A. N. BOETTIGER** ; **H. Y. CHANG** ; **A. Y. TING**. Atlas of Subcellular RNA Localization Revealed by APEX-Seq. *Cell*, 2019, vol. 178
- **X. WANG** ; **Z. LU** ; **A. GOMEZ** ; **G. C. HON** ; **Y. YUE** ; **D. HAN** ; **Y. FU** ; **M. PARISIEN** ; **Q. DAI** ; **G. JIA**. N6-methyladenosine-dependent regulation of messenger RNA stability. *Nature*, 2014, vol. 505, 117-120
- **H. SHI** ; **X. WANG** ; **Z. LU** ; **B. S. ZHAO** ; **H. MA** ; **P. J. HSU** ; **C. LIU** ; **C. HE**. YTHDF3 facilitates translation and decay of N6-methyladenosine-modified RNA. *Cell Res*, 2017, vol. 27, 315-328
- **A. SAKAUE-SAWANO** ; **H. KUROKAWA** ; **T. MORIMURA** ; **A. HANYU** ; **H. HAMA** ; **H. OSAWA** ; **S. KASHIWAGI** ; **K. FUKAMI** ; **T. MIYATA** ; **H. MIYOSHI**. Visualizing spatiotemporal dynamics of multicellular cell-cycle progression. *Cell*, 2008, vol. 132, 487-498
- **S.-B. KOH** ; **P. MASCALCHI** ; **E. RODRIGUEZ** ; **Y. LIN** ; **D. I. JODRELL** ; **F. M. RICHARDS** ; **S. K. LYONS**. Quantitative FastFUCCI assay defines cell cycle dynamics at single-cell level. *Journal of Cell Science*, 2016, vol. 130 (2), 512-520
- **D. MAHDESSIAN** ; **A. J. CESNIK** ; **C. GNANN** ; **F. DANIELSSON** ; **L. STENSTRÖM** ; **M. ARIF** ; **C. ZHANG** ; **T. LE** ; **F. JOHANSSON** ; **R. SHUTTEN**. Spatiotemporal dissection of the cell cycle with single-cell proteogenomics. *Nature*, 2021, vol. 590, 649-654
- **B. S. ZHAO** ; **I. A. ROUNDTREE** ; **C. HE**. Post-transcriptional gene regulation by mRNA modifications. *Nat. Rev. Mol. Cell Biol.*, 2017, vol. 18, 31-42
- **A. SAUNDERS** ; **E. Z. MACOSKO** ; **A. WYSOKER** ; **M. GOLDMAN** ; **F. M. KRIENEN** ; **H. DE RIVERA** ; **E. BIEN** ; **M. BAUM** ; **L. BORTOLIN** ; **S. WANG**. Molecular Diversity and Specializations among the Cells of the Adult Mouse Brain. *Cell*, 2018, vol. 174
- **Z. YAO** ; **C. T. J. VAN VELTHOVEN** ; **T. N. NGUYEN** ; **J. GOLDY** ; **A. E. SEDENO-CORTES** ; **F. BAFTI-ZADEH** ; **D. BERTAGNOLLI** ; **T. CASPER** ; **M. CHIANG** ; **K. CRICHTON**. A taxonomy of transcriptomic cell types across the isocortex and hippocampal formation. *Cell*, 2021, vol. 184 (12), 3222-3241
- **A. ZEISEL** ; **H. HOCHGERNER** ; **P. LÖNNERBERG** ; **A. JOHNSSON** ; **F. MEMIC** ; **J. VAN DER ZWAN** ; **M. HÄRING** ; **E. BRAUN** ; **L. E. BORM** ; **G. LA MANNO**. Molecular Architecture of the Mouse Nervous System. *Cell*, 2018, vol. 174
- Tabula Muris Consortium, Overall coordination, Logistical coordination, Organ collection and processing, Library preparation and sequencing, Computational data analysis, Cell type annotation, Writing group, Supplemental text writing group, Principal investigators, Single-cell transcriptomics of 20 mouse organs creates a Tabula Muris. *Nature*, 2018, vol. 562, 367-372
- **TABULA MURIS CONSORTIUM**. A single-cell transcriptomic atlas characterizes ageing tissues in the mouse. *Nature*, 2020, vol. 583, 590-595
- **X. HAN** ; **R. WANG** ; **Y. ZHOU** ; **L. FEI** ; **H. SUN** ; **S. LAI** ; **A. SAADATPOUR** ; **Z. ZHOU** ; **H. CHEN** ; **F. YE**. Mapping the Mouse Cell Atlas by Microwell-Seq. *Cell*, 2018, vol. 173, 1307
- **J. CAO** ; **M. SPIELMANN** ; **X. QIU** ; **X. HUANG** ; **D. M. IBRAHIM** ; **A. J. HILL** ; **F. ZHANG** ; **S. MUNDLOS** ; **L. CHRISTIANSEN** ; **F. J. STEEMERS**. The single-cell transcriptional landscape of mammalian organogenesis. *Nature*, 2019, vol. 566, 496-502
- **E. S. LEIN** ; **M. J. HAWRYLYCZ** ; **N. AO** ; **M. AYRES** ; **A. BENSINGER** ; **A. BERNARD, A** ; **F. BOE** ; **M. S. BOGUSKI** ; **K. S. BROCKWAY** ; **E. J. BYRNES**. Genome-wide atlas of gene expression in the adult mouse brain. *Nature*, 2007, vol. 445, 168-176
- **M. S. FERNANDOPULLE** ; **J. LIPPINCOTT-SCHWARTZ** ; **M. E. WARD**. RNA transport and local translation in neurodevelopmental and neurodegenerative disease. *Nat. Neurosci.*, 2021, vol. 24, 622-632
- **A. BIEVER** ; **C. GLOCK** ; **G. TUSHEV** ; **E. CIIRDAEVA** ; **T. DALMAY** ; **J. D. LANGER** ; **E. M. SCHUMAN**. Monosomes actively translate synaptic mRNAs in neuronal processes. *Science*, 2020, vol. 367

- **C. GLOCK** ; **A. BIEVER** ; **G. TUSHEV** ; **B. NASSIM-ASSIR** ; **A. KAO** ; **I. BARTNIK** ; **S. TOM DIECK** ; **E. M. SCHUMAN**. The translatome of neuronal cell bodies, dendrites, and axons.. *Proc. Natl. Acad. Sci. U. S. A.*, 2021, vol. 118
- **L. M. MESERVEY** ; **V. V. TOPKAR** ; **M.-M. FU**. mRNA Transport and Local Translation in Glia.. *Trends Cell Biol.*, 2021, vol. 31, 419-423
- **K. SAKERS** ; **A. M. LAKE** ; **R. KHAZANCHI** ; **R. OUWENGA** ; **M. J. VASEK** ; **A. DANI** ; **J. D. DOUGHERTY**. Astrocytes locally translate transcripts in their peripheral processes. *Proc. Natl. Acad. Sci. U. S. A.*, 2017, vol. 114, E3830-E3838
- **C. MÜLLER** ; **N. M. BAUER** ; **I. SCHÄFER** ; **R. WHITE**. Making myelin basic protein - from mRNA transport to localized translation. *Frontiers in Cellular Neuroscience*, 2013, vol. . 7
- **Y. HE** ; **X. TANG** ; **J. HUANG** ; **J. REN** ; **H. ZHOU** ; **K. CHEN** ; **A. LIU** ; **H. SHI** ; **Z. LIN** ; **Q. LI**. ClusterMap for multi-scale clustering analysis of spatial gene expression.. *Nat. Commun*, 2021, vol. 5909, 12
- **K. S. BURKE** ; **K. A. ANTILLA** ; **D. A. TIRRELL**. A Fluorescence in Situ Hybridization Method To Quantify mRNA Translation by Visualizing Ribosome-mRNA Interactions in Single Cells.. *ACS Cent. Sci.*, 2017, vol. 3, 425-433
- **D. W. HUANG** ; **B. T. SHERMAN** ; **R. A. LEMPICKI**. Systematic and integrative analysis of large gene lists using DAVID bioinformatics resources. *Nat. Protoc*, 2009, vol. 4, 44-57
- **D. W. HUANG** ; **B. T. SHERMAN** ; **R. A. LEMPICKI**. Bioinformatics enrichment tools: paths toward the comprehensive functional analysis of large gene lists. *Nucleic Acids Res.*, 2008, vol. 37, 1-13